(19) Europäisches Patentamt
European Patent Office
Office européen des brevets

(11) **EP 1 881 423 A1**

(12) **EUROPEAN PATENT APPLICATION**
published in accordance with Art. 153(4) EPC

(43) Date of publication:
**23.01.2008 Bulletin 2008/04**

(51) Int Cl.:
**G06F 17/30** (2006.01)    **G06F 19/00** (2006.01)

(21) Application number: **06732329.5**

(22) Date of filing: **25.04.2006**

(86) International application number:
**PCT/JP2006/308669**

(87) International publication number:
**WO 2006/115260 (02.11.2006 Gazette 2006/44)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.04.2005 JP 2005127118**

(71) Applicant: **Intellectual Property Bank Corp.**
**1-21-19, Toranomon, Minato-ku**
**Tokyo 1050001 (JP)**

(72) Inventors:
• **MASUYAMA, Hiroaki**
  **Osaka, 5600054 (JP)**
• **YOSHINO, Noriaki,**
  **c/o Intellectual Property Bank Corp**
  **1050001   Tokyo (JP)**

(74) Representative: **Zimmermann, Gerd Heinrich et al**
**Zimmermann & Partner,**
**P.O. Box 330 920**
**80069 München (DE)**

(54) **DEVICE FOR AUTOMATICALLY CREATING INFORMATION ANALYSIS REPORT, PROGRAM FOR AUTOMATICALLY CREATING INFORMATION ANALYSIS REPORT, AND METHOD FOR AUTOMATICALLY CREATING INFORMATION ANALYSIS REPORT**

(57)     [PROBLEMS] To provide a device and others for automatically creating an information analysis report by analyzing information on a document to be surveyed with comparison to a document as a comparison object.

[MEANS FOR SOLVING PROBLEMS] A device (100) for automatically creating an information analysis report includes a processing device (1), an input device (2), a recording device (3), and an output device (4). When creating an information analysis report: a document to be surveyed and a document to be compared are specified and inputted; an information analysis condition is inputted; a population document formed by a document group similar to the document to be surveyed is selected from the document to be compared; an index word characteristic to the population document of the document to be surveyed is extracted; according to the population document and the index word, an information analysis report representing the feature of the document to be surveyed is created; and the created information report document is outputted to display means, recording means, or communication means.

FIG. 2

**Description**

TECHNICAL FIELD

The present invention relates to a device for analyzing a document, particularly a document to be surveyed and document group, and a device for, a program for, and a method for automatically creating information analysis report being characterized by that the document and document group.

BACKGROUND ART

The number of technical documents including patent documents and other documents has surely been increased year by year. In recent years, since computerized documentation of distribution has been enabled, automatic searching systems capable of searching only for documents similar to a document to be surveyed among an enormous number of documents have been reduced to practice. However, the number of similar documents obtained from a search result is still large, and a person skilled in the art must read and judge for the similar documents in order to understand the content or nature of the document to be surveyed.

For example, in the "search device for similar document and search method for the same" disclosed in Patent Document 1, index terms contained in a document or a document group for research must be compared to index terms included in a document group for comparison, similarity measures must be calculated based on the kinds of similar index terms or the occurrence frequencies of the terms, and the documents are input in the descending order of similarity measures starting from the most similar document. Fig. 34 is a diagram of the general structure of the device disclosed in Patent Document 1. An inputted document to be surveyed from an input device 602 is compared to a document group in a database in an external auxiliary storage 603 based on an extraction condition by a similarity measure calculation system in a controller 601. Then, the similarity measure processing is carried out, the result is output by an output device 604, and a skilled evaluator reads the contents of documents having high similarity measures based on the result of the output list of documents to evaluate the document to be surveyed. The evaluator has to inspect a large number of documents from those several documents to about several thousands documents to know the contents of documents with high similarity measures.

[Patent Document 1] JP-A-11-73415

DISCLOSURE OF THE INVENTION

In the automatic similar document searching system like the system disclosed in Patent Document 1, a list of documents similar to a document to be surveyed must be output from a document group for comparison as a result of search, and the evaluator must extract and read about as many as several to several thousand similar documents from the list of the documents similar to the document to be surveyed, find documents similar to the document to be surveyed to evaluate them, and then determine the nature of the document to be surveyed based on them. Therefore, the evaluator must extract and read about as many as several to several thousand documents before the evaluator finds exact expression for the nature of the document to be surveyed.

It is therefore an object of the invention to automatically create an information analysis report that can exactly report about the information of a document to be surveyed without the necessity of human inspection of the contents of the document to be surveyed and an enormous number of documents to be compared.

In order to solve the above disadvantage, a device for automatically creating information analysis report according to the invention creates a report representing characteristics of a document to be surveyed relative to a document to be compared in information analysis of the document to be surveyed, and the device includes input means for receiving input of at least the document to be surveyed, selecting means for selecting a population document group from the information of a document to be compared group stored in a database based on the input document to be surveyed, the population document group being a set of population documents similar to the document to be surveyed, extracting means for extracting characteristic index terms of the document to be surveyed relative to the population documents, creating means for creating an information analysis report representing characteristics of the document to be surveyed based on the population documents and the index terms, and output means for outputting the information analysis report to display means, recording means, or communicating means.

For example, the device further includes calculating means for calculating a similarity relative to the document to be compared, and the selecting means selects population documents based on the result by the calculating means. Further, the calculating means calculates a similarity based on a function value of an occurrence frequency per index term in each document and a document frequency.

The device further includes map creating means for having the population or the index terms distributed in a map state, output data obtaining means for obtaining part of the data of the population or the index terms, fixed comment obtaining means for obtaining a fixed comment corresponding to the content of the map and data, and comment entering means for entering a free comment, and the creating means creates an information analysis report representing characteristics of the document to be surveyed by combining the map, the data and/or the comment.

In a preferred embodiment, the creating means carries out totaling for each of the index terms or prescribed items in the population documents, the totaling including keyword totaling, time-series totaling representing the time-series transition of keywords or prescribed items in the population documents, and/or matrix totaling for a plurality of prescribed items in the population documents and creates an information analysis report including the results of totaling.

More preferably, the creating means creates a portfolio represented by the totaling result of prescribed items in the keywords or the population documents and a matrix of the time-series increase ratio of the totaling result in the time-series totaling, and creates an information analysis report including the portfolio.

In another preferred embodiment, the creating means, includes first occurrence value frequency calculating means for calculating a function value of the occurrence frequency of the extracted index term in the document to be compared group, second occurrence value frequency calculating means for calculating a function value of the occurrence frequency of the extracted index term in the population document group, and frequency scatter diagram creating means for creating a frequency scatter diagram including each index term and their positioning data based on a combination of the function value of the occurrence frequency in the calculate document to be compared and the function value of the occurrence frequency in the population document group for each index term.

According to yet another embodiment, the creating means includes extracting means for extracting the content data and time data of the population documents or the document to be surveyed and the population documents, tree-like diagram creating means for creating a tree-like diagram representing the co-relation between the plurality of documents based on the content data of each document, clustering means for cutting the tree-like diagram according to a prescribed rule and extracting a cluster, and inside cluster arranging means for determining the arrangement of the document group belonging to each cluster in the cluster based on the time data of each document.

More preferably, the clustering means cuts the tree-like diagram to extract a parent cluster, creates a partial tree-like diagram representing the co-relation of the document group belonging to the parent cluster based on the content data of each document belonging to the parent cluster, and cuts the created partial tree-like diagram according to a prescribed rule to extract a descendant cluster.

The clustering means preferably removes from each document vector a vector component whose deviation among a plurality of documents belonging to the parent cluster is smaller than a value determined by a prescribed method in order to create the partial tree-like diagram.

According to a still further embodiment, the creating means includes evaluation value calculating means for calculating an evaluation value in each cluster for each index term, concentration degree calculating means for calculating the sum of the evaluation values in the each cluster for each index term in all the clusters, calculating the ratio of the evaluation values in each cluster relative to the sum, calculating a square of each ratio, and calculating the degree of concentration in the distribution of each index term in the cluster obtained by calculating the sum of the square of the ratio in all the clusters, share calculating means for calculating the sum of the evaluation values of the index terms in the clusters to be analyzed for all the index terms extracted from each cluster, and calculating the share of each index term in the cluster to be analyzed obtained by calculating the ratio of each index term relative to the sum for each index term, first inverse calculating means for calculating a function value of the inverse of the occurrence frequency of each index term in the cluster, second inverse calculating means for calculating a function value of the inverse of the occurrence frequency of each index term in all the documents including the cluster, creativity degree calculating means for calculating a creativity degree by a function value of the result of subtracting the result of calculation by the second inverse calculating means from the result of calculating by the first inverse calculating means, and keyword extracting means for extracting keywords based on a combination of a degree of concentration calculated by the concentration degree calculating means, a share calculated for each of the document group for analysis by the share calculating means, and a creativity degree calculated by the creativity degree calculating means.

The device for creating information analysis report according to the invention further includes a web server connected to a network and accepting input of a document to be surveyed from a client connected through the network, a management server that queues said document to be surveyed and requests the analysis server to process a document to be surveyed to be processed next, and the analysis server that responds to said request to select a population document group that is a set of population documents similar to the document to be surveyed from information of a document to be compared group stored in a database based on said input document to be surveyed, extract characteristic index terms of said document to be surveyed relative to the population document group, and creates an information analysis report representing characteristics of said document to be surveyed.

In order to solve the above described disadvantage, an program for automatically creating information analysis report creates a report representing characteristics of a document to be surveyed relative to a document to be compared in information analysis of the document to be surveyed and enables a computer to function as input means for accepting input of at least the document to be surveyed, selecting means for selecting a population document group from the information of a document to be compared group stored in a database based on the input document to be surveyed, the population documents being a set of population documents similar to the document to be surveyed, extracting means for extracting characteristic index terms of the document to be surveyed relative to the population documents, creating means for creating an information analysis report representing characteristics of the document to be surveyed based on the population documents and the index terms, an output means for outputting the information analysis report to display means, recording means, or communicating means.

For example, the program further enables the computer to function as calculating means for calculating a similarity relative to the document to be compared, and the selecting means selects population documents based on the result by the calculating means. The calculating means calculates a similarity based on a function value of an occurrence frequency per index term in each document and a document frequency.

For example, program further enables the computer to function as at least one of map creating means for having the population or the index terms distributed in a map state, output data obtaining means for obtaining part of the data of the population or the index terms, fixed comment obtaining means for obtaining a fixed comment corresponding to the content of the map and data, and comment entering means for entering a free comment, the creating means creating an information analysis report representing characteristics of the document to be surveyed by combining the map, the data and/or the comment.

In order to solve the above-described disadvantage, an method for automatically creating information analysis report creates a report representing characteristics of a document to be surveyed relative to a document to be compared in information analysis of the document to be surveyed, the method includes the steps of inputting by accepting input of at least the document to be surveyed, selecting a population document group from the information of a document to be compared group stored in a database based on the input document to be surveyed, the population document group being a set of population documents similar to the document to be surveyed, extracting characteristic index terms of the document to be surveyed relative to the population documents, creating an information analysis report representing characteristics of the document to be surveyed based on the population documents and the index terms, and outputting the information analysis report to display means, recording means, or communicating means.

For example, the method further includes the step of calculating a similarity relative to the document to be compared, wherein in the selecting step, population documents are selected based on the result by the calculating step. Further, in the calculating step, a similarity is calculated based on a function value of an occurrence frequency per index term in each document and a document frequency.

For example, the method further includes a map creating step of having the population or the index terms distributed in a map state, an output data obtaining step of obtaining part of the data of the population or the index terms, a fixed comment obtaining step of obtaining a fixed comment corresponding to the content of the map and data, and a comment entering step of entering a free comment, and in the creating step, an information analysis report representing characteristics of the document to be surveyed is created by combining the map, the data and/or the comment.

According to the invention, based on an input document to be surveyed and documents to be compared and conditions for information analysis, population documents consisting of a document group similar to a document to be surveyed are selected from the documents to be compared, index terms characteristic of the document to be surveyed relative to the population documents are extracted, and an information analysis report representing the characteristics of the document to be surveyed is created based on the population documents and the index terms.

In this way, a person does not have to read the contents of the document to be surveyed and an enormous number of documents to be compared and still an information analysis report that can exactly report about the information of the document to be surveyed can automatically be created.

An information analysis report representing characteristics of the document to be surveyed can be created by combining a map formed by distributing the population or the index terms, the data of the population or the index terms, and a fixed comment or a free comment according to the content of the map and data can be created.

According to the invention, the document to be surveyed and the documents to be compared are specified and input, a condition for information analysis is input, population documents consisting of a document group similar to the document to be surveyed are selected from the documents to be compared, index terms characteristic of the document to be surveyed relative to the population are extracted, an information analysis report representing the characteristics of the document to be surveyed is created, and the obtained information analysis report is output to display means, recording means or communicating means.

For example, similarities relative to the documents to be compared are calculated and population documents are selected based on the result of calculation. In the calculating step, a similarity based on a function value of the occurrence frequency and a document frequency for each index term in an each document is calculated.

In this way, an information analysis report that can exactly report about the information of a document to be surveyed can automatically be created without the necessity of human reading of the contents of the document to be surveyed and an enormous number of documents to be compared.

The device further includes map creating means for having the population or the index terms distributed in a map state, output data obtaining means for obtaining part of the data of the population or the index terms, fixed comment obtaining means for obtaining a fixed comment corresponding to the content of the map and data, and comment entering means for entering a free comment, and the creating means creates an information analysis report representing characteristics of the document to be surveyed by combining the map, the data and/or the comment. Therefore, an information analysis report including the map, the population or the index term data, and a fixed comment or free comment according to the contents of the map and the data can be created.

BRIEF DESCRIPTION OF THE DRAWINGS

Fig. 1 is a diagram of the configuration of a device for automatically creating information analysis report according to an embodiment of the invention.
Fig. 2 is a block diagram of the configuration of components in the device for automatically creating information analysis report 100.
Fig. 3 is a flowchart showing the operation of an input device 2.
Fig. 4 is a flowchart showing the operation of a processing device 1.
Fig. 5 is a flowchart showing the operation of an output device 4.
Fig. 6 is a view showing an input condition-setting example (1).
Fig. 7 is a view showing an input condition-setting example (2).
Fig. 8 is a view showing an input condition-setting example (3).
Fig. 9 shows an output condition-setting example.
Fig. 10 shows an example of an information analysis report.
Fig. 11 shows a patent applicant ranking in all the period.
Fig. 12 shows a patent applicant ranking in the last three years.
Fig. 13 shows the ranking of classes in International Patent Classification (IPC).
Fig. 14 shows the ranking of classes and sub classes in International Patent Classification (IPC).
Fig. 15 is a matrix map of the applicants and International Patent Classification (IPC).
Fig. 16 is a table representing the relation between the top ten applicants and the top five classes in International Patent Classification (IPC).
Fig. 17 shows the relation between the top 20 applicants and classes in International Patent Classification (IPC).
Fig. 18 shows a distribution of cases for each of important keywords (for all the documents to be compared).
Fig. 19 shows a distribution of cases for each of important keywords (for the population).
Fig. 20 shows a transition of the number of filed applications for each of the applicants.
Fig. 21 is a table representing the relation between the applicants and the number of applications.
Fig. 22 shows a transition of the number of cases based on International Patent Classification (IPC).
Fig. 23 is a table showing the relation between the International Patent Classification (IPC) and the number of applications.
Fig. 24 shows a transition of the number of cases based on prescribed International Patent Classification (IPC).
Fig. 25 shows a portfolio for the entire population.
Fig. 26 shows a portfolio for International Patent Classification (IPC).
Fig. 27 shows a transition of the number of cases for each of important keywords (for all the documents to be compared).
Fig. 28 is a table showing the relation between important keywords (for all the documents to be compared) and the number of applications.
Fig. 29 shows a transition of the number of cases for each of important keywords (for the population).
Fig. 30 is a table showing the relation between important keywords (for the population) and the number of applications.
Fig. 31 is a frequency scatter diagram of a keyword distribution in a document to be surveyed.
Fig. 32 is a structure diagram of a document to be surveyed.
Fig. 33 is a table showing the similarity ranking based on similarity in populations and publication content abstracts.
Fig. 34 is a diagram of the configuration of a conventional similar document search device.
Fig. 35 shows tables for use in illustrating similarity calculation.
Fig. 36 is a diagram of the configuration including a device for automatically creating information analysis report according to a second embodiment of the invention and a client.
Figs. 37A and 37B are view of examples of a screen on the display device of the client.
Fig. 38 is a flowchart showing processing carried out by a first analysis server.
Fig. 39 is a flowchart showing an example of totaling processing.
Fig. 40 is a flowchart sequentially showing all the process steps necessary for calculating a coordinate for each keyword in a frequency scatter diagram.

Fig. 41 is a block diagram of a configuration for creating a patent structure diagram in the first analysis server.

Fig. 42 is a flowchart showing a general idea of the process of creating a patent structure diagram in the first analysis server.

Fig. 43 is a flowchart for use in illustrating in more detail the process of extracting a cluster.

Figs. 44A to 44F show examples of tree-like arrangement in the process of extracting a cluster according to the embodiment.

Fig. 45 is a block diagram of a configuration for extracting keywords.

Fig. 46 is a flowchart for use in illustrating more in detail the process of extracting keywords.

Fig. 47 is a diagram showing the flow of the process until cluster information is output.

Fig. 48 is a flowchart showing processing carried out by a client, a web server, a management server, first and second analysis servers, and a database server according to another embodiment.

Fig. 49 is a flowchart showing processing carried out by a client, a web server, a management server, first and second analysis servers, and a database server according to yet another embodiment.

Fig. 50 is a flowchart showing processing carried out by a client, a web server, a management server, first and second analysis servers, and a database server according to a still further embodiment.

DESCRIPTION OF REFERENCE NUMERALS SIGNS

| | |
|---|---|
| 1 | Processing device |
| 2 | Input device |
| 3 | Recording device |
| 4 | Output device |
| 100 | Device for automatically creating information analysis report |
| 110 | Document to be surveyed d read out unit |
| 120 | Index term (d) extraction unit |
| 130 | Document to be compared P read out unit |
| 140 | Index term (P) extraction unit |
| 121 | TF(d) calculation unit |
| 141 | TF(P) calculation unit |
| 142 | IDF(P) calculation unit |
| 150 | Similarity calculation unit |
| 151 | Population narrowing unit |
| 160 | Population document S selection unit |
| 170 | Index term (S) extraction unit |
| 171 | IDF(S) calculation unit |
| 180 | Characteristic index term, similarity in population, frequency scatter diagram, structure diagram calculation unit |
| 210 | Document to be surveyed d condition input unit |
| 220 | Document to be compared P condition input unit |
| 230 | Extraction condition and others input unit |
| 310 | Condition recording unit |
| 320 | Work result storage unit |
| 330 | Document storage unit |
| 410 | Map creation condition read out unit |
| 412 | Map data obtaining portion unit |
| 420 | Data output condition read out unit |
| 422 | Output data obtaining unit |
| 430 | Comment condition read out unit |
| 432 | Fixed comment acquisition unit |
| 435 | Comment addition unit |
| 440 | Report creation unit |
| 450 | Output unit |

BEST MODE FOR CARRYING OUT THE INVENTION

Now, it will be described in detail about an embodiment of the invention with the accompanied drawings.

Definitions

Terms used herein are defined or detailed.

d: document to be surveyed (a case related to research. For example, a document such as Patent Publication No.＿＿＿＿＿＿＿ or group of such documents)

documents to be compared: all documents P or population documents S

P: all documents (the entire set of documents to be compared including the document to be surveyed d)

N: the number of all the documents P

p: one document in all the documents (N documents exist such as $p_a$, $p_b$, ...)

S: population documents (part of all the documents P and a document group similar to a document to be surveyed d among all the documents P in the embodiment (including d))

N': the number of the population documents S (N'<N)

s: one document in the population documents (N' documents exist such as $s_a$, $s_b$, ...)

In the drawings, d or (d), P or (P), p or (p), or S or (S) attached to components represent a document to be surveyed, a document to be compared, one document of all documents, or population documents, respectively, and these characters will hereinafter be attached to components as well as operation for clarification. For example, an index term (d) means an index term in a document to be surveyed d. More specifically, according to the embodiment, it will be assumed that there are x index terms in a document d represented as $d_1$, $d_2$, $d_3$, ..., $d_x$. There are ya index terms in a document pa represented as $P_{a1}$, $p_{a2}$, ..., $P_{aya}$, and a part of or all of these words would match the d'index terms represented as $d_1$, $d_2$, ..., $d_x$ in some cases.

There are yb index terms in a document pb, represented as $p_{b1}$, $p_{b2}$,..., $p_{byb}$, and similarly, a part of or all of these terms would match the d'index terms represented as $d_1$, $d_2$, ... , $d_x$ in some cases.

Similarly, there are yy index terms in a document py represented as $p_{y1}$, $p_{y2}$, ..., $P_{yyy}$, and similarly, a part of or all of these terms would match the d'index terms represented as $d_1$, $d_2$, ..., $d_x$ in some cases.

Note that if vectors are created for those other than index terms in coincidence with the index terms represented as $d_1$, $d_2$,..., $d_x$ among the index terms for the document $p_a$, their inner product is "0" as will be described. Therefore, only the d'index terms represented as $d_1$, $d_2$, ..., $d_x$ are necessary as index terms for processing.

TF Operation

TF calculation represents Term Frequency calculation, and an calculation to obtain a function value of the count of occurrence frequencies (index term frequencies) of index terms in a document to be surveyed.

DF calculation represents Document Frequency calculation, and an calculation to obtain the count of the number of hits (document frequencies) when a group of documents to be compared is searched based on index terms included in a certain document.

IDF calculation represents for example the inverse of the result of DF calculation or an calculation to obtain the logarithm of the result obtained by multiplying the inverse by the number of documents P or S. The meaning or effect for the logarithm is that the interval in the scale of the function values near zero is allowed to expand while the interval in the scale of the function values for larger numbers is allowed to decrease, so that they can easily be viewed in one plane.

Functions used in the embodiment will be expressed as follows.

TF(d): the occurrence frequency in d based on d's index terms ($d_1$, ..., $d_x$). Then, TF (d) can be rewritten into the form of TF(index term; document) as follows.

$TF(d_1;d)$ : the occurrence frequency based on document d's index term $d_1$ in document d

$TF(d_2;d)$ : the occurrence frequency based on document d's index term $d_2$ in document d

　　　...

$TF(d_x;d)$ : the occurrence frequency based on document d's index term $d_x$ in document d

$TF(P_a)$ : the occurrence frequency based on P's index terms ($P_{a1}$,..., $p_{aya}$) in $P_a$

Then, $TF(P_a)$ can be rewritten in the form of TF(index term; document) as follows.

$TF (p_{a1}; p_a)$ : the occurrence frequency based on document $p_a$'s index term $p_{a1}$ in document $p_a$

$TF (p_{a2};p_a)$ : the occurrence frequency based on document $p_a$'s index term $p_{a2}$ in document $p_a$

　　　...

$TF(p_{aya}; p_a)$ : the occurrence frequency based on document $p_a$'s index term $p_{aya}$ in document $P_a$

However, as will be described, as for $TF(p_a)$, only the following occurrence frequencies are necessary.

$TF(d_1; p_a)$: the occurrence frequency based on document $p_a$'s index term $d_1$ in document $p_a$

$TF(d_2; p_a)$: the occurrence frequency based on document $p_a$' s index term $d_2$ in document $p_a$

　　　...

$TF(d_x;p_a)$: the occurrence frequency based on document $p_a$' s index term $d_x$ in document $p_a$

$TF(d_1;p_b)$: the occurrence frequency based on document $p_b$'s index term $d_1$ in document $P_b$

$TF(d_2;p_b)$: the occurrence frequency based on document $p_b$'s index term $d_2$ in document $p_b$

　　　...

$TF(d_x;p_b)$: the occurrence frequency based on document $p_b$'S index term $d_x$ in document $P_b$.

$TF(d_1;p_y)$ : the occurrence frequency based on document $p_y$'s index term $d_1$ in document $p_y$

$TF(d_2;p_y)$: the occurrence frequency based on document $p_y$'s index term $d_2$ in document $p_y$

...

$TF(d_x;py)$: the occurrence frequency based on document $p_y$'s index term $d_x$ in document $p_y$

In other words, among document $P_a$' index terms $(P_{a1}, ... , P_{aya})$, it is only necessary to calculate about $(d_1, ..., d_x)$.

$TF(p_b)$ is the occurrence frequency in document $p_b$. For example, $TF(d_1; P_b)$ represents the occurrence frequency based on document $p_b$'s index term $d_1$ in document $p_b$ and $TF(p_y)$ represents the occurrence frequency in document $p_y$. For example, $TF(d_2;p_y)$ represents the occurrence frequency based on document $p_y$'s index term $d_2$ in document $p_y$.

DF Operation

$DF(P)$: the document frequency based on d's index term d in P

$DF(P)$ is a value that indicates how frequently the same index terms $d_1, ... , d_x$ as index terms in document d are used in all the documents. For example, if an index term "device" is used in 1/10 of six million documents, DF is 600 thousands.

Similarly, it can be rewritten in the form of DF(index term; all documents) as follows.

$DF(d_1;P)$ : in N documents ($p_a$ to $p_y$) in the all of the P, the document frequency (the number of documents) with at least one occurrence of $d_1$ based on d's index term $d_1$

$DF(d_2; P)$ : in N documents ($p_a$ to $p_y$) in all of the P, the document frequency (the number of documents) with at least one occurrence of $d_2$ based on d's index term $d_2$

...

$DF(d_x; P)$ : in N documents ($p_a$ to $p_y$) in all of the P, the document frequency (the number of documents) with at least one occurrence of $d_x$ based on d's index term $d_x$

As for DF(S), the definition may be written in the same manner, but the detailed description is not provided.

DF(S): the document frequency in S based on d's index term

IDF

IDF as will be described is the inverse of the ratio of DF (the document frequency based on d's index term in all the documents P) to N (the number of all the documents), and is represented by its logarithm for equal distribution.

```
IDF(P): inverse of DF(P) × logarithm of document number:
ln[N/DF(P)]
```

```
IDF(S): inverse of DF(S) × logarithm of document number:
ln[N'/DF(S)]
```

If for example N (the number of all the documents) is six millions, and $DF(d_1;P)$ = six millions, in other words, if a certain index term $d_1$ is included in all the documents P, $IDF(d_1;P)$ = 0. If $DF(d_2; P)$ - 600 thousands, in other words, if a certain index term $d_2$ is included in 1/10 of all the documents, $IDF(d_2; P)$ = 1.

TFIDF and Document Vectors

TFIDF: the product of the function value of TF and the function value of IDF (inverse of DF) that is calculated for each index term in a document. This is a numerical value for each index term based on which the similarity of documents is determined, and the value is in proportion with the occurrence frequency of a certain term in a document and a document frequency is made into its function value and made inverse-proportion.

As a simple example, let us now consider multiplication of TF(d) and IDF(P) on a one to one basis. Note however that the calculation is not necessarily limited to the one-to-one basis. For example, the component of the document vector of d may be considered as follows.

$$TF(d_1;d)*IDF(d_1;P)$$
$$TF(d_2;d)*IDF(d_2;P)$$
$$\ldots$$
$$TF(d_x;d)*IDF(d_x;P)$$

The document vector of $p_a$ is considered as follows:

$$TF(d_1;p_a)*IDF(d_1;P)$$
$$TF(d_2;p_a)*IDF(d_2;P)$$
$$\ldots$$
$$TF(d_x;p_a)*IDF(d_x;P)$$

Herein, the document vector has as a component the values of index terms obtained by operating TFIDF for each index term in a document.

The component of the vector of document d is represented for example as TF $(d_1;d)$*IDF $(d_1;P)$, ..., TF $(d_1;d)$*IDF $(d_1;P)$. The component of the vector of the document $p_a$ is represented for example as TF$(d_x; p_a)$*IDF$(d_x;P)$. More specifically, the document vectors are as follows.

$$\{\text{document vector of document } d\}=\{TF(d_1;d)*IDF(d_1;P),$$
$$TF(d_2;d)*IDF(d_2;P), \ldots, TF(d_x;d)*IDF(d_x;P)\}$$

$$\{\text{document vector of document } p_a\} = \{TF(d_1;p_a)*IDF(d_1;P),$$
$$TF(d_2;p_a)*IDF(d_2;P), \ldots, TF(d_x;p_a)*IDF(d_x;P)\}$$

Similarity Ratio (Similarity Measure)

A similarity indicates the degree of similarity between two documents and it is also referred to as "similarity measure" in this specification. According to the embodiment, a numerical value is obtained as the inner product of two document vectors in order to measure the proximity of the natures of the two document vectors. For example, the similarity (D, $P_a$: P) of a search document d to a document to be compared $P_a$ that belongs to a document to be compared group P is obtained as the inner product of the document vector (d) of the search document d and the document vector ($p_a$) of the document to be compared $p_a$ that belongs to the document to be compared group P.

$$\{\text{similarity }(d,p_a;P)\}$$
$$=\{\text{document vector of document } d\}\bullet\{\text{document vector of document}$$
$$p_a\}=[\{TF(d_1;d)*IDF(d_1;P)\}*\{TF(d_1;p_a)*IDF(d_1;P)\}+\{TF(d_2;d)*IDF$$
$$(d_2;P)\}*\{TF(d_2;p_a)*IDF(d_2;P)\}+\ldots+\{TF(d_x;d)*IDF(d_x;P)\}*\{TF(d_x$$
$$;P_a)*IDF(d_{x2};P)\}]$$

The similarity of document to be compared p: according to the embodiment, the similarity of a search document d to a certain document to be compared p that belongs to a document to be compared group P. The ratio refers to the sum of the inner products of the document vector (d) of the search document d and the document vector (p) of the certain document to be compared p that belongs to the document to be compared group P.

Herein, the index term means a so-called keyword that is segmented from all or part of the document. Words may be extracted using a known conventional method or commercially available software by extracting significant nouns removed of particles and conjunctions. Alternatively, a database of dictionaries (thesaurus) of index terms may be acquired in advance and index terms available from the database may be used.

Note that as for a document group consisting of a plurality of search documents, an item to be extracted may be an index term as described above while a group of terms on the basis of individual documents, IPC classes, a corporation, a group of corporations, an industry, a year such as a patent application filing year, or a patent registration year may be extracted. In the following description, index terms are mostly used as typical examples in the specification. Device for automatically creating information analysis report

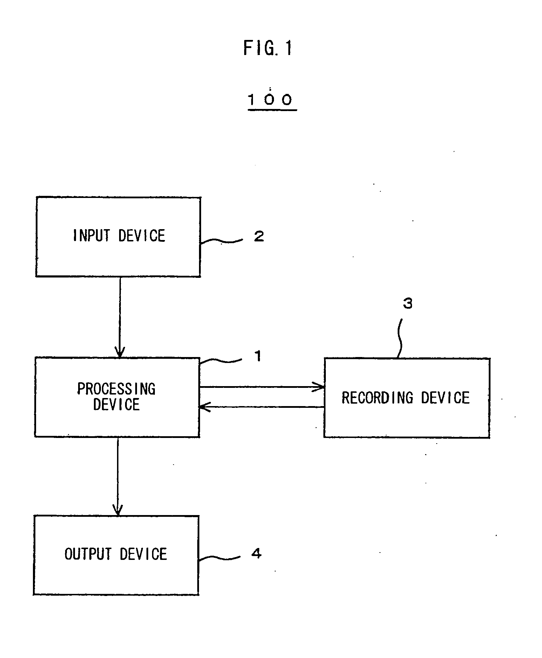

Fig. 1 is a diagram of the hardware configuration of a device for automatically creating information analysis report according to the embodiment of the invention.

As shown in Fig. 1, the device for automatically creating information analysis report 100 includes a processing device 1 including a CPU (Central Processing Unit) and a memory (storage), an input device 2 as input means such as a keyboard (manual input equipment), a recording device 3 as storing means for storing document data, conditions, operation results by the processing device 1 and the like, and an output device 4 as output means for displaying extraction results of characteristic index terms in the forms of a map and data.

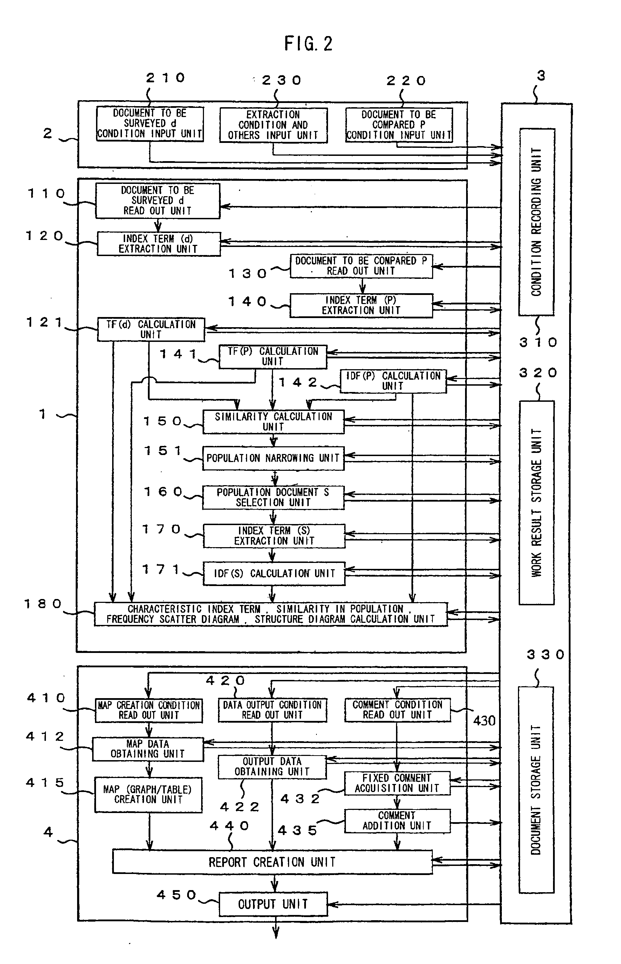

Fig. 2 is a block diagram for use in illustrating the functions of various parts of the device for automatically creating information analysis report according to the invention.

As shown in Fig. 2, the processing device 1 includes a search document d read out unit 110, an index term (d) extraction unit 120, a TF(d) calculation unit 121, a document to be compared P read out unit 130, an index term (P) extraction unit 140, a TF(P) calculation unit 141 for a document to be compared P, an IDF(P) calculation unit 142 for the document to be compared P, a similarity calculation unit 150, a population narrowing unit 151, a population document S selection unit 160, an index term(S) extraction unit 170, an IDF(S) calculation unit 171, and a characteristic index term/ similarity in population/frequency scatter diagram/structure calculation unit 180.

The input device 2 includes a search document d condition input unit 210, a document to be compared P condition input unit 220, and an extraction condition and others input unit 230.

The recording device 3 includes a condition recording unit 310, an work result storage unit 320, and a document storage unit 330. The document storage unit 330 includes an external database and an internal database. The external database means a document database such as IPDL whose services are available by Japanese Patent Office and PATOLIS whose services are available by PATOLIS Corporation. The internal database means a personally compiled database that stores commercially available data such as patent JP-ROM, a device that reads data from a medium such as an FD (flexible disk), a CD-ROM (compact disk), an MO (Optical-magnetic disk), and a DVD (digital video disk), an OCR (optical character reader) that reads a document output or manually written on paper, and a device that converts read data into electronic data such as text.

The output device 4 includes a map creation condition read out unit 410, a map data obtaining unit 412, a map (graph/table) creation unit 415, a data output condition read out unit 420, an output data obtaining unit 422, a comment condition read out unit 430, a fixed comment acquisition unit 432, a comment addition unit 435, a report creation unit 440 that creates a report by combining a map, data, and a comment, and an output unit 450 that outputs the created report.

In Figs. 1 and 2, examples of communicating means used to exchange signals and data between the processing device 1, the input device 2, the storage unit 3, and the output device 4 include a USB (universal system bus) cable that directly connects them, or they may exchanged through a network such as a LAN (local area network), or through a media such as an FD, a CD-ROM, an MO, and a DVD that stores a document. Alternatively, one of them may be used or several of the above may be combined.

The functions in the device for automatically creating information analysis report 100 shown in Figs. 1 and 2, a program for the device for automatically creating information analysis report 100, and a method of creating a report will be described in detail.

In the input device 2 shown in Fig. 2, the document to be surveyed d condition input unit 210 sets a condition for reading out a search document d by an input screen or the like. The document to be compared P condition input unit 220 sets a condition for reading documents to be compared P by the input screen or the like. The extraction condition and others input unit 230 sets an index term extraction condition for the search document d and the documents to be compared P, a condition for TF calculation, a condition for IDF calculation, a condition for operating a similarity, a condition for selecting similar documents, a condition for creating a map, a data output condition, a comment adding condition, a population narrow-down condition, and the like. These input conditions are sent to the condition recording unit 310 in the recording device 3 and stored therein.

In the processing device 1 shown in Fig. 2, the document to be surveyed d read out unit 110 read a document to be surveyed from the document storage unit 330 based on a read condition stored in the condition recording unit 310 and transfers the documents to the index term (d) extraction unit 120. The index term (d) extraction unit 120 extracts index terms from the document obtained by the document to be surveyed d read out unit 110 based on the extraction condition stored in the condition recording unit 310 and stores the extracted index terms in the work result storage unit 320.

The document to be compared P read out unit 130 reads population documents from the document storage unit 330 based on the reading condition stored in the condition recording unit 310 and transfers the documents to the index term (P) extraction unit 140. The index term (P) extraction unit 140 extracts index terms from documents obtained at the document to be compared P read out unit 130 according to the extraction condition stored in the condition recording unit 310 and stores the extracted index terms in the work result storage unit 320.

In the document to be compared P read out unit 130 and the index term (P) extraction unit 140, it is often the case that a patent publication as a whole, one type of a document to be compared is extracted. Once index terms are segmented, prepared, and stored, the index terms do not have to be segmented again and the process can be omitted.

The TF (d) calculation unit 121 carries out TF calculation to the calculation result of the index term (d) extraction unit 120 for the document to be surveyed d stored in the work result storage unit 320 based on the condition stored in the condition recording unit 310 to obtain TF (d; d), then stores the result in the work result storage unit 320 or transfers the result directly to the similarity calculation unit 150 or a characteristic index term/similarity in population/frequency scatter diagram/structure calculation unit 180.

The TF (P) calculation unit 141 carries out TF calculation to the calculation result of the index term (P) extraction unit 140 for the documents to be compared P stored in the work result storage unit 320 to obtain TF(d;p) according to the condition stored in the condition recording unit 310, stores the result in the work result storage unit 320 or directly transfers the result to the similarity calculation unit 150 or the characteristic index term/similarity in population/frequency scatter diagram/structure calculation unit 180.

The IDF(P) calculation unit 142 carries out IDF calculation to each of the index terms (d) extracted from the document to be compared d stored in the work result storage unit 320 to obtain IF(d;P) according to the condition stored in the condition recording unit 310, stores the result in the work result storage unit 320 or directly transfers the result to the similarity calculation unit 150 or directly to the characteristic index term/similarity in population/frequency scatter diagram/structure diagram and the like calculation unit 180.

The similarity calculation unit 150 obtains the calculation results of the TF(d) calculation unit 121, the TF(P) calculation unit 141, and the IDF(P) calculation unit 142 directly from them or from the work result storage unit 320 based on the conditions stored in the condition recording unit 310. Note that as described above, the calculation result of the TF (d) calculation unit 121 is TF (d; d), the calculation result of the TF(P) calculation unit 141 is TF (d; p), and the calculation result of the IDF(P) calculation unit 142 is IDF(d;P). The similarity calculation unit 150 then operates similarities of the documents to be compared P to the document to be surveyed d, and the results are attached to the documents to be compared P as their similarity data and are transferred to the work result storage unit 320 or directly transferred to the population document S selection unit 160.

In the calculation of similarities by the similarity calculation unit 150, a calculation typically represented by TFIDF calculation is carried out, and the similarities of the documents to be compared P to the document to be surveyed d are calculated. The TFIDF calculation corresponds to the product of the TF calculation result and the IDF calculation result. An example of a method of calculating the similarities (similarity measures) will be described in detail.

Now, let us assume that d is a document to be surveyed, and p represents individual documents among documents to be compared P. As a result of calculation to these documents d and p, assume that index terms segmented from the document d are "red", "blue", and "yellow". Also, it is assumed that index terms segmented from the document P are "red" and "white". In this case, the index term frequency of the index terms in the document d is TF (d), the index term frequency of the index terms segmented from the document p is TF(p), the document frequency of the index terms obtained from the document to be compared group P is DF(P), and the number of all the documents is 50.

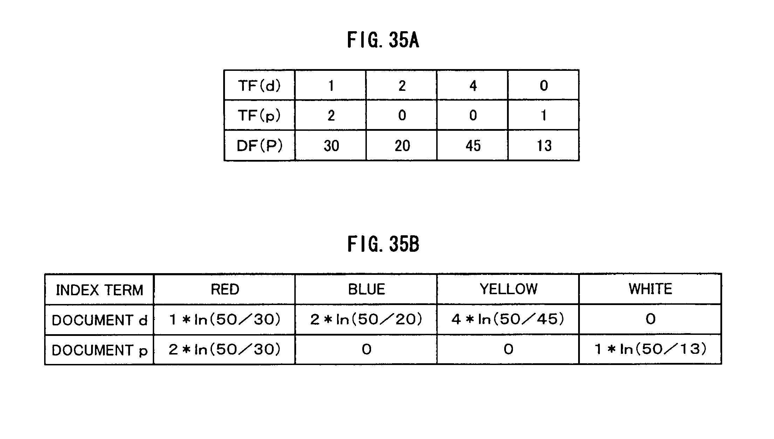

In this example,, the frequencies are shown in Fig. 35A. If TF*IDF(P) is calculated for each index term in the documents, the result as shown in Fig. 35B is obtained.

Those in the boxes in Fig. 35B represent vectors including the TF(d)*IDF(P) or TF(p)*IDF(P) of the document d or p as a component. The document vector d and the document vector p are represented as follows. Note however, the rows and columns are replaced from one another.

```
Document vector d=(1*ln(50/30),2*ln(50/20),4*ln(50/45),0)


      Document vector p=(2*ln(50/30),0,0,1*ln(50/13))
```

Then, similarity measures are calculated. More specifically, by obtaining the inner product of the document vector d and the document vector p, the similarity measure between the document vector d and the document vector p is obtained. Note that the larger the value of the similarity measure between the document vectors, the higher the degree of the similarity between the documents, and in terms of the distance between the document vectors (dissimilarity measure), the smaller the value is, the higher will be the degree of the similarity. The inner product of the document vectors is the sum of the products of the components of the vectors and can therefore be obtained as follows.

$$\text{(document vector d} \bullet \text{document vector p)}$$
$$=1*\ln(50/30)*2*\ln\ (50/30)+0+0+0$$

where the last term of the right side is "0." More specifically, the component of the inner product of index terms other than index terms (d) extracted from the document to be surveyed d, in other words, the similarity is "0" and it is only necessary that the TFIDF calculation is carried out for each of index terms (d). In other words, if there is no index term on one side, the component of the inner product is "0" and only the index terms in d are subjected to calculation, so that the amount of calculation can be reduced.

Based on the above-described similarities, with more index terms similar to the d's index terms exist in p, the component of the inner product is not zero, so that a high value is obtained as the similarity. With a smaller number of index terms similar to the d's index terms in p, the inner products of more components are zero, so that a low value is obtained as the similarity of the sum of the components.

Since there are other various kinds of methods for operating similarities, and if the similarity calculation unit 150 based on the TF(d) calculation unit 121, the TF(P) calculation unit 141 and the IDF(P) calculation unit 142 may be carried out as described. Meanwhile, it is understood that if the method of operating a similarity does not require the TF (d) calculation unit 121, the TF(P) calculation unit 141, and the IDF(P) calculation unit 142, all these units may be omitted, and only the similarity measure calculation unit 150 may be provided.

The population narrowing unit 151 is used to narrow down a population to be selected based on a selecting condition stored in the condition recording unit 310. For example, the population may be narrowed down to those by applicants with a large number of applications or a smaller number of applications conversely, special IPC, or limited fields of industry. If such narrow-down process is not necessary, the process may be omitted.

The population document S selection unit 160 selects population documents S as many as a number set in the condition from the work result storage unit 320 or directly as a result of the calculation of similarity calculation unit 150 based on the selecting condition stored in the condition recording unit 310 or from the population narrowing unit 151. For example, documents are sorted in the descending order of similarities, documents exactly as many as a necessary number in the condition are selected, and the selected documents are transferred to the work result storage unit 320 or directly to the index term (S) extraction unit 170.

The process proceeds to the map data obtaining unit 412 or the output data obtaining unit 422 directly from the output of the population document S selection unit 160, and if it is the case, it is understood that the following process is not necessary.

The index term (S) extraction unit 170 extracts index terms (S) from the population documents S obtained from the work result storage unit 320 or as the result of the population document S selection unit 160 based on the condition stored in the condition recording unit 310 and transfers the extracted index terms (S) to the work result storage unit 320 or directly to the IDF(S) calculation unit 171.

The IDS(S) calculation unit 171 carries out IDF calculation to the result of calculation from the work result storage unit 320 or directly from the index term (S) extraction unit 170 and stores the result in the work result storage unit 320 or transfers the result directly to the characteristic index term/similarity in population/frequency scatter diagram/ structure diagram/etc calculation unit 180 based on the condition stored in the condition recording unit 310.

The characteristic index term/similarity in population/frequency scatter diagram/structure diagram/etc calculation unit 180 selects population documents and index terms according to the condition stored in the condition recording unit 310 from the work result storage unit 320 or as the result of the TF(d) calculation unit 121, the result of the TF(P) calculation unit 141, the result of the IDF(P) calculation unit 142, and directly as the result of the IDF(S) calculation unit 171 as many as the necessary number written in the condition for selection or the number selected based on the result of calculation based on the condition for example in descending order of similarities or keyword importance degrees, operates the frequency scatter diagram (keyword distribution diagram) or the structure diagram, and stores the result to the work result storage unit 320.

In the recording device 3 shown in Fig. 2, the condition recording unit 310 records information such as the condition obtained from the input device 2 and sends necessary data for them based on the request from the processing device 1 or the output device 4. The work result storage unit 320 stores the result of calculation at each component in the processing device 1 and responds to the request from the processing device 1 or the output device 4 to send the necessary data respectively.

The document storage unit 330 stores the necessary document data obtained from an external database or an internal database in response to the input device 2 or the processing device 1 and provides the data in response to the request from the processing device 1 or the output device 4.

In the output device 4 shown in Fig. 2, the map creation condition read out unit 410 reads out a condition for creating a map based on the condition stored in the condition recording unit 310 and transmits the condition to the map data obtaining unit 412. The data output condition read out unit 420 reads out a data output condition based on the condition stored in the condition recording unit 310 and transmits the condition to the output data obtaining unit 422. The comment condition read out unit 430 reads out a comment output condition or an adding condition according to the condition in the condition recording unit 310 and transmits the conditions to the fixed comment acquisition unit 432. Note that the comment addition unit 432 can add a free comment.

The map data obtaining unit 412 obtains the result of the population document S selection unit 160 and the characteristic index term/similarity in population/frequency scatter diagram/structure diagram/etc calculation unit 180 stored in the work result storage unit 320 as well as the data at the document storage unit 330 based on the conditions read out by the map creation condition read out unit 410 and transmits the results to the work result storage unit 320 or directly to the map (graph/table) creation unit 415.

Using the data from the map data obtaining unit 412, the map (graph/table) creation unit 415 creates a graph, a table, a title, a legend and the like. The result is transmitted to the report creation unit 440.

Based on the condition of the data output condition read out unit 420, the output data obtaining unit 422 obtains the result of the population document S selection unit 160 and the results of the characteristic index term TF(d)IDF(S) calculation unit 180 and the like stored in the work result storage unit 320 together with the data in the document storage unit 330 and sends the results to the work result storage unit 320 or directly to the report creation unit 440.

Based on the condition of the data output condition read out unit 430, the fixed comment acquisition unit 432 obtains data from the work result storage unit 320 and the document storage unit 330 and sends the data to the comment addition unit 435 or directly to the report creation unit 440.

Based on the condition of the comment condition read out unit 430, the comment addition unit 435 prepares data to be added as a comment by an evaluator for the research data d that has been prepared directly from an external input device such as a keyboard or an OCR or prepared in advance in the internal database in the document storage unit 330 and sends the data to the work result storage unit 320 or directly to the report creation unit 440.

The report creation unit 440 obtains the conditions and data output from the map (graph/table) creation unit 415, the output data obtaining unit 422, the fixed comment acquisition unit 432, and the comment addition unit 435 directly or from the work result storage unit 320, shapes a map/data/comment into an optimum form as a paper output and creates an information analysis report. The created information analysis report is transmitted to the output unit 450.

The output unit 450 outputs the information analysis report to the recording means or communicating means. The output unit 450 has an automatic distributing function and outputs a new information analysis report periodically (such as once a month). Alternatively, such a new information analysis report is automatically distributed when the report is greatly changed from the previous one (such as when 10% or more of the content is changed).

Note that the above described report creation unit 440 can create an information analysis report only of a map and can output the result through the output unit 450.

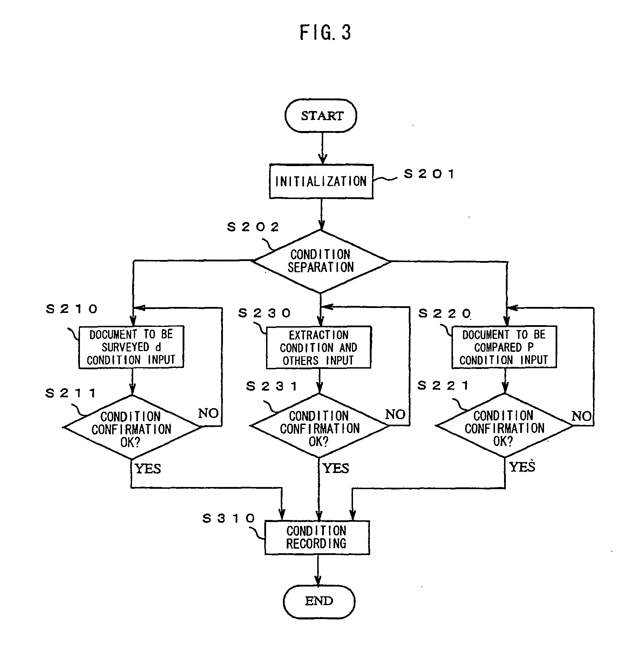

Now, with reference to Figs. 3, 4, and 5, the method for automatically creating information analysis report according to the embodiment and the procedure by the control of a program will be described.

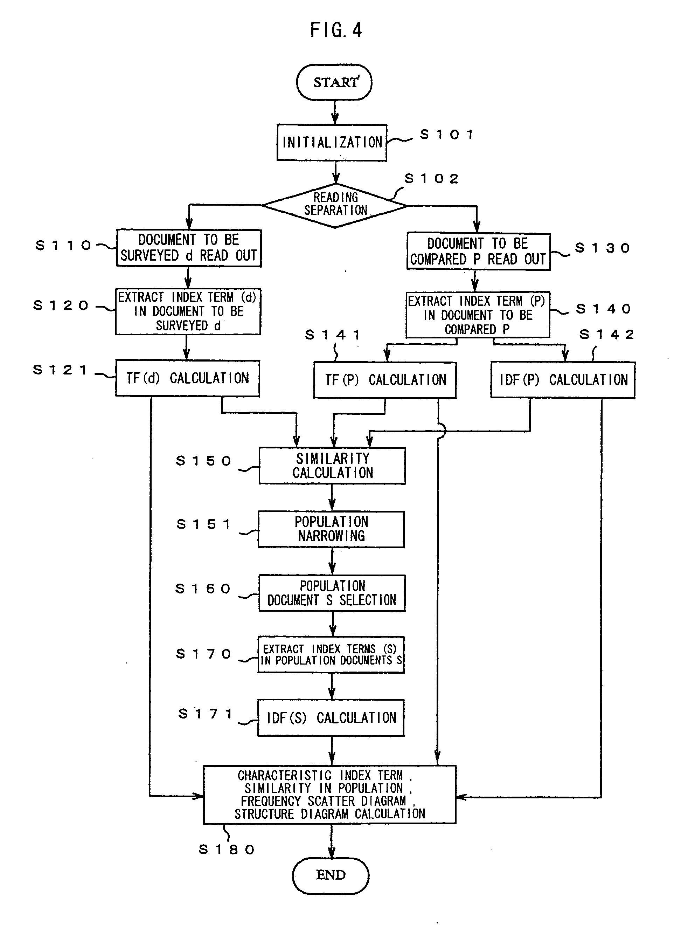

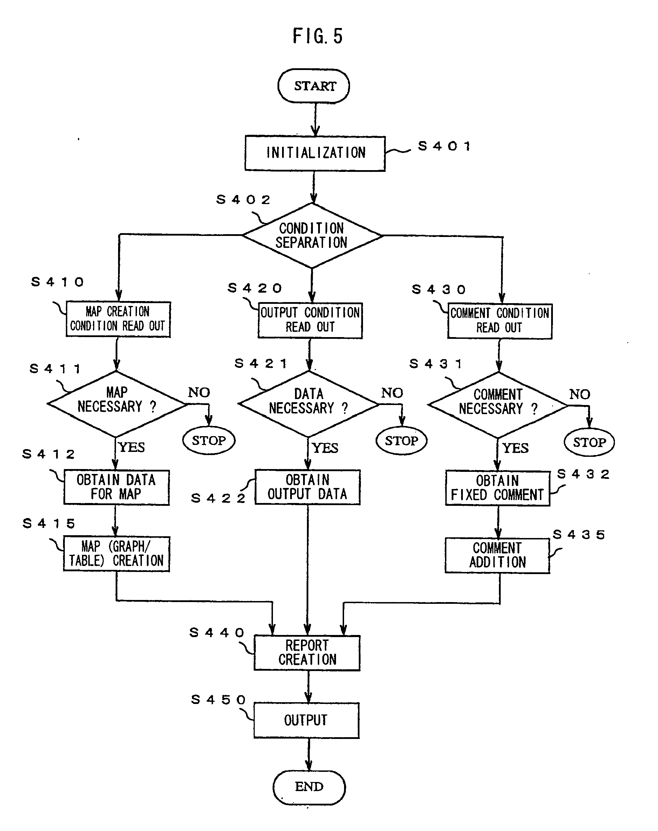

Fig. 3 is a flowchart showing the calculation of the input device 2. Fig. 4 is a flowchart showing the calculation of the processing device 1. Fig. 5 is a flowchart showing the calculation of the output device 4.

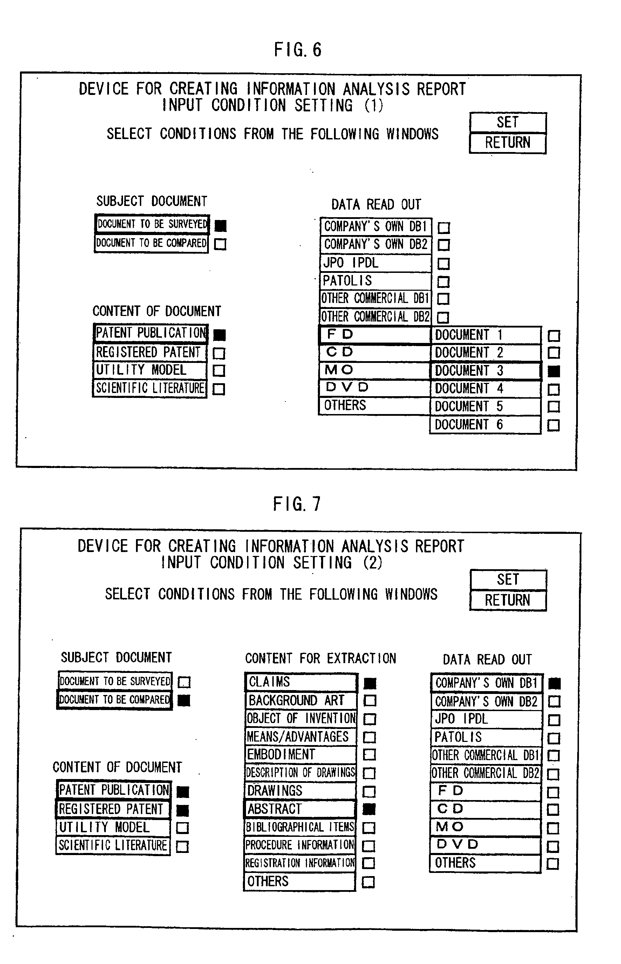

As shown in Fig. 3, when each condition is set in the input device 2, initialization is carried out in step S201 before each condition is set in the input device 2. After the initialization (step S201), conditions to be input are separated (step S202). At the time, if a condition is an input condition for a document to be surveyed d, the condition of the document to be surveyed d is input in the document to be surveyed d condition input unit 210 (step S210). Then, the display screen having the input condition displayed thereon (see Figs. 6 to 8) is checked, and if it is good, the "set" is selected and the input content is stored in the condition recording unit 310 (step S310), while if the input is not correct, the "return" is selected, so that the process returns to step S210 (step S211), and the above described calculation is repeated.

Meanwhile, if the condition in step S202 is a condition input for the documents to be compared P, the condition of the documents to be compared P is input in the document P condition input unit 220 (step S220). Then, the input condition is checked by the displayed screen (see Figs. 6 to 8). If the input is correct, the "set" is selected and the input content is stored in the condition recording unit 310 (step S310), while if the input is not correct, the "return" is selected, the process returns to step S220 (S221) and the above-described calculation is repeated.

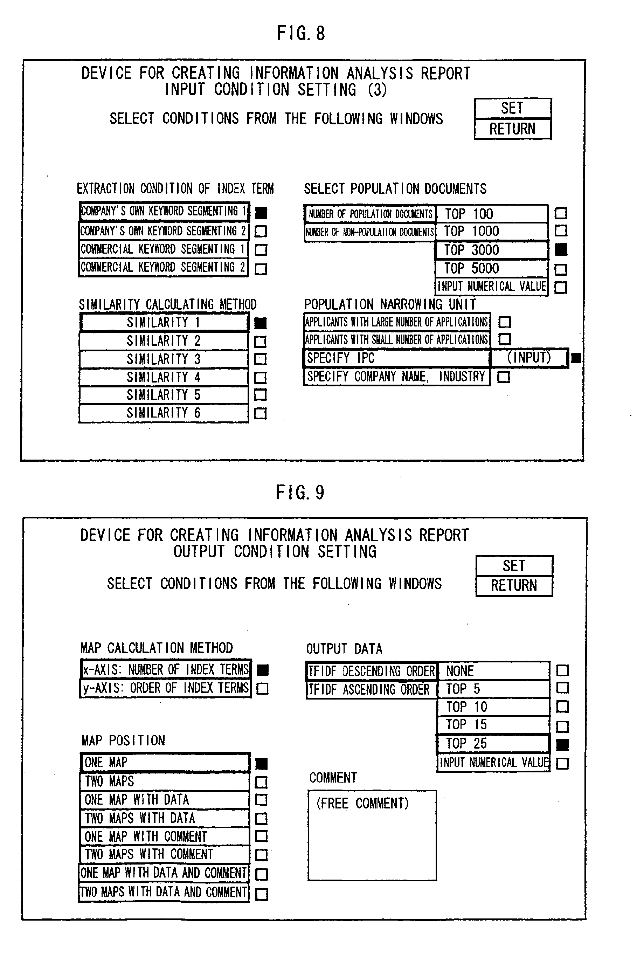

If the condition in step S202 is an extraction condition or any other condition, an extraction condition or the like is input in the extraction condition and others input unit 230 (step S230). Then, the input condition is checked by the displayed screen (see Figs. 6 to 8) and if the input is correct, the "set" is selected and the input content is stored in the condition recording unit 310 (step S310), while if the input is not correct, the "return" is selected, the process returns to step S230 (S231), and the above-described calculation is repeated. In the step S230, an extraction condition for the document to be surveyed d and an extraction condition for the population documents S from the documents to be compared P are both set. In step S230, the output condition is also set (as will be described with Fig. 9).

As shown in Fig. 4, when each kind of processing is carried out in the processing device 1, initialization is carried out in step S101. After the initialization (step S101), documents read out from the document storage unit 330 are separated between the document to be surveyed d and the documents to be compared P based on a condition in the condition recording unit 310 (step S102). If the document to be read out is the document to be surveyed d, the document to be surveyed is read out by the document to be surveyed d read out unit 110 from the document storage unit 330 (step S110). Then, index terms in the document to be surveyed d are extracted at the index term (d) extraction unit 120 (step S120). Subsequently, the extracted index terms are each subjected to TF calculation at the TF(d) calculation unit 121 (step S121).

Meanwhile, in step S102, if the document to be read is a document to be compared P, the document to be compared P is read out in the document to be compared P read out unit 130 (step S130). Then, index terms for the document to be compared P are extracted in the index term (P) extraction unit 140 (Step S140). Subsequently, the extracted index terms are each subjected to TF calculation at the TF(P) calculation unit 141 (step S141) and to IDF calculation in the IDF(P) calculation unit 142 (step S142).

Then, based on the TF (d) calculation result of the output of the TF (d) calculation unit 121, the TF (P) calculation result of the output of TF(P) calculation unit 141, and the IDF(P) calculation result of the output of the IDF(P) calculation unit 142, the calculation result for each of the index terms of the document is obtained at the similarity calculation unit 150, and the average of the index terms for example is output to be used as a similarity of the document, so that the calculation of the similarity is carried out (step S150).

If the method of calculating the similarity is not based on TFIDF, a similarity is sometimes obtained by another method from the index term (d) extraction unit 120 for the document to be surveyeds d and the index term (P) extraction unit 140 for the documents to be compared P.

Then, in step S151, the population narrowing unit removes the information of unnecessary part. Note that the step S151 may be omitted.

Then, the population document S selection unit 160 rearranges the documents operated in step S150 in the ranking of similarities, and population documents S as many as the number set in the extraction condition and others input unit 230 are selected (step S160).

These kinds of data are sometimes directly used in the map (graph/table) creation unit 415 or the report creation unit 440 in the output device 4.

Then, the index term (S) extraction unit 170 for the population documents S extracts index terms (S) from the population documents S selected in step S160 (step S170).

Then, each of the index terms (d) is subjected to IDF calculation by the IDF (S) calculation unit 171 (step 171).

Then, based on the result of the IDF (S) calculation of each of the index terms (d) in the population documents S in step S171 and the result of the TF(d) calculation of each of the index terms (d) in the document to be surveyed d in step S121, calculation regarding the characteristic index term/similarity in population/frequency scatter diagram/structure diagram etc. is carried out (step S180).

As shown in Fig. 5, when an information analysis report is created and output in the output device 4, the initialization is carried out in step S401. After the initialization (step S401), conditions read out from the condition recording unit 310 are separated into a map creation condition, a data output condition, and a comment adding condition (step S402).

. If the condition read out from the condition recording unit 310 is a map creation condition (S410) and a map is necessary by the condition (step S411), map data is obtained by the map data obtaining unit 412 from the work result storage unit 320 (step S412). Based on the map creation condition from the map creation condition read out unit 410, a map such as a graph and a table is created (step S415) and sent to the report creation unit 440.

Meanwhile, if the condition to be read out from the condition recording unit 310 is a population data output condition (step S420) and data is necessary by the condition (step S421), output data is obtained from the work result storage unit 320 by the output data obtaining unit 422 (step S422). Then, based on the data output condition of the data output condition read out unit 420, the data is output (step S423) and then sent to the report creation unit 440.

If the condition to be read from the condition recording unit 310 is a comment condition (step S430) and a comment is necessary by the condition (step S431), a frame to add a comment is prepared by the map/data/comment composite shaping output unit 440 and a comment is manually input with a keyboard or an OCR (step S435) or obtained using a comment prepared in advance in the internal database of the document storage unit 330 (step S432) and the comment is sent to the report creation unit 440.

If the condition does not indicate a map in step S411, if the condition is not a condition to output data in step S421, or the condition is not a condition to add a comment in step S431, the process ends each at the points, and the data is not sent to the report creation unit 440.

Fig. 6 is a view showing an input condition setting screen at the input device 2 of the device for automatically creating information analysis report 100.

Fig. 6 shows an example of the input condition setting (1) screen of the input device 2 in the device for automatically creating information analysis report. In the example in Fig. 6, the "document to be surveyed" is selected from the "document to be surveyed" and the "document to be compared" in the window of "subject document." Then, the "patent publication" is selected from the "patent publication," "registered patent," "utility model," "scientific literature" and the like in the window of "document content," and then the "FD" is selected from the "company's own DB1," "company's own DB2," "Patent Office IPDL," "PATOLIS," "other commercial DB1," and "other commercial DB2 "FD," "CD," "MO," "DVD," and "others" in the window of "data reading," and then the "document 3" is selected from the "document 1, " "document 2, " "document 3, " "document 4," "document 5," "document 6," and the like in "FD".

Fig. 7 is a display example of the input condition setting (2) screen in the input device 2 in the device for automatically creating information analysis report. In the example in Fig. 7, the "document to be compared" is selected from the "document to be surveyed, " and the "document to be compared" in the window of "subject document," and then the "patent publication" and the "registered patent" are both selected from the "patent publication," "registered patent," "utility model," "scientific literature" and the like in the window of "document content." Then, the "claims" and the "abstract" are selected from the "claims," "prior art," "object of invention," "means/advantages," "embodiments," "description of drawings," "drawings," "abstract," "bibliographic items," "procedure information," "registration information," and "others," and then the "company's own DB1" is selected from the same items as described above in the widow of "data reading." Based on the set condition in the input condition setting screen in the example, the document to be surveyed d condition input unit 210 and the document to be compared P condition input unit 220 are set.

Fig. 8 shows a display example of the input condition setting (3) screen in the input device 2 in the device for automatically creating information analysis report. in the example in Fig. 8, the "company's own key word segmentation 1" is selected from the "company's own keyword segmentation 1," "company's own keyword segmentation 2," "commercial keyword segmentation 1," commercial keyword segmentation 2," and the like in the window of "index term extraction condition, " and then the "similarity 1," is selected from the "similarity 1, " "similarity 2," "similarity 3," "similarity 4," "similarity 5," "similarity 6" in the window of "method of calculating similarity." Then, the "number of population documents" is selected from the "number of population documents," "number of non-population documents," or the like in the window of "population document selection, " then the "top 3000 cases" is selected from the "top 100 cases," "top 1000 cases, " " top 3000 cases, " "top 5000 cases, " "numerical value input," and the like, and the "applicants with a large number of applications," "applicants with a small number of applications," "IPC specification," and "company's name and industry specification," are selected in the window of "population narrow-down condition."
Based on the condition set in the extraction condition setting screen in the example, the extraction condition and others input unit 230 is set.

Fig. 9 shows a display example of the output condition setting screen in the input device 2 in the device for automatically creating information analysis report. In the example in Fig. 9, the "x-axis: index term number" is selected for the "x-axis" and the "y-axis: index term rank" for the "y-axis" in the window of "map calculation method. " The, the "one map" is selected from the "one map, " "two maps, " "one map with data, " "two maps with data, " "one map with comment, " "two maps with comment, " "one map with data and comment, " "two maps with data and comment" in the window of "map position," then the "TFIDF descending order" is selected from the "TFIDF descending order," "TFIDF ascending order," and the like in the window of "output data," and then the "top 20" is selected from the "none," "top 5," "top 10," "top 15," "top 20," and "numerical value input." Then, nothing is written in "(free comment) " in the frame of the widow of "comment. " In this way, the output conditions are set for the extraction condition and others input unit 230.

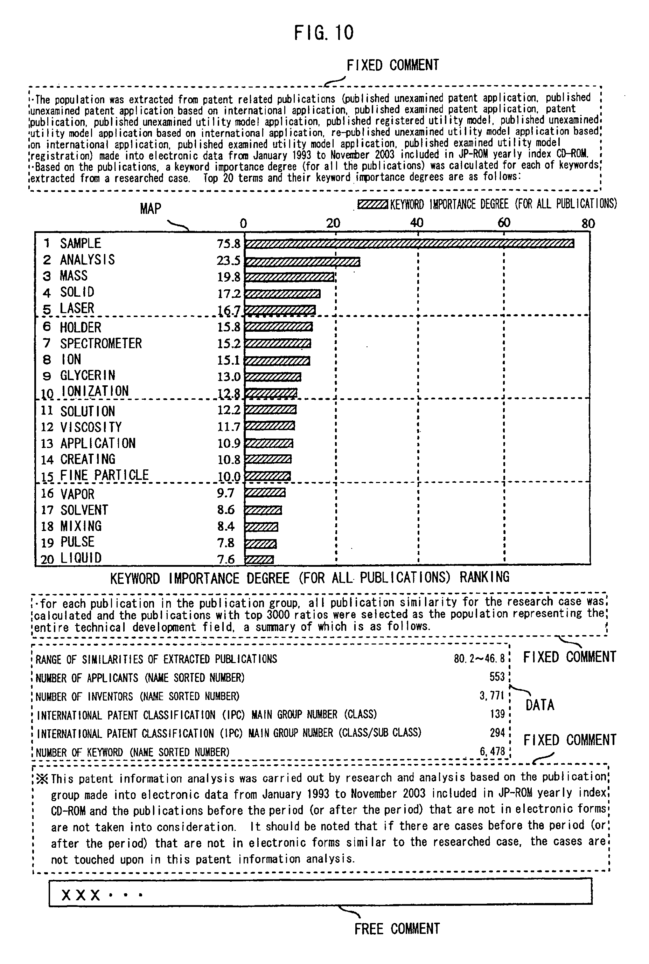

Fig. 10 shows an example of a created information analysis report when the examples shown in Figs. 6 to 9 are input in the device for automatically creating information analysis report 100. In this case, the report is created by adding data and a fixed comment to a map created by the map (graph/table) creation unit 415 based on the selecting result of the population document S selection unit 160 and the result of the characteristic index term/ similarity in population/frequency scatter diagram/structure calculation unit 180.

As can be understood from Fig. 10, in the device for automatically creating information analysis report 100, as a result of checking characteristic index terms by comparing the patent publication related to the "laser ionization mass spectrometer sample creating method and the sample holder" of the document to be surveyed d to volumes of patent laid open publications and patent publications issued for about ten years as documents to be compared and searching for characteristic index terms, "sample," "analysis," "mass," "solid," "laser," and the like are characteristic terms.

Note that in the information analyses report shown in Fig. 10, a map, data, and the contents of a fixed comment and a free comment are displayed, but the report is not limited to the above. For example, only a map may be displayed. Alternatively, a map and data may be displayed together.

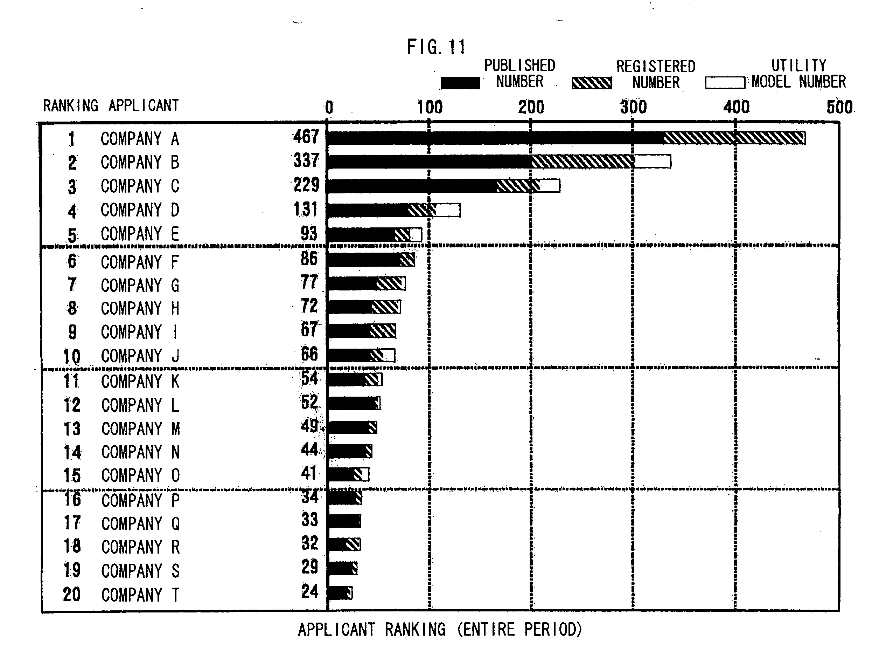

. Figs. 11 to 32 are views of other examples of the output of the device for automatically creating information analysis report 100.

Fig. 11 shows the ranking of patent applicants in all the periods. In this case, publications in the population are sorted on the applicant basis, and the applicants are displayed in the descending order of the number of patent applications filed by them. In Fig. 11, the publications in the population (for example a set of 3000 publications similar to the document to be surveyed) are sorted on the basis of applicants for the entire period of the data range of all the documents to be compared, and the top 20 applicants having larger number of publications in the population are displayed. Note that the number of applications is sorted into the number of publications, the registered number, and the utility model number for display.

From Fig. 11, the applicant ranking in the descending order of the number of publications included in the population is available, and the applicants having much interest in the field of technology are available. Based on the distribution tendency of the numbers in the ranking, it can be known whether the applicants have a high concentration (the concentration tendency by a few applicants) or a low concentration (the scattering tendency by a large number of applicants) in the field of technology.

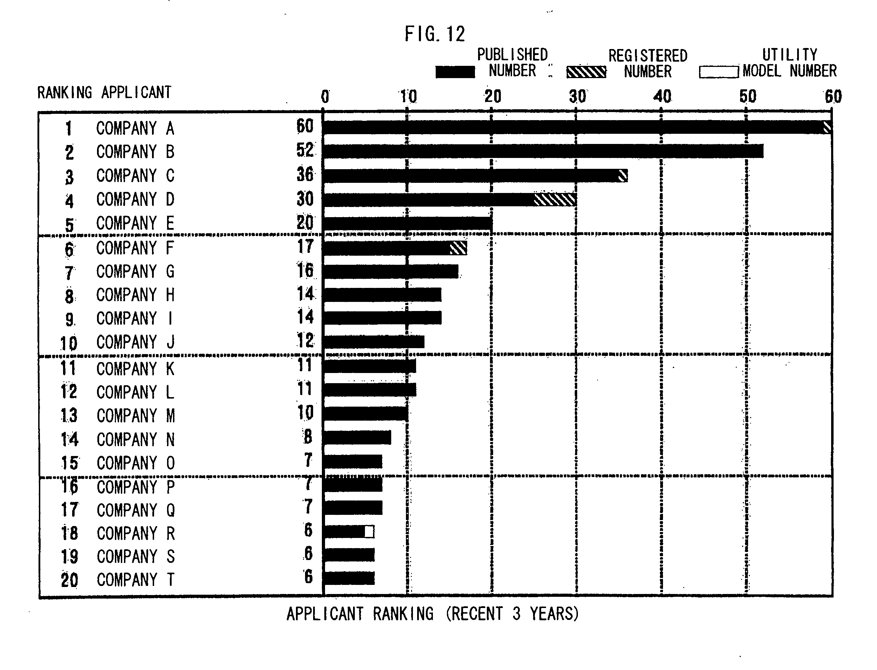

Fig. 12 shows the ranking of patent applicants in the last three years. In this case, the publications in the populations are totaled for each applicant in the past three years and applicants with a large number of filed applications are displayed. In Fig. 12, the publications in the population (for example the set of 3000 publications similar to the document to be surveyed) are summed up on the basis of applicants for the last three years and the top 20 applicants having the largest numbers of publications in the population in this period are displayed. Note that the number of applicants is divided into the number of publications, the registered number, and the utility model number for display.

From Fig. 12, the applicant ranking in the descending order of the number of publications included in the population for the last three years is available, and the applicants having much interest in the field of technology as the population are available. The applicant ranking for the last three years and the applicant ranking for the entire period (see Fig. 11) are compared, so that how the top ranking applicants are changed in their places or changes in the application number of the same applicant, in other words, changes in the interest in the field as the population is available.

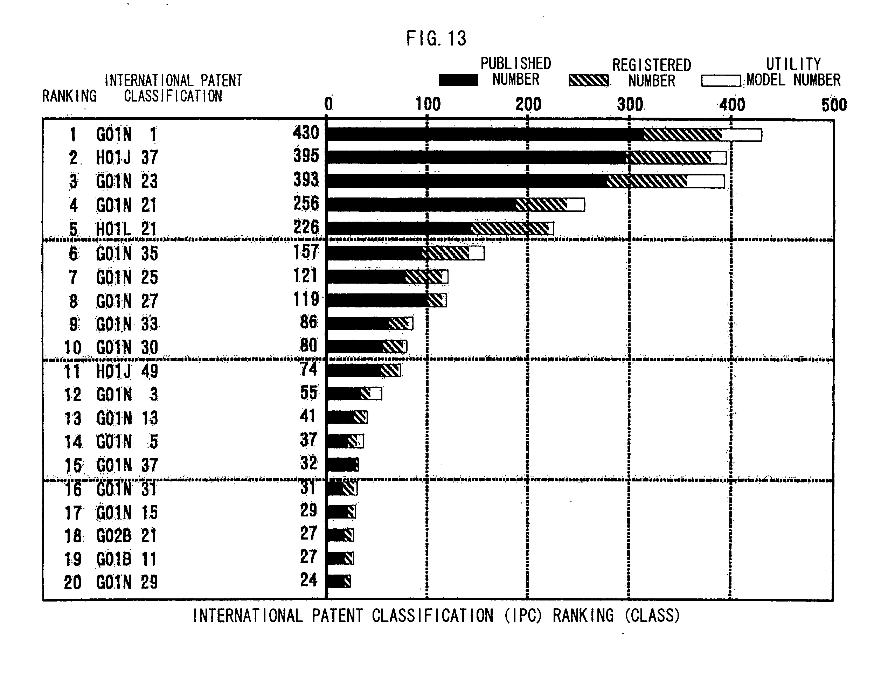

Fig. 13 shows the ranking of classes of International Patent Classification (IPC). In this case, the publications in the population are sorted on the basis of IPC classes and IPC classes with larger numbers of publications are displayed. In Fig. 13, the publications in the population (for example the set of 3000 publications similar to the document to be surveyed) are summed up on the basis of main groups in IPC classes attached to these publications, and the top 20 class ranking of the IPC main groups with larger numbers of publications are displayed. Note that the number of publications attached with IPC is displayed on the basis of the number of publications, the registered number, and the utility model number for display.

From Fig. 13, among main groups in the IPC classes attached as classes to publications related to techniques similar to the document to be surveyed, the classes with a large number of publications are known from the graph. If the number of publications greatly decreases as its class descends in the order, it indicates that the range of the field relevant to the technique similar to the document to be surveyed is narrow, while conversely if the number of publications does not greatly decrease as the class descends in the order, the range of field relevant to the technique is broad. Since the publications are summed up only regarding the main group classes in the IPC classes, the tendency would be the distribution tendency related to the main technical field of the publications similar to the document to be surveyed.

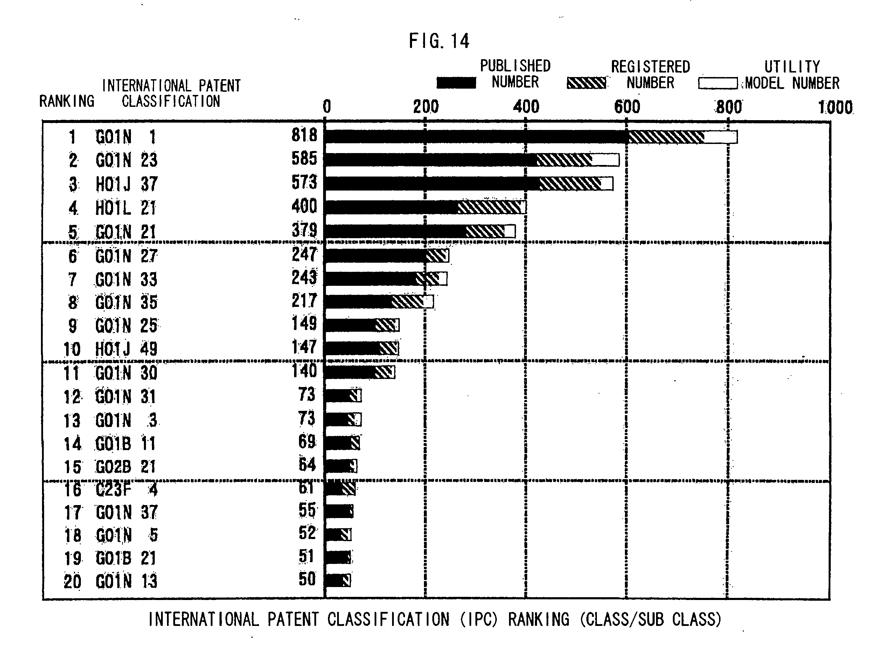

Fig. 14 shows the ranking of classes/sub classes in International Patent Classification (IPC). In this case, the publications in the population are counted on the basis of all IPC classes including the classes and the sub classes, and those with a large numbers of publications are displayed. In Fig. 14, the publications in the population (for example the set of 3000 publications similar to the document to be surveyed) are summed up on the basis of main groups in all the IPC classes including the classes and the sub classes attached to these publications, and the top 20 class ranking with larger numbers of publications are displayed. Note that the number of publications attached with IPC is displayed on the basis of the number of publications, the registered number, and the utility model number.

From Fig. 14, among main groups in the IPC classes or sub classes attached to publications related to techniques similar to the document to be surveyed, the classes with large numbers of publications are known from the graph. If the number of publications greatly decreases as its class descends in the order, it indicates that the range of the field relevant to the technique similar to the document to be surveyed is narrow, while conversely if the number of publications does not greatly decrease as the class descends in the order, the range of field relevant to the technique is broad. As compared to the ranking of the number of publications totaled based on the IPC classes (see Fig. 13), the distribution tendency of techniques relevant to techniques similar to the document to be surveyed would be expressed in a broader range.

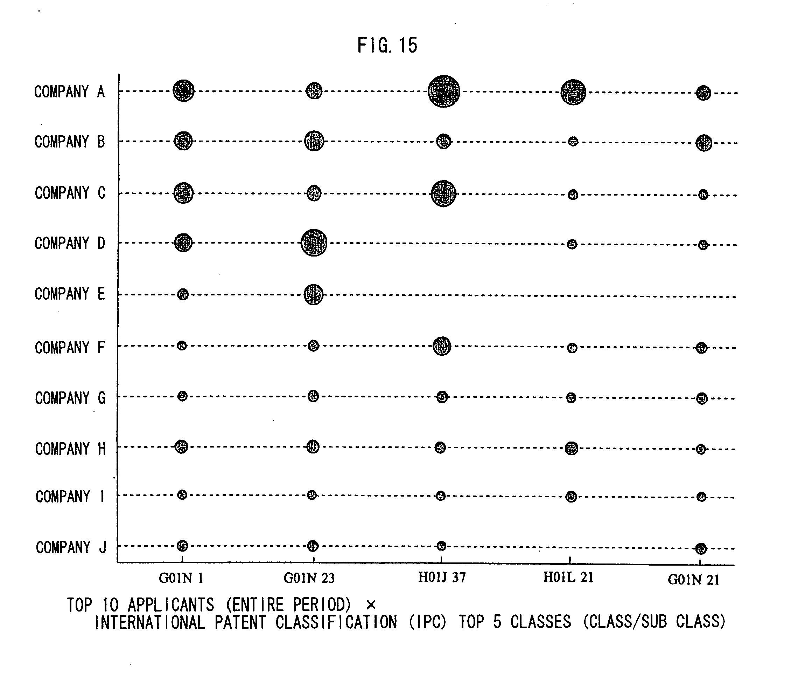

Fig. 15 shows a matrix map of applicants and International Patent Classification (IPC). In this case, among publications by the top ten applicants in terms of the number of publications in the population, the number of publications attached with any one of the top five classes in the IPC in the population. In Fig. 15, among the publications in the population of the top ten applicants with larger number of in the population (for example the set of 3000 publications similar to the document to be surveyed), the number of publications attached with IPC main groups the same as any one of the IPC top five classes attached most to the population as the classes or sub classes are totaled in the form of a matrix. The size of each bubble (circle) in Fig. 15 relatively represents the number of publications.

Fig. 15, it can be known to which class of top five class in IPC the applications of the top ten applicants in terms of the number of publications in the population belong most, or which applicant has been allowed patent in each of the top five IPC classes. Among the top ten applicants in terms of the number of publications related to the technology similar to the document to be surveyed, there is a unique tendency in the case number distribution on the basis of IPC depending on the applicant, and tendencies of technological fields in which the applicants try to solve problems or provide means therefor may be compared based on such difference.

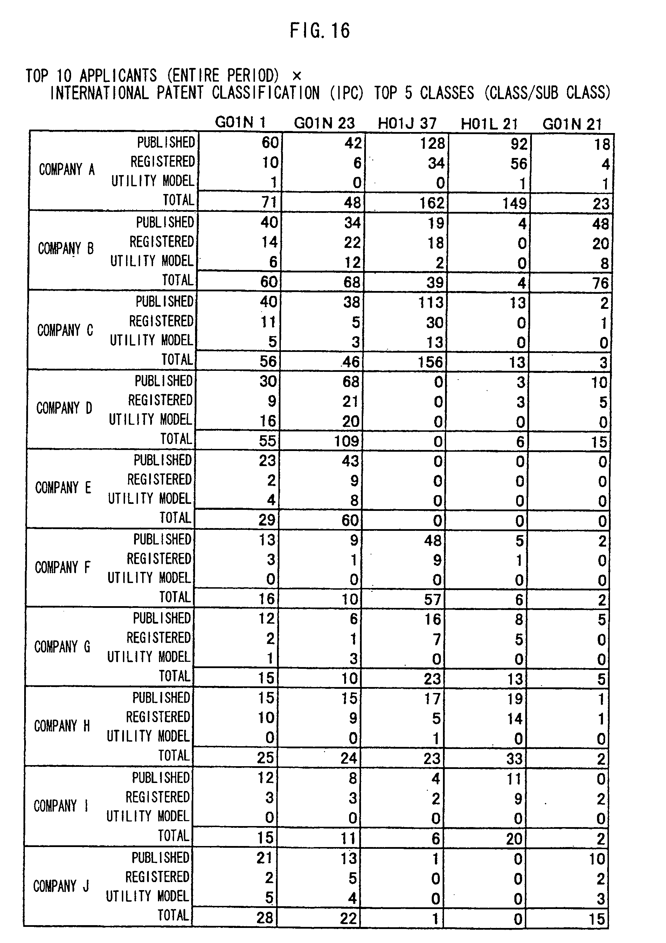

Fig. 16 is a table showing the relation between the top 10 applicants and the top five classes in International Patent Classification (IPC). In Fig. 16, the above Fig. 15 is expressed in a table form, in which the number of cases are displayed on the basis of the number of publications, the registered number, and the utility model number.

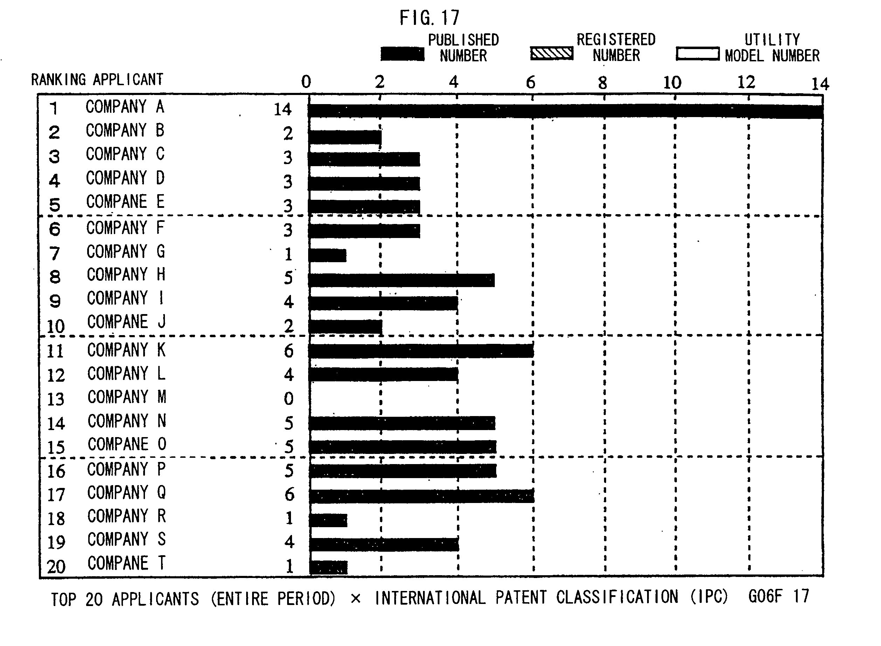

Fig. 17 shows the relation between the top 20 applicants and classes in International Patent Classification (IPC). In this case, among publications by the top 20 applicants in terms of the number of publications in the population, the number of publications attached with the same IPC main group as the IPC class of the document to be surveyed is displayed. In Fig. 17, among the publications in the population by the top 20 applicants that filed many applications among the publications of the population (for example the set of 3000 publications similar to the document to be surveyed), the number of publications attached with the same IPC main group as the IPC class of the document to be surveyed as the class or subclass is totaled and displayed. Note that the number of publications by each applicant is displayed on the basis of the number of publications, the registered number, and the utility model number.

From Fig. 17, the number of publications attached with the same main group as the IPC class of the document to be surveyed among the publications by the top 20 applicants in terms of the number of publications in the population can be obtained, so that the applicants with many publications related to the same field of technology as that of the document to be surveyed among main applicants in the population can be known.

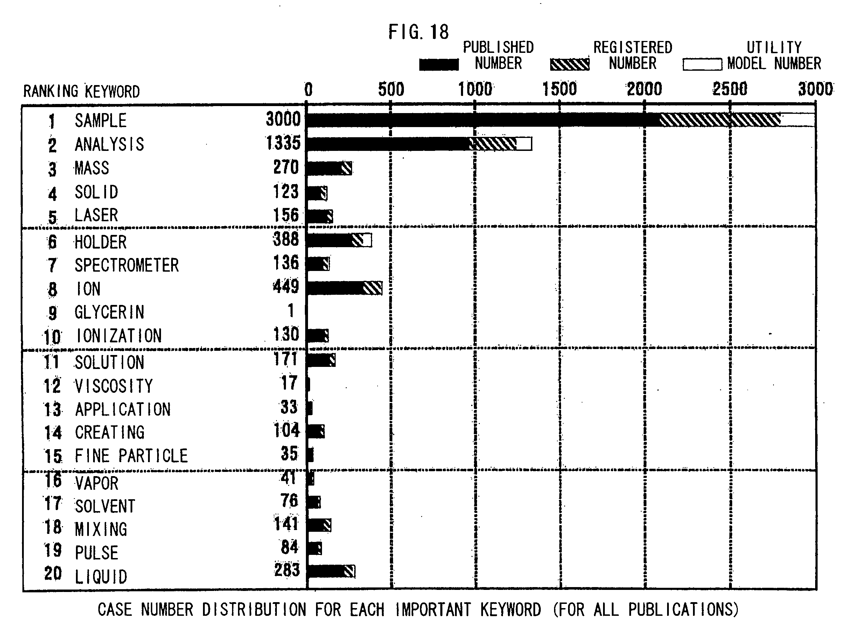

Fig. 18 shows another distribution of the publication numbers on the basis of important keywords (for all the documents to be compared). In this example, the numbers of publications in the population including the same keywords as the important keywords (for all the documents to be compared) in the document to be surveyed is displayed. In Fig. 18, the use frequency of each keyword in the document to be surveyed and the use frequency of each keyword in all the documents to be compared are quantified and compared, so that the degrees of keyword importance (for all the documents to be compared) that more significantly represent the technical characteristic of the document to be surveyed are obtained. The numbers of publications in the population (the set of 3000 publications similar to the document to be surveyed) that use the top 20 words in the descending order of importance is each summed up and displayed. Note that the number of publications that uses each keyword is displayed on the basis of the number of publications, the registered number, and the number of utility models.

From Fig. 18, for the 20 keywords (for all the documents to be compared) considered to more strongly represent the technical characteristic of the document to be surveyed, the number of publications in which each of the important keywords is used in the population is indicated. Some important keywords are used in most of the publications included in the population, and others are used only in a limited number of publications. Such an important keyword used only in a limited number of publications may represent the unique characteristic of the document to be surveyed.

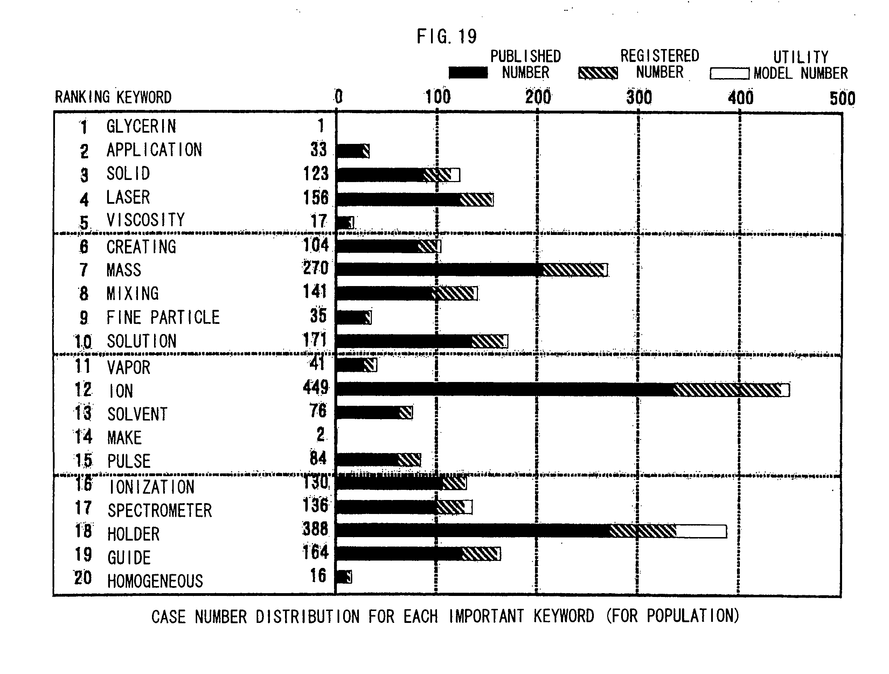

Fig. 19 shows another distribution of the numbers of publications on the basis of important keywords (for the population). In this case, the number of publications in the population including the same keywords as the important keywords (for the population) in the document to be surveyed is indicated. In Fig. 19, the use frequency of each keyword in the document to be surveyed and the use frequency of each keyword in all the documents to be compared are quantified and compared, so that the degrees of keyword (for the population) that more significantly represent the technical characteristic of the document to be surveyed are obtained. The numbers of publications in the population (the set of 3000 publications similar to the document to be surveyed) that use the top 20 words in the descending order of importance are each summed up and displayed. Note that the number of publications that use each keyword is displayed on the basis of the number of publications, registered number, and the number of utility models.

From Fig. 19, for the 20 important keywords (for the population) considered to more strongly represent the technical characteristic of the document to be surveyed, the number of publications in which each of the important keywords is used in the population is indicated. Some important keywords are used in most of the publications included in the population, and others are used only in a limited number of publications. Such important keywords used only in a limited number of publications may represent the unique characteristic of the document to be surveyed.

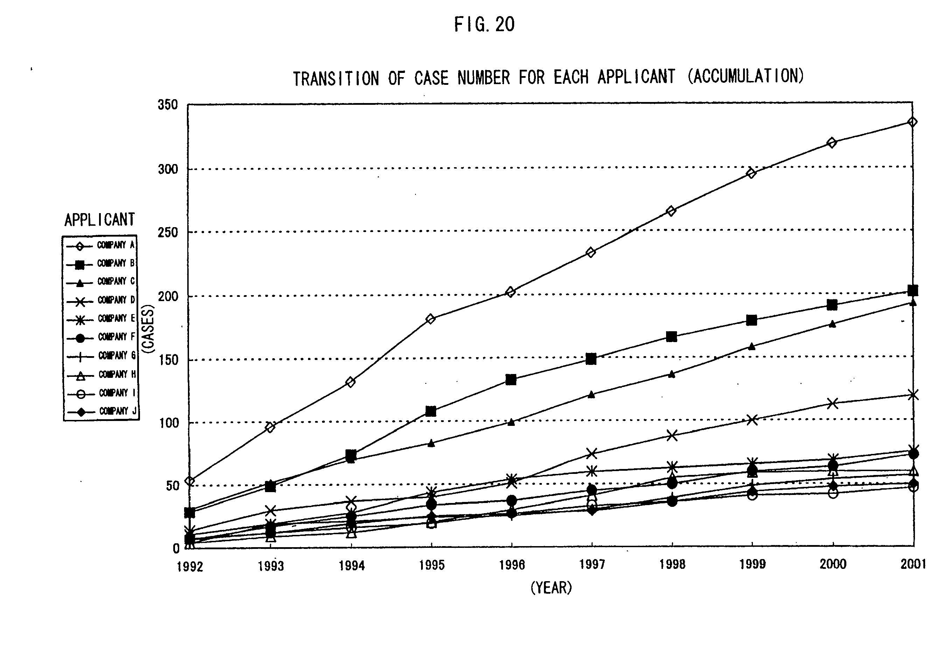

Fig. 20 shows the transition of the number of applications for each applicant. In this example, the number of applications by each of the top 10 applicants in the population is summed up for each filing year, and the transition of the number is indicated. In Fig. 20, the number of publications by each of the top 10 applicants based on the number of applications in the population (the set of 3000 publications similar to the document to be surveyed) is summed up for each filing year from year 1992 for each applicant. The numbers in and after 1993 are displayed by the accumulated numbers created by adding the numbers up to the previous year.

From Fig. 20, based on the transition of the number of applications by each of the top 10 applicants in the population, an applicant with a remarkable change tendency or change with time related to the technological field of the population can be read.

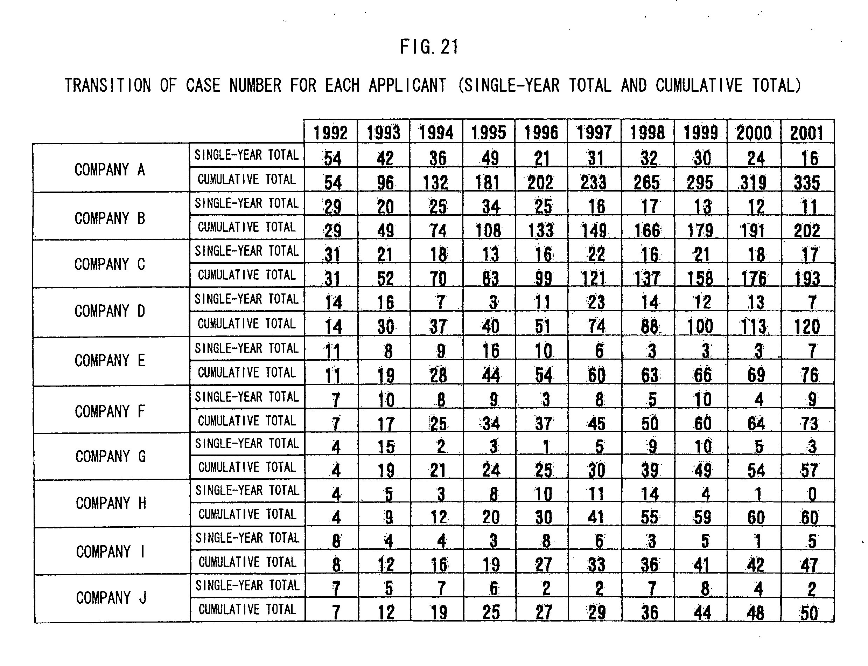

Fig. 21 is a table showing the relation between applicants and the numbers of applications. In Fig. 21, Fig. 20 described above is represented in the table form, and numbers on a single-year-basis for each summed up year are also displayed.

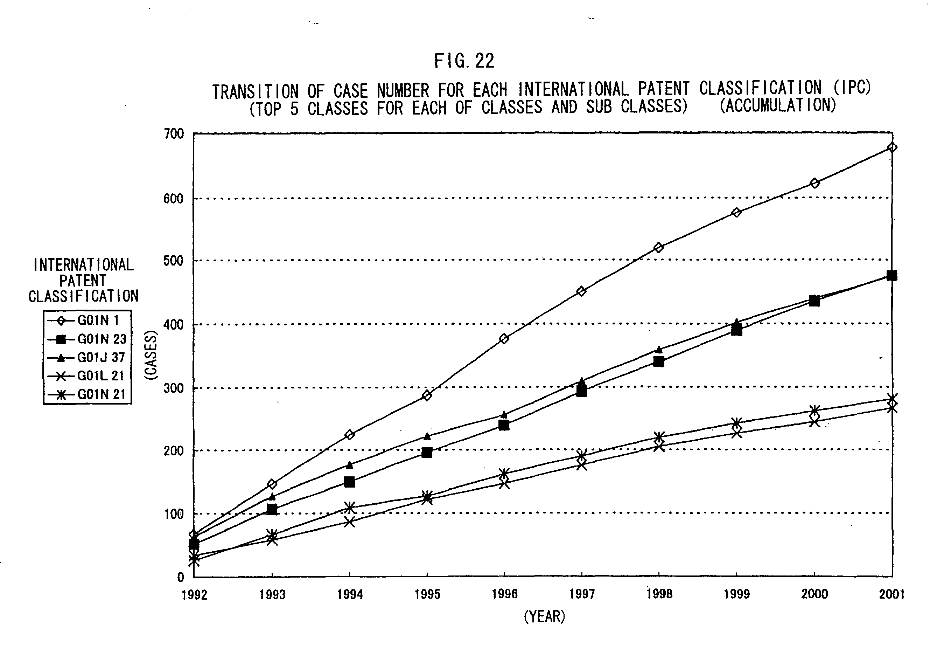

Fig. 22 is a graph showing the transition of the number for each International Patent Classification (IPC). In this case, the numbers of applications with the top five IPC classes based on the number of publication in the population are summed up for each filing year and the transition of the numbers is displayed. In Fig. 22, for the top five classes based on the number of applications in the IPC main group attached as classes or sub classes in the publications (the set of 3000 publications similar to the document to be surveyed), the applications in the population provided with them as classes or subclasses for each IPC are summed up for each filing year from 1992, and the transition of the numbers is indicated. The numbers in and after 1993 are displayed by the accumulated numbers created by adding the numbers up to the previous year.

From Fig. 22, based on the transition of the numbers of applications provided with the top five IPC classes in the population for each year, change with time related to the technical field of the population can be read.

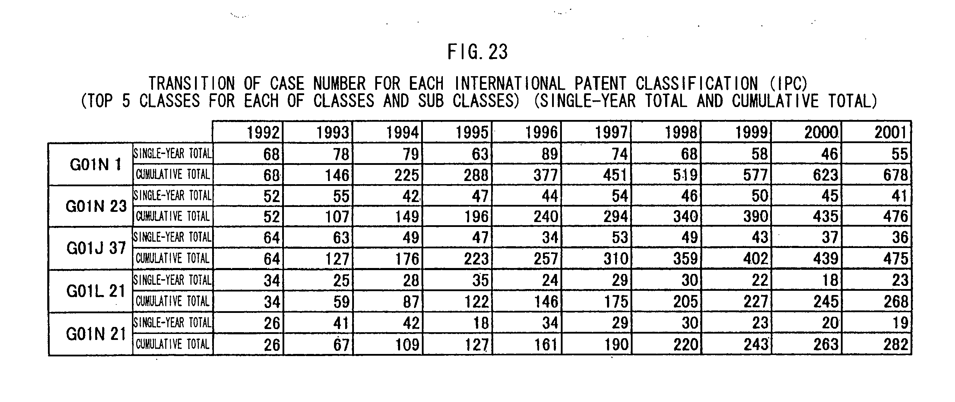

Fig. 23 is a table showing the relation between International Patent Classification (IPC) and the numbers of applications. In Fig. 23, Fig. 22 described above is expressed in the table form, and the numbers on a single-year-basis for each summed up year are also displayed.

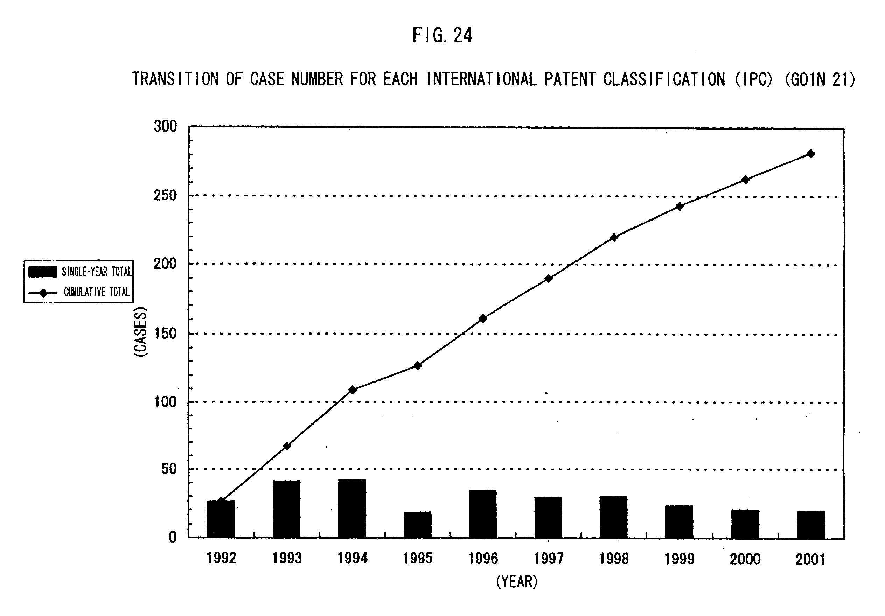

Fig. 24 is a graph showing the number transition for each of prescribed International Patent Classification (IPC) class. In this case, the number of applications provided with the same IPC main group as the class of the document to be surveyed in the population (the set of 3000 publications similar to the document to be surveyed) is summed up for each filing year, and the number transition is indicated. In Fig. 24, the applications in the population provided with the IPC main group as classes or subclasses the same as the IPC class of the document to be surveyed are summed up for each filing year from 1992, and the transition of the number is indicated. The numbers in and after 1993 are displayed in the form of a line graph by the accumulated numbers created by adding the numbers up to the previous year.

From Fig. 24, the tendency of the number of publications similar to the document to be surveyed and having the same main technical field can be obtained in time series.

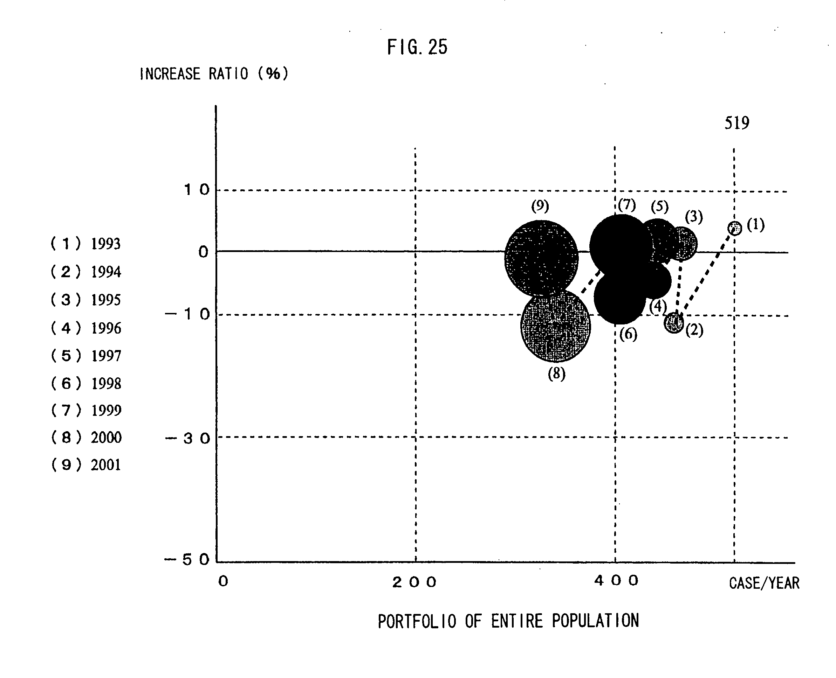

Fig. 25 is a portfolio of the entire population. In this example, the number of applications in the entire population is summed up for each filing year, and the number transition is indicated by comparison between each year and its previous year. In Fig. 25, all the applications in the population (the set of 3000 publications similar to the document to be surveyed) are summed up for each filing year from 1992, the abscissa represents the number for each summed up year (number/year), and the ordinate plots the increase ratio (%) created by comparing between each year after 1993 and the previous year starting from the number in 1992 as the origin. The sizes of the plotted circles indicate the accumulation of the numbers of applications from 1992 to the respective summed up years.

From Fig. 25, the transition of the number of publications similar to the document to be surveyed in the research period for each decades may be obtained in a schematic expression, and the state of technological development has been made for the population can be read.

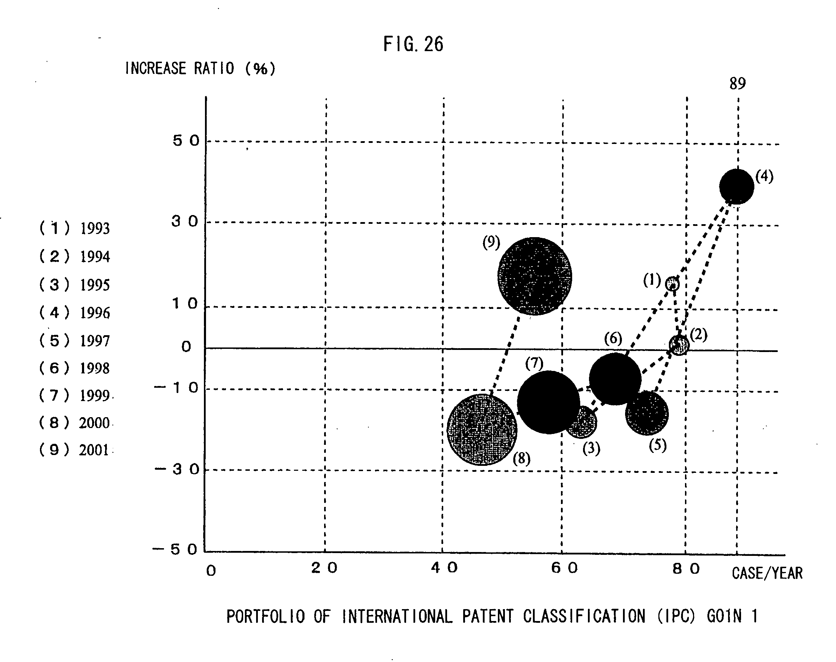

Fig. 26 shows a portfolio of International Patent Classification (IPC). In this case, the number of applications provided with IPC in the population are summed up for each filing year and compared to its previous year, so that the number transition is indicated and the state of technological development is schematically represented. In Fig. 26, IPC provided as classes or sub classes to the publications in the population (the set of publications similar to the document to be surveyed) are summed up on the basis of main groups, the applications in the population provided with the IPC main groups as classes or sub classes are summed up for each filing year from 1992, the abscissa represents the number for each year (number/year), and the ordinate plots the increase ratio (%) created by comparing the numbers of each year after 1993 and its previous year starting from the numbers in 1992 as the origin. The size of the circle of a plotting dot represents the accumulation of the number from 1992 to each year.

From Fig. 26, for applications in the population provided with IPC main groups as classes or sub classes, one important kind of IPC in the population, the yearly number transition is available in the schematic form, so that the state of technological development in the field can be read.

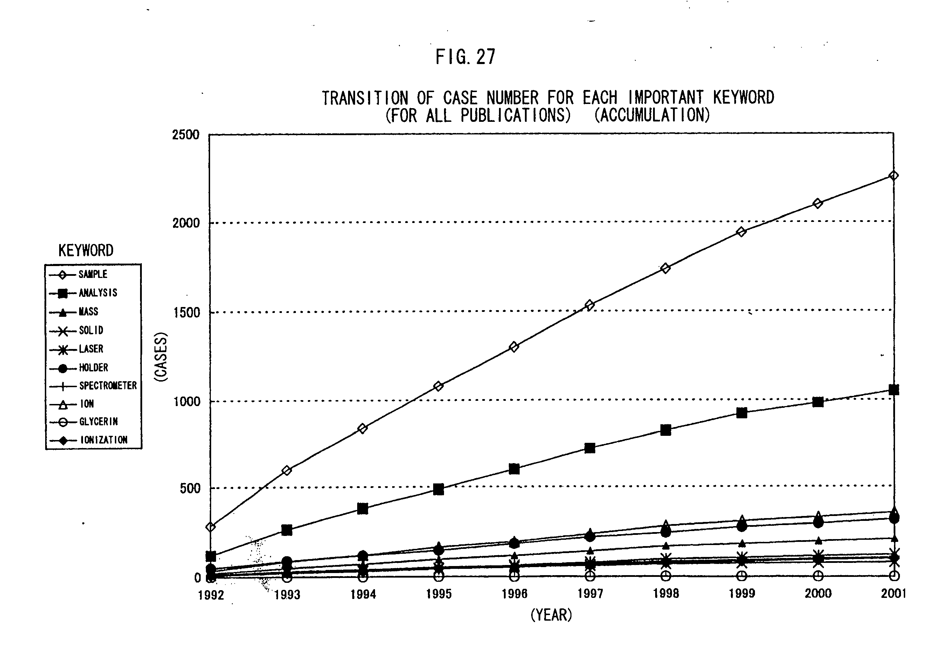

Fig. 27 shows the transition of the number for each important keyword (for all the documents to be compared: for all the publications). In this case, the transition of the application number in the population including the same keywords as the important keywords in the document to be surveyed is displayed. In Fig. 27, the use frequency of each keyword in the document to be surveyed and the use frequency of each keyword in all the documents to be compared are quantified and compared, so that the degrees of keyword importance (for all the documents to be compared) that more strongly represent the technical characteristic of the document to be surveyed are obtained. The number of applications in the population (the set of 3000 publications similar to the document to be surveyed) including the same keywords as the important keywords (for the population) is summed up for each filing year from 1992 for each keyword and the transition is displayed. The numbers in and after 1993 are the accumulated numbers created by adding the numbers up to the previous year.

From Fig. 27, the transition of the number of applications in the population including the same keywords as the keywords (for all the documents to be compared) that more strongly represent the technological characteristic of the document to.be surveyed is available.

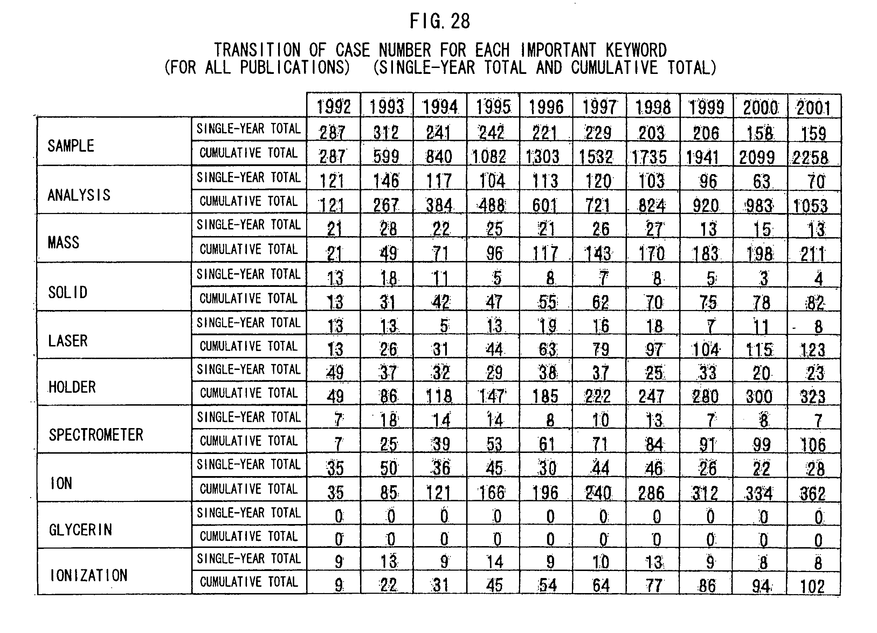

Fig. 28 is a table representing the relation between the important keywords (for all the documents to be compared) and the number of applications. In Fig. 28, Fig. 27 described above is displayed in a table form, and the number on a single-year basis in each year is also displayed.

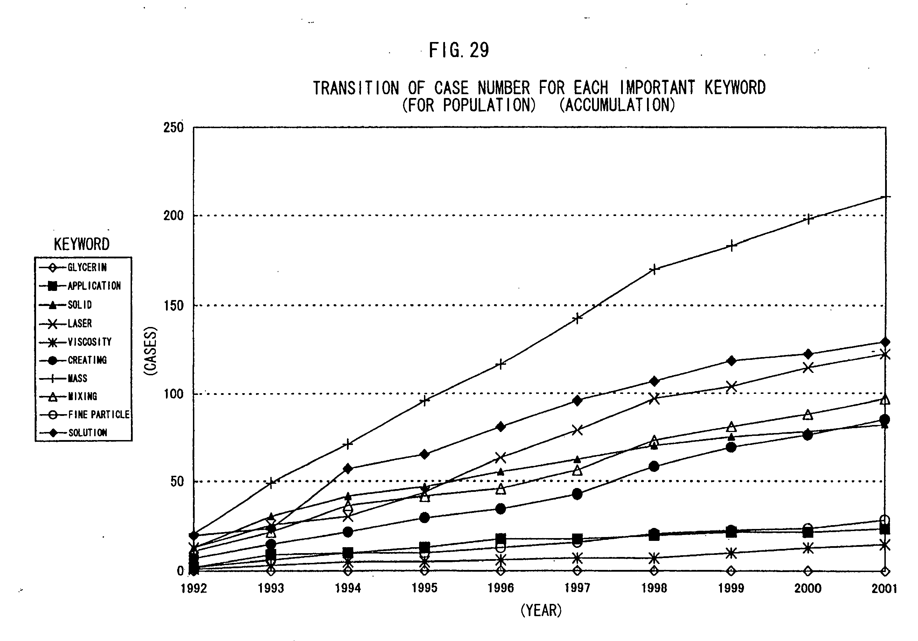

Fig. 29 shows the transition of the number of applications for each important keyword (for the population). In this case, the transition of the application number in the population including the same keywords as the important keywords in the document to be surveyed is displayed. In Fig. 29, the use frequency of each keyword in the document to be surveyed and the use frequency of each keyword in all the documents to be compared are quantified and compared, so that the degrees of keyword importance (for the population) that more strongly represent the technological characteristic of the document to be surveyed are obtained. The number of applications in the population (the set of 3000 publications similar to the document to be surveyed) including the same keywords as the important keywords (for the population) is summed up for each filing year from 1992 for each keyword and the transition is displayed. The numbers in and after 1993 are the accumulated numbers created by adding the numbers up to the previous year.

From Fig. 29, the transition of the number of applications in the population including the same keywords as the keywords (for the population) that more strongly represent the technological characteristic of the document to be surveyed is available.

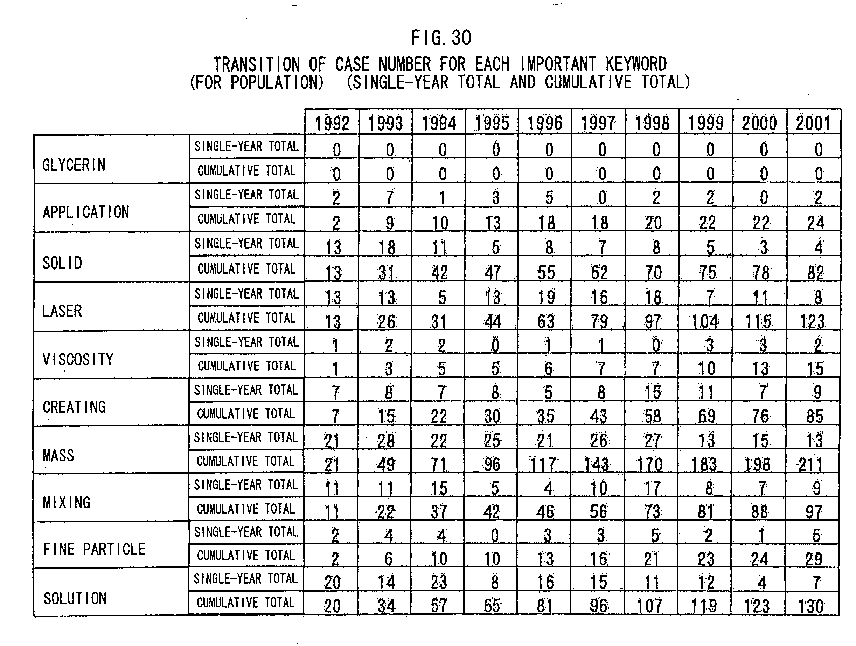

Fig. 30 is a table showing the relation between the important keywords (for the population) and the number of applications. In Fig. 30, Fig. 29 described above is expressed in a table from, and the number for each year is displayed on a single year basis as well.

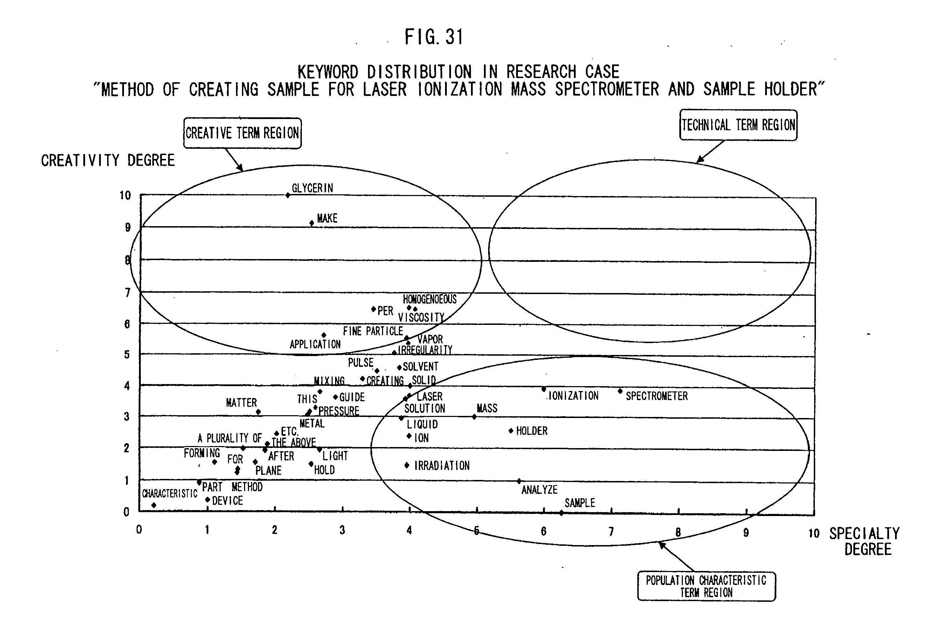

Fig. 31 is a frequency scatter diagram showing the distribution of keywords in the document to be surveyed. In this case, for each of keywords extracted from the document to be surveyed, the technicality and uniqueness are calculated and plotted into the scatter diagram in a plane having them as the axes. The way of creating the frequency scatter diagram will be described later in detail in connection with the description of a device according to a second embodiment.

In Fig. 31, as the number of publications that uses a word in all the documents to be compared is smaller, the value for its technicality increases, and as the number of publications that use a word in the population is smaller, the value for its creativity increases. Based on a region in which each keyword is plotted in the distribution map, it can be known which the word more strongly represents between the characteristic of the document to be surveyed or the population.

The following can be seen from Fig. 31.

(1) Words in the lower right region of the keyword distribution map have low creativity values and high technicality values. More specifically, the words are used in many documents in the population but used only in a small number of documents in all the documents to be compared. The words in the region should represent the characteristic of the technical field segmented as that of the population. The region is a population characteristic word region.

(2) Words in the upper left region of the keyword distribution map have low technicality values and high creativity values. More specifically, the words are used in many documents in all the documents to be compared but used only in a small number of documents in the population. The words in the region should represent the creativity of the document to be surveyed in the technical field segmented as that of the population. The region is a creative word region.

(3) Words in the upper right region of the keyword distribution map have high values both for technicality and creativity. More specifically, the words are used only a little both in all the documents to be compared and in the population. The words in the region should be very technical words little used other than in the document to be surveyed. The region is a technical word region.

(4) Words in the lower left region of the keyword distribution map have low values both for technicality and creativity. The words are therefore used in many documents in all the documents to be compared and also in many documents in the population. The words in the region should be words generally used in documents irrespective of whether they are from all the documents to be compared or the population. The region is a general word (unnecessary word) region.

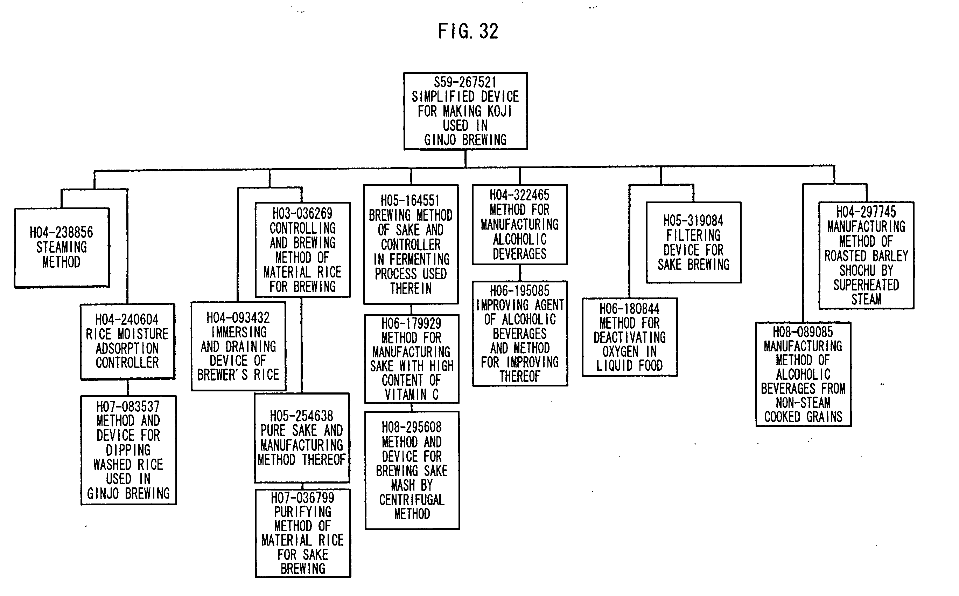

Fig. 32 shows a patent structure diagram showing the document to be surveyed or the relation between the document to be surveyed and the population. In this case, the publications of 17 Japanese applications related to *"seishu"* extracted by keyword search are each used as a document element, and those with higher similarities are placed to close to each other and analyzed in the time series of filing dates.

From Fig. 32, the relation between the document to be surveyeds or the positioning of the document to be surveyed relative to the population is easily available. It is understood that the document to be surveyeds, the documents to be compared and the population are not read, and all the process is carried out automatically by the computer.

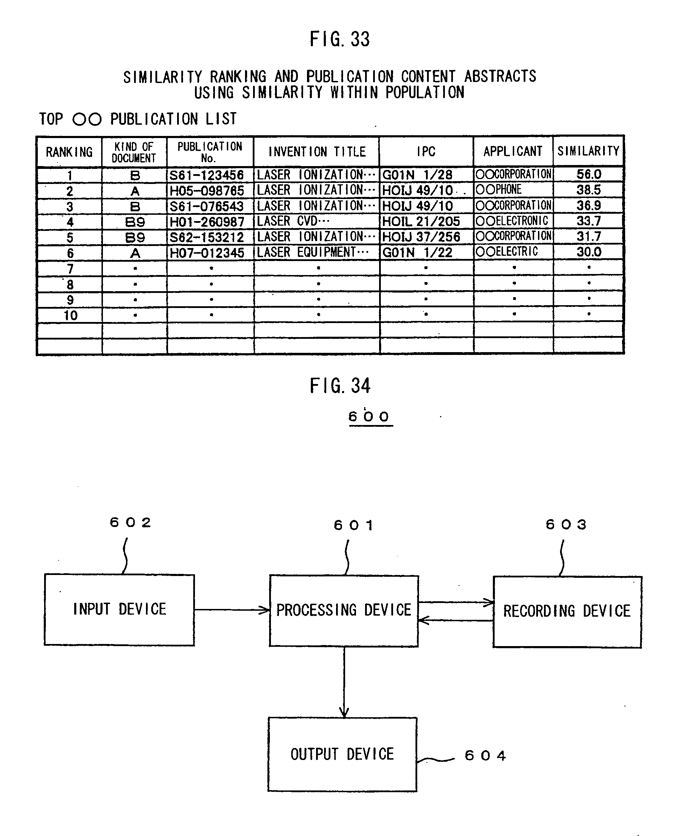

Fig. 33 shows the similarity ranking using the similarity in populations and publication content abstracts. In this case, information such as application numbers, invention titles, and applicants is displayed for the top 300 cases based on the similarity in populations. In Fig. 33, importance degrees of the keywords (for the population) in the document to be surveyed are compared, so that the inside population similarities representing the similarity measures of the publications in the population to the document to be surveyed are calculated, and information such as an application number, an invention title, and an applicant is displayed for the cases with the top 300 inside population similarities.

According to the embodiment, the device for automatically creating information analysis report 100 includes the processing device 1, the input device 2, the recording device 3, and the output device 4. When an information analysis report is created, a document to be surveyed and documents to be compared are specified and input, conditions for information analysis are input, population documents consisting of a document group similar to the document to be surveyed are selected from the documents to be compared, characteristic index terms in the document to be surveyed relative to the population documents are extracted. Then, based on the population documents and the index terms, an information analysis report representing the characteristic of the document to be surveyed is created, and the created information analysis report is output to the display means recording means, or the communicating means.

In this way, an information analysis report that can exactly report about the information of the document to be surveyed can automatically be created without human inspection of the contents of the document to be surveyed and an enormous number of documents to be compared. In addition, an information analysis report having a map, data about the population or index terms, and a fixed comment or a free comment based on the contents of the map and data can be created.

Second Embodiment

Now, a device for automatically creating information analysis report according to a second embodiment of the invention will be described. The device for automatically creating information analysis report according to the second embodiment basically has the same functions as those of the first embodiment, but the device is connected to a network in particular to carry out processing in response to a request from a client through the network and can transmit the file of an information analysis report obtained as the result of processing to the client through the network.

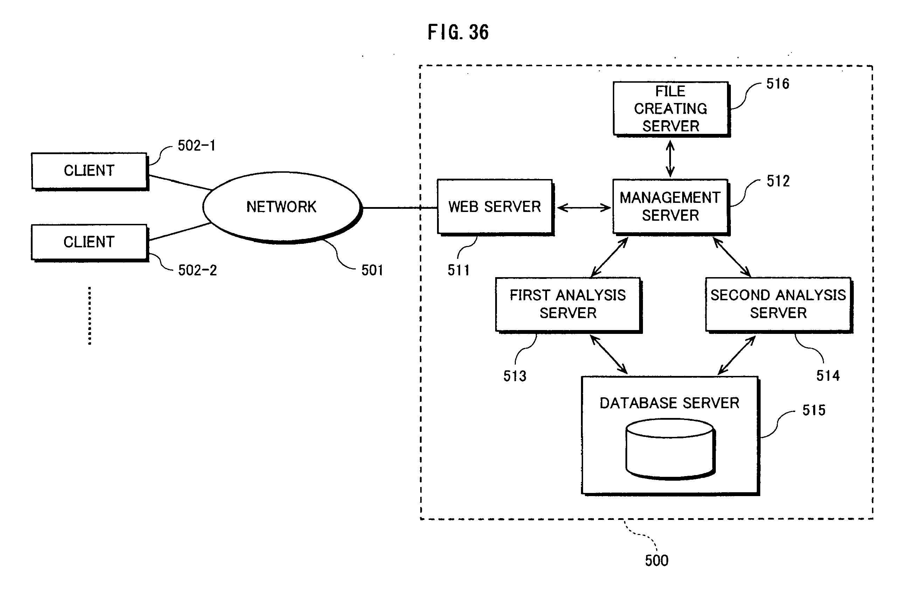

Fig. 36 is a diagram of the device for automatically creating information analysis report according to the second embodiment including clients. As shown in Fig. 36, the device for automatically creating information analysis report 500 is connected to a network 501 such as the Internet. The network 501 is connected with clients 502-1, 502-2,... . Therefore, data communication can be carried out between the device for automatically creating information analysis report 500 and the clients 502-1, 502-2,... through the network 501. Hereinafter, when any particular client is not referred to, the clients will be each simply referred to as "client 502."

As shown in Fig. 36, the device for automatically creating information analysis report 500 includes a web server 511, a management server 512 including a queuing mechanism, a first analysis server 513 that creates a structure diagram, a frequency scatter diagram or the like, a second analysis server 514 that creates cluster information, a database server 515, and a file creating server 516. According to the second embodiment, the web server 511, the management server 512, the first analysis server 513, and the second analysis server 514 as a whole carry out almost the same functions as those of the processing device 1, the input device 2 and the output device 4 according to the first embodiment. The database server 515 carries out almost the same function as that of the recording device 3 according to the first embodiment.

The web server 511 serves as an interface with the client 502 and receives/transmits data from/to the client 502. The web server 511 creates the information of a case on which an information analysis report should be created, i.e., the information of the document to be surveyed (hereinafter referred to as "research case information") based on the user input transmitted to the web server 511 from the client 502 through the network and provides the management server 512 with the created information.

The management server 512 queues research cases and requests to the first analysis server 513 and the second analysis server 514 in the order of input. The management server 512 includes a first queuing mechanism for requesting the first analysis server 513 and a second queuing mechanism that queues the research cases processed by the first analysis server and requests the second analysis server 514.

The first analysis server 513 extracts a population, carries out various kinds of totaling processing, and creates a structure diagram. The second server 514 creates cluster information representing the characteristic of each cluster in the structure diagram.

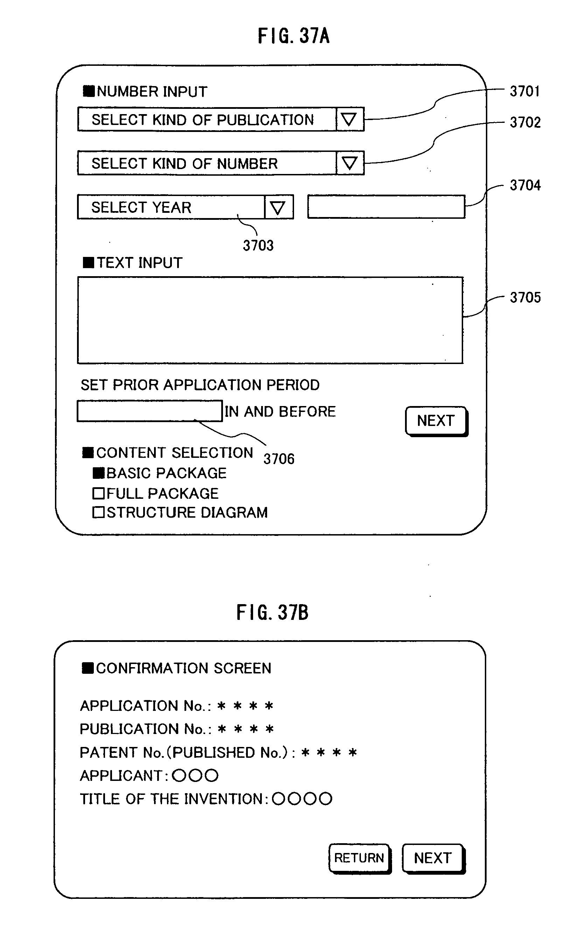

Now, processing carried out by the device for automatically creating information analysis report 500 according to the second embodiment will be described. The user operates the client 502 to log in, so that the web server 511 transmits a search screen used to specify a document to be surveyed to the client 502. Fig. 37A is a view of an example of the search screen. As shown in Fig. 37A, the search screen has boxes 3701 to 3704 used to specify a patent document, a text input box 3705, and a content selecting box. According to the embodiment, a text message input by the user may be handled as the document to be surveyed in addition to patent laid-open publications or patent publications. As a text input, a summary of a technique on which the user is to file a patent application may be input.

If the document to be surveyed is a patent document such as a laid-open publication, the user operates the client 502 and inputs necessary information in the boxes 3701 to 3704. Alternatively, the user may input information to be researched in the text input box 3705.

Note that the box 3706 is used to provide service such as emphasizing similar publications for a period based on an input in the box 3706 in a different color at the time of listing similar publications.

When the user operates the client 502 to turn on a button, information input in each box is transmitted to the web server 511 through the network 501. The web server 511 transmits a check screen to confirm the input of the user to the client 502. Fig. 37B is a view of an example of the check screen. After checking the content, the user operates the client 502 to turn on a prescribed button, so that the document to be surveyed is determined.

As described above, according to the embodiment, once a document to be surveyed is determined, the research case information is transmitted from the web server 511 to the management server 512. The management server 512 queues research cases by the first queuing mechanism, requests the first analysis server 513 to operate and provides the research case data.

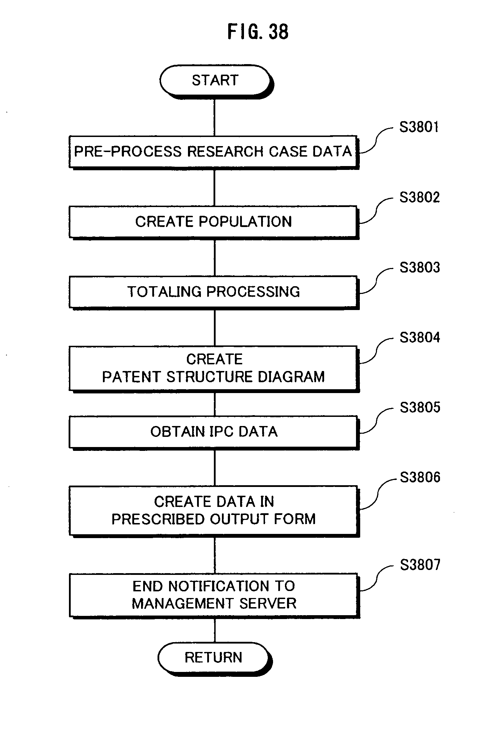

Fig. 38 is a flowchart showing processing carried out in the first analysis server. As shown in Fig. 38, the first analysis server 513 carries out pre-processing to the research case information so that the server itself can easily handle the data (step S3801) and then creates a population (step S3802).

According to the embodiment, for a patent document, its claims and abstract of a patent document constitute a document to be surveyed. If it is a text input, the input text itself is a document to be surveyed. According to the second embodiment, for example the claims and abstract in each of publications such as JP-ROM are documents to be surveyed.

As a population, 3000 cases are extracted in the descending order of similarity measures to the document to be surveyed among the documents to be compared. The similarity measures is calculated in the same manner as that described in connection with the first embodiment and therefore the description is not provided.

Note that the information of the extracted documents constituting the population or the like is stored in the recording device (not shown) in the first analysis server 513.

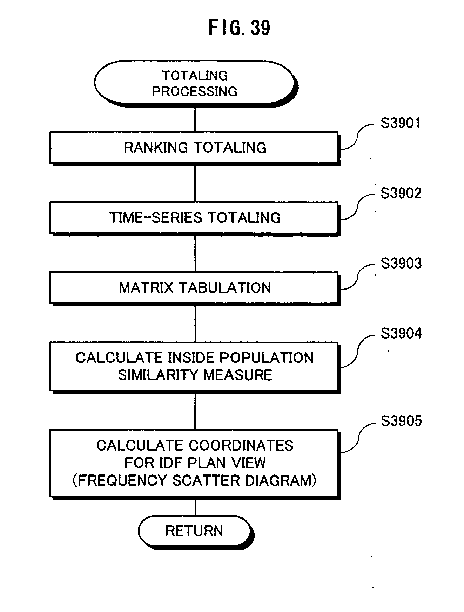

Then, the first analysis server 513 carries out totaling processing. Fig. 39 is a flowchart showing an example of the totaling processing according to the second embodiment. As shown in Fig. 39, the first analysis server 513 carries out ranking totaling (step S3901), time series totaling (step S3902), and matrix tabulation (step S3903) as the totaling processing.

The ranking totaling includes keyword totaling, applicant-related totaling, and IPC related totaling. In the keyword totaling, distribution diagrams as shown in Figs. 18 and 19 are created. The first analysis server 513 obtains information of a prescribed number of keywords (for all the publications) in the descending order of importance degrees from the recording device and creates a graph representing the number publications that use the keywords (index terms) for each of the important keywords (Fig. 18). The first analysis server 513 obtains information of important keywords (for the population) from the recording device and creates a graph representing the number of publications that use the keywords (index terms) for each of the important keywords (for the population)(Fig. 19).

The first analysis server 513 obtains the information of the population from the recording device and totals the publications of the population for each of the applicants (see Figs. 11 and 12). The first analysis server 513 obtains the information of the population from the recording device and creates a graph in which IPC classes in the publications of the population are totaled for each main group (Fig. 13) and creates a graph created by totaling them for each of the IPC classes and sub classes (Fig. 14). The totaling results (tables and graphs) are stored in the recording device in the first analysis server 513.

The first analysis server 513 obtains the information of the population from the recording device and totals the number of applications filed by the top 10 applicants based on the number of publications in the population for each filing year and creates a graph representing the number transition (Fig. 20), and creates a table (Fig. 21) representing the cumulative total of the number of applications and each single year total. The first analysis server 513 obtains the information of the population from the recording device and creates a graph in which for the top five IPC classes attached to as classes or sub classes in the publications of the population, the number of the applications are totaled for each year (Fig. 22) and a table representing each single year total and the cumulative total of the number of applications (Fig. 23). These totaling results are also stored in the recording device in the first analysis server 513.

Furthermore, the first analysis server 513 obtains important keywords (for all the publications) from the recording device and creates a graph representing the accumulation of the yearly use frequencies of the important keywords (for all the publications) (Fig. 27) and a table representing the total of the keywords on a single year basis and the cumulative total (for all the publications) (Fig. 28). The first analysis server 513 obtains important keywords (for the population) from the recording device and creates a graph representing the accumulation of the yearly use frequency of each of the important keywords (for the population) (Fig. 29) and a table (Fig. 30) representing each single year total and the cumulative total of the important keywords (for the population). These graphs and tables are also stored in the recording device in the first analysis server 513.

The first analysis server 513 creates a graph based on the totaling result of the number of applications for each year in the population in which the abscissa represents the number of applications for each year and the ordinate represents the increase ratio compared to the number of applications in the previous year (Fig. 25). In the graph in Fig. 25, the sizes of the plotted circles indicate the accumulation of the numbers of applications. Similarly, the first analysis server 513 creates a graph based on the totaling result of the number of applications provided with certain IPC (IPC main group) in the population in which the abscissa represents the number of applications for each year and the ordinate represents the increase ratio compared to the number of applications in the previous year (Fig. 26). In Fig. 26, the sizes of the plotted circles indicate the accumulation of the numbers of applications. The graph created in this way is stored in the recording device in the first analysis server 513.

Hereinafter, the matrix tabulation will be described. The first analysis server 513 further obtains the information of the population from the recording device and refers to the IPC attached to the applications of the top 10 applicants based on the number of application in the population to create the number of applications provided with the IPC groups into a table in a matrix form including the rows of applicants and the columns of IPC main groups (see Fig. 15). Using the same information in Fig. 15, a table separately showing the number of laid-open publications, the number of registered patents, and the number of utility models (Fig. 16) is also created. The first analysis server 513 obtains the information of the population from the recording device and creates a graph representing the number of applications provided with the same IPC main group as the IPC class of the document to be surveyed for each applicant in the publications by the top 20 applicants based on the number of applications in the population (Fig. 17). In Fig. 17, it is desirable to display separately the number of laid-open publications, the number of patents, and the number of registered utility models for each applicant. The result of the matrix tabulation is also stored in the recording device in the first analysis server 513.

After various kinds of totaling processing ends, the first analysis server 513 obtains the information of the population from the recording device and calculates inside population similarity measures (step S3904). The inside population similarity measure refers to the similarity (similarity measure) of the document to be surveyed relative to each of the documents that belong to the population.

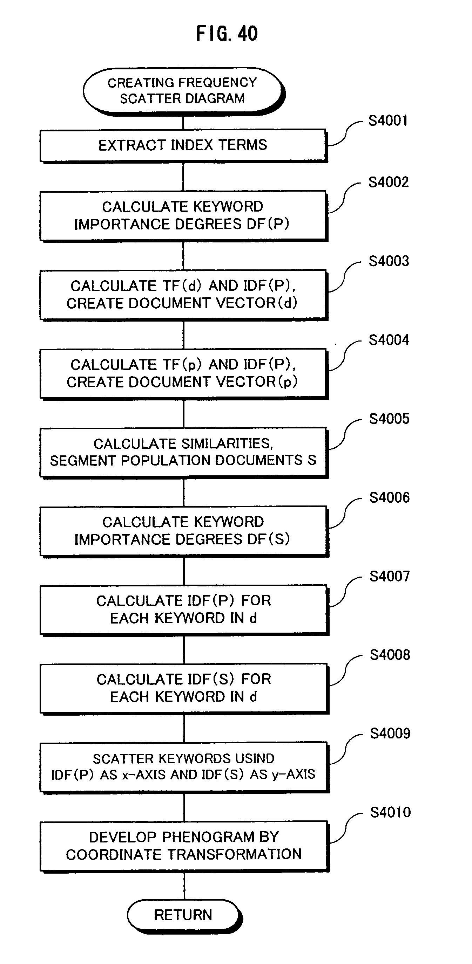

Furthermore, the first analysis server 513 carries out the process of calculating the coordinates for a frequency scatter diagram (step S3905). As shown in Fig. 31, the frequency scatter diagram represents the distribution of keywords included in the document to be surveyed. The calculation of the coordinates for each keyword in the frequency scatter diagram will be described in detail by referring to the flowchart in Fig. 40. Fig. 40 sequentially shows all the process steps necessary for calculating a coordinate for each keyword for ease of understanding. Therefore, it is not that all the process steps shown in Fig. 40 are carried out in S3905 in Fig. 39. More specifically, in S3905 in Fig. 39, a value already calculated in the first analysis server 513 and stored in the recording device is not re-calculated but used as it is and only process steps that have not been carried out before the processing in step S3905 are carried out.

As shown in Fig. 40, index terms are extracted from a document to be surveyed or documents to be compared (step S4001). Then, based on the index terms in the document to be surveyed d, the document frequencies DF(P) by the index terms in the document d in all the documents (all the documents to be compared) P are calculated (step S4002). The DF(P) corresponds to the keyword importance degree.

Thereafter, the product of TF(d) (the occurrence frequencies of d's index terms ($d_1$, ... , $d_x$) in d) and IDF(P) (the logarithm of DF(P) $\times$ the logarithm of the number of documents: $\ln[N/DF(P)]$), i.e., the document vector (d) is calculated (step S4003). Similarly, the product of TF(P) (the occurrence frequencies of P's index terms ($P_1$, ..., $P_{ya}$) in P) and IDF(P), i.e., the document vector (p) is calculated (step S4004).

When the document vector (d) and the document vector (p) are calculated, the inner product of the vectors is obtained as similarity measures (step S4005). Furthermore, a prescribed number of documents in the descending order of similarity measures relative to the document to be surveyed d are extracted from the documents to be compared P as a population S and the information of the documents is stored in the recording device (step S4005). Thereafter, the keyword importance degree DF(S) (the document frequency in S based on S's index terms) is calculated (step S4006).

Thereafter, for each of the index terms ($d_1$, ..., $d_x$) of the document to be surveyed d, the function value IDF of the document frequency is obtained for the documents to be compared P and the population S (steps S4007 and S4008). In step S4007, IDF ($d_1$;P), IDF ($d_2$; P), ..., IDF($d_x$;P) are obtained, and in step S4008, IDF ($d_1$; S), IDF($d_2$;S), ... , IDF($d_x$; S) are obtained. The first analysis server 513 creates a plane by IDF(P) and IDF(S), and for example creates a frequency scatter diagram having the index terms provided in prescribed positions on the plane where the x-axis represents the IDP (P) and the y-axis represents the IDF(S) based on the values of IDF(P) and IDF(S) for each of the index terms $(d_1, ..., d_x)$ (step S4009).

Note that from step S4009, in the frequency scatter diagram (IDF plan view), the index terms are arranged (scattered), while the scattered index terms are sometimes unevenly localized and become less viewable. Therefore, according to the second embodiment, the density of the index terms provided on the plane is inspected, and if the density in a prescribed region exceeds a prescribed value, the first analysis server 513 widens the scale on the axis in the region to expand the region and narrows the scale on the axis in the other region to compress the other region. Therefore, when a region is expanded and the other region is compressed in this way, the first analysis server 513 carries out coordinate transformation (step S4010). The IDF plan view has a rhombus shape, which can look unusual as a phenogram or can be inconvenient in handling. Therefore, the first analysis server 513 may carry out coordinate transformation, so that the plane can be represented in a square form. The information of the frequency scatter diagram is also stored in the recording device in the first analysis server 513.

After the totaling processing ends, the first analysis server 513 carries out the process of creating a patent structure diagram. Hereinafter, the patent structure diagram will be described in detail.

Patent Structure Diagram

Terms to be used in the following paragraphs are defined.

E: document element (Document elements constitute a document group to be analyzed, and individual objects to be treated as a unit for analysis according to the embodiment. According to the embodiment, the document to be surveyed d or a document p in the population corresponds to the element.)

Tree-like diagram: a diagram in which document elements constituting a document group to be analyzed are connected in a tree-like line.

Dendrogram: a tree-like diagram created by hierarchical cluster analysis. The principle of creating it will be briefly described. Based on the degree of dissimilarities (degree of similarities) between document elements that constitute a document group to be analyzed, the document elements having the smallest dissimilarity measure (largest similarity measure) are connected to form a connected body. Then, the connected body and another document element, or the connected body and another connected body are connected one after another in the ascending order of the dissimilarities between them to generate a new connected body. In this way, a hierarchical representation is formed.

For the ease of representation, the abbreviations are determined as follows.

D: the height of the position of combination (combination distance) of document elements, document element groups, or a document element and a document element group in a tree-like diagram.

$\alpha$: the height of the cutting position of a tree-like diagram

$\alpha^*$: the cutting height of a tree-like diagram created by $<D>+\delta\sigma_D$ (where $-3\leq\delta\leq3$). Note that $<D>$ is the average of all the connection heights D in the tree-like diagram, $\sigma_D$ is the standard deviation of all the connection heights D.

N: the number of document elements to be analyzed. Unlike the first embodiment, the number refers to the number of objects to be analyzed.

t: the time data of a document element. If for example the document element is a patent document, t refers to any of the filing date, the publication date, the registration date, and the priority date. If the application numbers, the publication numbers and the like are in the order of filing, publication and the like, these application numbers, the publication numbers and the like may be treated as time data. If a document element includes a plurality of documents, the average value, the median value, and the like of the time data of the documents forming the document element may be obtained as the time data of the document element.

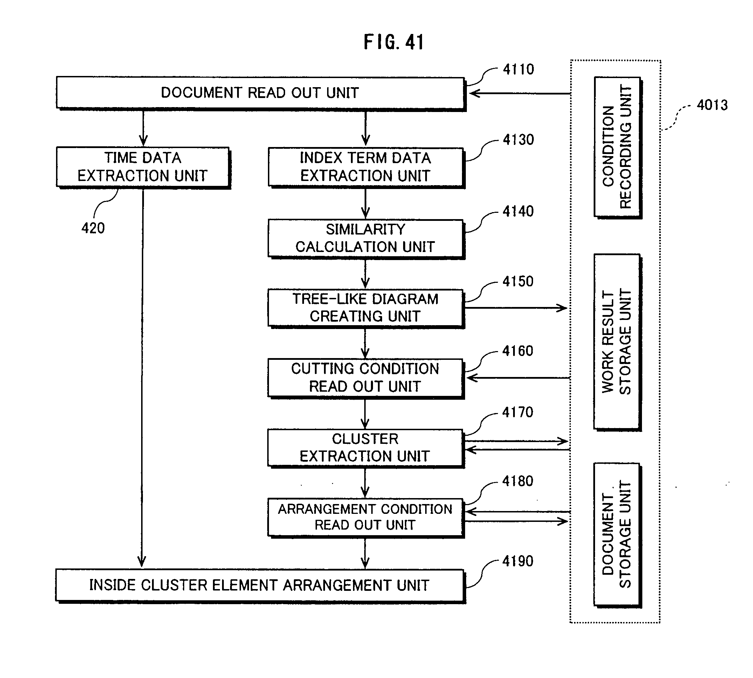

Now, a configuration used to create a patent structure diagram in the first analysis server 513 according to the second embodiment will be described. Fig. 41 is a block diagram showing the configuration used to create a patent structure diagram in the first analysis server. As shown in Fig. 41, the first analysis server 513 includes a document read out unit 4110, a time data extraction unit 4120, an index term data extraction unit 4130, a similarity measure calculation unit 4140, a tree-like diagram creation unit 4150, a disconnecting condition read out unit 4160, a cluster extraction unit 4170, an arrangement condition read out unit 4180, and an inside cluster element arranging unit 4190. The recording device 4103 includes a condition recording unit, an work result storage unit, and a document storage unit.

The document read out unit 4110 reads out a plurality of document elements to be analyzed from the document storage unit in the recording device 4103. The data of the read out document element group are directly sent to the time data extraction unit 4120 and the index term data extraction unit 4130 and used for processing therein, or sent to the work result storage unit in the recording device 4103 and stored therein.

Note that the data transmitted from the document read out unit 4110 to the time data extraction unit 4120 and the index data extraction unit 4130 or the work result storage unit may be the entire data including the time data and the content data of the read out document element group. Alternatively, the data may be only bibliographic data used to specify each of the document element group (such as an application number and a publication number for a patent document). For the latter data, if necessary in subsequent processing, the data of each document element may be read out again from the document storage unit based on the bibliographic data.

The time data extraction unit 4120 extracts the time data of each element from the document element group read out by the document read out unit 4110. The extracted time data is directly sent to the inside cluster element arranging unit 4190 and used for processing therein or sent to the work result storage unit in the recording device 4103 and stored therein.

The index term data extraction unit 4130 extracts the index term data as the content data of each document element from the document element group read out by the document read out unit 4110. The index term data extracted from each of the document elements is directly sent to the similarity measure calculation unit 4140 and used for processing therein or sent to the work result storage unit in the recording device 4103 and stored therein.

The similarity measure calculation unit 4140 operates similarity measures between the document elements based on the index term data of the document elements extracted by the index term extraction unit 4130. The calculated similarity measures are directly sent to the tree-like diagram creation unit 4150 and used for processing therein or directly sent to the work result storage unit in the recording device 4103 and stored therein.

The tree-like diagram creation unit 4150 creates a tree-like diagram of the document element group to be analyzed based on the similarity measures operated by the similarity measure calculation unit 4140 based on conditions for creating the tree-like diagram. The created tree-like diagram is sent to the work result storage unit in the recording device 4103 and stored therein. The tree-like diagram is stored for example in the form of coordinate value data of the coordinate values of document elements and the starting points and end points of individual connecting lines connecting them or in the form of data representing the connection combinations of the document elements and the positions of combination arranged on the two-dimensional coordinate plane.

The disconnecting condition read out unit 4160 reads out a tree-like diagram disconnecting condition recorded in the condition recording unit in the recording device 4103. The read out disconnecting condition is sent to the cluster extraction unit 4170.

The cluster extraction unit 4170 reads out the tree-like diagram created in the tree-like diagram creation unit 4150 from the work result storage unit recorded in the recording device 4103 and cuts the tree-like diagram based on the disconnecting condition read out by the disconnecting condition read out unit 4160, and a cluster is extracted. Data related to the extracted cluster is sent to the work result storage unit in the recording device 4103 and stored therein. The cluster data includes for example information used to specify document elements that belong to each of clusters and connection information among the clusters.

The arrangement condition read out unit 4180 reads out for example a document element arrangement condition in a cluster recorded in the condition recording unit in the recording device 4103. The read out arrangement condition is sent to the inside cluster element arranging unit 4190.

The inside cluster element arranging unit 4190 reads out the data of the cluster extracted by the cluster extraction unit 4170 from the work result storage unit in the recording device 4103 and determines the arrangement of document elements in each of the clusters based on the document arrangement condition read out by the arrangement condition read out unit 4180. The document correlation diagram according to the invention is completed by thus determining the arrangement in the cluster. The document correlation diagram is sent to the work result storage unit in the recording device 4103, stored therein, and output as required.

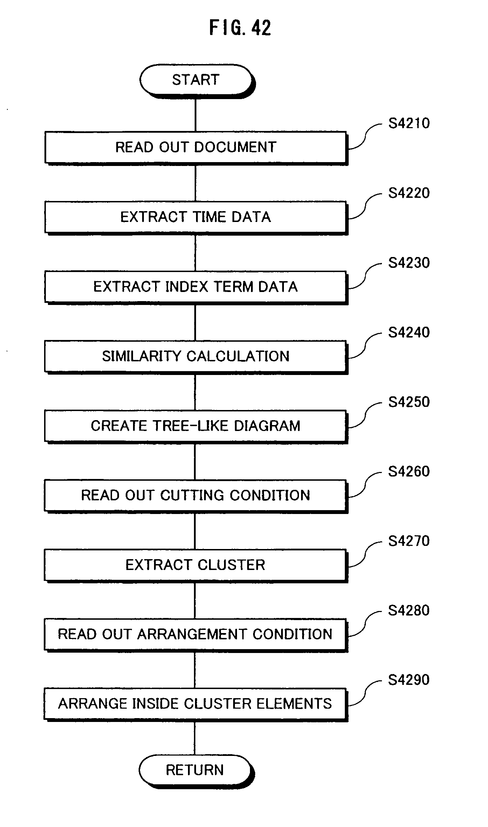

Now, with reference to the flowchart in Fig. 42, the general idea of the patent structure diagram creating process in the first analysis server 513 will be described.

The document read out unit 4110 reads out a plurality of document elements to be analyzed from the document storage unit in the recording device 4103 (step S4210). According to the second embodiment, examples of the document elements to be analyzed include population documents or a document to be surveyed and population documents.

Then, the time data extraction unit 4120 extracts the time data of each element from the document element group read out in the document reading step S4210 (step S4220).

The index term data extraction unit 4130 extracts index term data as the content data of each document element from the document element group read out in the document reading step S4210 (step S4230). The index terms are extracted in the same manner as the first embodiment.

Then, the similarity measure calculation unit 4140 operates similarity measures between the document elements based on the index term data of each of the document elements extracted in the index data extracting step S4230 (step S4240). The similarity measure (similarity) calculation has been described and therefore the description is not provided.

Then, the tree-like diagram creation unit 4150 creates a tree-like diagram of the document element group to be analyzed is created according to a tree-like diagram creating condition based on the similarity measures operated in the similarity measure operating step S4240 (step S4250). As the tree-like diagram, a dendrogram in which the similarity measures between the document elements are reflected on the height of the combination positions (combination distances) is desirably created. A specific example of a method of creating such a dendrogram includes a known Ward method.

The cutting condition read out unit 4160 then reads out a tree-like cutting condition recorded in the condition recording unit in the recording device 4103 (step S4260).

The cluster extraction unit 4170 then cuts the tree-like diagram created in the tree-like diagram creating step S4250 based on the cutting condition read out in the cutting condition reading step S4260 and a cluster is extracted (step S4270).

The arrangement condition read out unit 4180 reads out a document element arrangement condition recorded in the condition recording unit in the recording device 4103 (step S4280).

The inside cluster element arranging unit 4190 then determines the arrangement of the document elements in the cluster extracted in the cluster extracting step S4270 based on the document element arrangement condition read out in the arrangement condition reading step S4280 (step S4290). The structure diagram according to the embodiment is completed by thus determining the arrangement in the cluster. Note that the arrangement condition may be in common for all the clusters. Therefore, if step S4280 is carried out once for one cluster, the step does not have to be carried out again for the other clusters.

The process of creating the structure diagram will be described in detail. According to the embodiment, after parent clusters are extracted by cutting a tree-like diagram at a cutting height $\alpha$ determined by a certain method, a tree-like diagram is created again using only document elements that belong to each of the parent clusters, in order to divide each of the parent clusters into child clusters. At the time of creating the partial tree-like diagram, an index term dimension in which the deviation of the document element vector in the parent cluster takes a value smaller than a value determined by a prescribed method is removed before analysis.

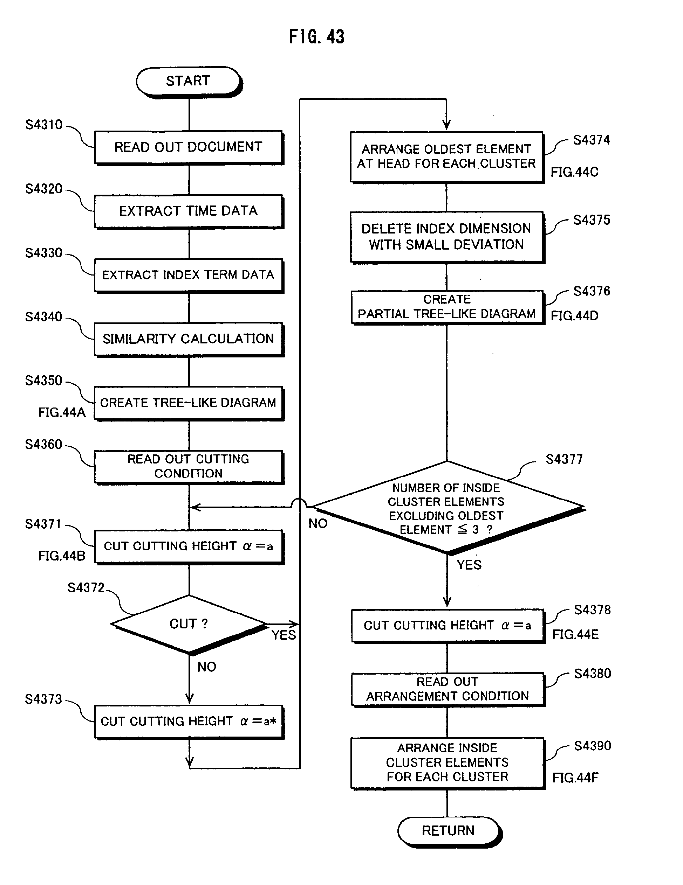

Fig. 43 is a flowchart for use in illustrating in detail the process of extracting a cluster according to the embodiment. The flowchart shows a part of Fig. 42 more in detail. Therefore, the steps the same as those in Fig. 42 are denoted by numbers created by adding 100 to the reference numbers of the steps in Fig. 42, so that the last two figures are the same as those in Fig. 42 and the same description is not repeated in some cases.

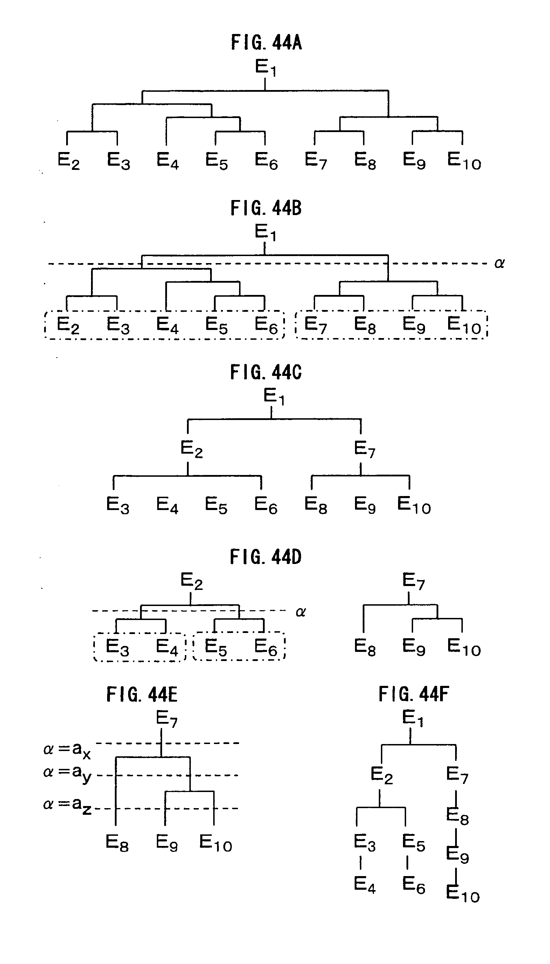

Figs. 44A to 44F show examples of a tree-like arrangement in the process of extracting a cluster according to the embodiment, and form a supplement to Fig. 43. The reference characters $E_1$ to $E_{10}$ denote document elements, and herein those with smaller suffix numbers are document elements with smaller time t (older document elements) for the ease of representation.

The document read out unit 4110 reads out a plurality of document elements to be analyzed from the document storage unit in the recording device 4103 (step S4310).

The time data extraction unit 4120 extracts time data from each document element in the document group to be analyzed (step S4320).

The time data extraction unit 4130 extracts time data from each document element in the document group to be analyzed (step S4330). At the time, the index term data of the oldest element (oldest document element) $E_1$ of the document group is not necessary as will be described, and therefore the index term data excluding the data of the oldest element is preferably extracted based on the time data extracted in step S4320.

The similarity measure calculation unit 4140 operates similarity measures among document elements (step S4340). Also at this time, similarity measures among the elements excluding the oldest element $E_1$ are operated.

The tree-like diagram creation unit 4150 then creates a tree-like diagram including the document elements of the document group to be analyzed (step S4350, Fig. 44A). At the time, the oldest element $E_1$ is arranged at the head of the three-like diagram irrespective of its similarity measure with the other elements.

The cutting condition read out unit 4160 reads out a cutting condition (step S4360). In this example, the cutting position $\alpha$, a deviation determining threshold that will be described or the like is read out.

The cluster extraction unit 4170 carries out cluster extracting. The tree-like diagram is cut at the cutting height $\alpha$ = a (step S4371, Fig. 44B). If cluster separation is not generated at $\alpha$ = a (NO in step S4372), the cutting is carried out at $\alpha^* = <D> + \delta\sigma_D$ (where $-3 \leq \delta \leq 3$, particularly preferably $0 \leq \delta \leq 2$, most preferably $\delta = 1$) (step S4373). When the tree-like diagram is cut, the oldest elements $E_2$ and $E_7$ in each cluster are arranged at the head of the cluster (step S4374, Fig. 44C). The following processing is carried out for the document element group other than the oldest elements in each of the clusters.

For each of the clusters, an index term dimension in which the deviation between the elements in the cluster other than the oldest elements is a value smaller than a value determined by a prescribed method is removed (step S4375). Assume for example that in the cluster having a document element $E_2$ as the head in Fig. 44C, index terms in the document elements $E_3$, $E_4$, $E_5$, and $E_6$ and the component values of the document element vectors created for the index terms are as shown in the following Table 1.

Table 1

| Index terms of each document element and vector component values | | | | | | |
|---|---|---|---|---|---|---|
| index term | $E_3$ | $E_4$ | $E_5$ | $E_6$ | average | standard deviation |
| $w_a$ | 30 | 20 | 20 | 30 | 25 | 5 |
| $w_b$ | 90 | 90 | 80 | 80 | 85 | 5 |
| $w_c$ | 10 | 10 | 20 | 20 | 15 | 5 |
| $w_d$ | 70 | 70 | 100 | 100 | 85 | 15 |
| $w_e$ | 12 | 10 | 12 | 10 | 11 | 1 |
| $w_f$ | 30 | 40 | 40 | 30 | 35 | 5 |

If for example the threshold for determining the deviation is specified to 10% in the ratio of the standard deviation relative to the cluster inside average, the index terms $w_b$ and $w_e$ are determined as having small deviations and removed.

Then, for each cluster, a partial tree-like diagram including the inside cluster elements other than the oldest element is created (step S4376, Fig. 44D). In the example of Table 1, using the remaining index terms $w_a$, $w_c$, $w_d$, and $w_f$, the partial tree-like diagram is created. Therefore, a cluster inside branch different from the branch in the three-like diagram created in step S4350 is obtained. Since the index term dimension having a small deviation value is removed, the difference between the remaining index terms is emphasized. Therefore, for the similarity measures between the same document elements, the similarity measures at the time of creating the partial tree-like diagram in step S4376 is evaluated as being smaller than the similarity measure at the time of creating the tree-like diagram in step 4350.

Now, for each cluster, the number of inside cluster elements excluding the oldest element is obtained and compared to a prescribed threshold (such as "3") (step S4377). Like the document elements $E_3$ to $E_6$ in Fig. 44D, if the number of document elements excluding the oldest element $E_2$ exceeds the threshold (NO in step S4377), the process returns to step S4371, the tree-like diagram is cut, and a descendant cluster is extracted. At the time, the cutting height $\alpha$ (or $\alpha^*$) is as described in conjunction with step S4371 (or step S4373), while the index term dimension having a small deviation value is removed, so that the similarity measure is evaluated as being small, and therefore a tree-like diagram can be cut again at the same height $\alpha$ (or $\alpha^*$).

Note that if cutting is carried out at the cutting height $\alpha^*$ in step S4373 at the time of extracting a descendant cluster, $\alpha^*$ may be updated depending on the height D of each combination position in a parent cluster to be cut (variation method) or the initial value of $\alpha^*$ may be used (fixed method).

Like the document elements $E_8$ to $E_{10}$ in Fig. 12D, if the number of document elements excluding the oldest element $E_7$ in the cluster is not more than the threshold (YES in step S4377), the cluster is finally cut the cutting height $\alpha = a$ (step S4378, Fig. 44E). In step S4378, the process proceeds to step S4380 if cluster separation is not actually generated.

In step S4380, the arrangement condition read out unit 4180 reads out the arrangement condition in the cluster. The inside cluster element arranging unit 4190 determines the arrangement of the document element group in the cluster according to the arrangement condition based on the time data of each document element (step S4390, Fig. 44F).

For example in step S4378, if cutting is carried out at the cutting height $\alpha = a_x$ in Fig. 44E and cluster separation is not generated, the arrangement is in a serial-chain arrangement in the order of the time data of the document elements $E_7$ to $E_{10}$ (Fig. 44F).

For example in step S4378, if cutting is carried out at the cutting height $\alpha = a_y$ in Fig. 44E, the document element $E_7$ branches to the document element $E_8$ and the serial chain of the document elements $E_9$ and $E_{10}$ in the order of time data (not shown).

For example in step S4378, if cutting is carried out at the cutting height $\alpha = a_z$ in Fig. 44E, the document element $E_7$ branches to three branches, the document element $E_8$, the document element $E_9$, and the document element $E_{10}$ (not shown).

The arrangement condition in the cluster is preferably in the order of occurrence based on the time data in this example, while other arrangements may be applied.

Note that in the example used for describing the threshold for determining the deviation, the ratio of the standard deviation relative to the average is 10%, but this is a preferable example in which the document elements each include one document. The determination threshold for the document elements each including one document is preferably in the range from 0% to 10%. Meanwhile, if each document element includes a plurality of documents, and the ratio of the standard deviation relative to the average of the inside cluster document elements is 60% or not more than 70%, the case is preferably treated as having a small deviation.

The first analysis server 513 carries out the above described processing, so that a patent structure diagram as shown in Fig. 32 can be obtained. Once the production of the patent structure diagram is completed (step S3804 in Fig. 38), the first analysis server 513 obtains IPC data (step S3805), and forms the result of processing stored in the recording device (such as a totaling result, a frequency scatter diagram, and a patent structure diagram) into a file in a prescribed form (such as a Zip file) (step S3806). Thereafter, the first analysis server 513 notifies the management server 512 of the end of the processing (step S3807).

Upon receiving the notification of the end of processing from the first analysis server 513, the management server 512 input the research cases by a queuing mechanism, issues a request to the second analysis server 514 about a research case to be processed next in the order, and provides information about the research case data and the patent structure diagram.

Creating Cluster Information

Now, the processing for obtaining cluster information will be described.

The first analysis server 513 calculates the importance degree of each keyword based on the use frequency of the keyword (index term) in the document to be surveyed and the use frequency of the keyword (index terms) in all the publications. The keywords with importance degrees in a prescribed top range are determined as important keywords. The importance of the keywords or the important keyword information is also stored in the recording device in the first analysis server 513.

The use frequency of each keyword in the document to be surveyeds and the use frequency of each keyword in all the publications are quantified and compared, and the degree of how strongly each keyword express the technical characteristic of the document to be surveyed is calculated as the "importanc.e degree" of each keyword. Keywords with higher importance degrees more strongly express the characteristic of the document to be surveyed, and therefore the keywords with importance degrees in a prescribed high range will be referred to as important keywords. Now, the definition of terms and abbreviations used in the following paragraphs will be described. Cluster information includes titles, the number of publications, the total of IPC classes (top five), the total of applicants (top five) and cluster important keywords for each cluster. The important keywords represent the ten most important keywords extracted from all the publications that belong to the cluster and the keywords are divided into the following four kinds.

Technical Region Terms: Terms used in common for other clusters among the cluster important keywords. Such keywords used in common among many clusters are generally keywords that represent the technical region to which the clusters belong.

Main Terms: Among the cluster important keywords excluding the "technical region terms," those particularly much used in the cluster. The main terms are not much used in other clusters, and often represent the main technical elements of the cluster. The main terms typically distinguish the cluster from other clusters.

Characteristic Terms: It is often the case that the cluster important keywords excluding the "technical region terms" and the "main terms" are keywords related to means or structures. Among all, general terms relatively often used but not much used in the group of publications to be analyzed (with the top 300 similarity measures in all the publications) would be keywords that could suggest characteristic aspects in means or structures. Such keywords are calculated according to a prescribed standard and indicated as "characteristic terms."

Other Important Terms: Terms that do not correspond to any one of the above three kinds among the cluster important keywords. It is often the case that "the other important terms" are technical terms relate to means or structures that do not belong to any of the above-three aspects.

Now, the process of extracting such important keywords and obtaining keywords that belong to these kinds will be described. In the following description, as for abbreviations, the parameters used in conjunction with the first analysis server 513 according to the first and second embodiments are denoted by other abbreviations in some examples, while the previously used abbreviations are used in different contexts. Therefore, it is noted that the abbreviations in the following paragraphs are applied only in these paragraphs.

High Frequency Terms: A prescribed number of terms among index terms whose high occurrence frequency in a document group to be analyzed is included in evaluation and have a large weight. For example, such terms are extracted by calculating a function value including GF(E) as the weight of an index term or GF(E) as a variable and extracting a prescribed number of terms with large values therefor.

E: A document group to be analyzed. As the document group E, a document group constituting individual clusters when a large number of documents are clustered based on the similarity measures. Each document group in a set of document groups S including a plurality of document groups E is represented as $E_u$(u=1, 2, ..., n)(n is the number of document groups).

S: A document group set including a plurality of document groups E, which consists of for example 300 patent documents similar to a patent document or a group of patent documents.

P: All the documents including a set of documents (large document set) including document groups E and a document group set S. As all the documents P, regarding analysis of patent documents, for example, about five million documents including all the patent publications and registered utility model publications issued in the past 10 years in Japan may be used.

N(E) or N(P): The number of documents included in a document group E or a document set P.

D, $D_k$ or $D_1$ to $D_{N(E)}$ : Individual documents included in a document group E.

W: The total number of index terms included in a document group E.

w, $w_i$, $w_j$: Individual index terms (i=1, ..., W, j=1, ..., w).

$\Sigma_{\{condition\ H\}}$: To obtain a sum in the range that satisfies the condition H.

$\Pi_{\{condition\ H\}}$: To obtain a product in the range that satisfies the condition H.

$\beta(w,D)$ : The weight of a index term w in a document D.

$C(w_i, w_j)$ : The degree of co-occurrence in a document group calculated based on the presence/absence of co-occurrence of index terms on a document basis. The presence/absence (1 or 0) of co-occurrence of index terms $w_i$ and $w_j$ in one document D is summed up for all documents D that belong to a document group E (as weighted by $\beta(w_i, D)$ and $\beta(w_j, D)$.

g or $g_h$ : A "ground" made of high frequency terms having similar co-occurrence degrees with each index terms. The number of grounds = b(h=1, 2, ..., b).

Co(w,g): Index term-ground co-occurrence degree. The co-occurrence degree $C(w, w')$ between an index term w and a high frequency term w' that belongs to a ground g is summed up for all w' (excluding w) that belong to the ground g.

$a_k$: The title (name) of a document $D_k$.

s: a character string connection of a title $a_k$ (k=1, ... , N(E)).

$x_k$: The appearance ratio of a title. The appearance ratio of each title $a_k$ (for the number of documents N(E)) in the sum of tittles s.

$m_k$: The genus of index terms $w_v$ (title words) appearing in each title $a_k$.

$f_k$: The appearance ratio of a title term in a title sum s (for the number of documents N(E)).

$y_k$: The average of the appearance ratio of a title word, which is created by dividing a title word appearance ratio $f_k$ by the genus $m_k$ of index terms $w_v$ appearing in each title $a_k$.

$\tau_k$: a title score. The score is calculated for each of the titles of documents that belong to a document group E in order to determine the order of extracting labels.

$T_1$, $T_2$, ...: Titles (names) extracted in the descending order of the title scores $\tau_k$.

k: keyword adaptability, which is calculated to determine the number of extracted labels (that will be described) and indicates the ratio occupied by a keyword in a document group E.

TF(D) or TF(w,D) : The occurrence frequency of an index term w in a document D (Term Frequency).

DF(P) or DF(w,p): The document frequency based on an index term w in all the documents P constituting a population (Document Frequency). The document frequency refers to the number of hit documents when search is carried out among a plurality of documents.

DF(E) or DF(w,E): The document frequency in a document group E based on an index term w.

DF(w,D): The document frequency in a document D based on an index term w. If the index term w is included in the document D, the frequency is 1 and if not, the frequency is zero.

IDF(P) or IDF(w,P): The logarithm of "the inverse of DF (P) $\times$ the total document number N (P) of all the documents." For example, ln (N(P)/DF (P)).

GF (E) or GF (w, E) : The occurrence frequency in a document group E based on an index term w (Global Frequency).

TF*IDF(P): The product of TF(D) and IDF(P), which is operated for each index term in a document.

GF(E)*IDF(P) : The product of GF (E) and IDF(P), which is operated for each index term in a document.

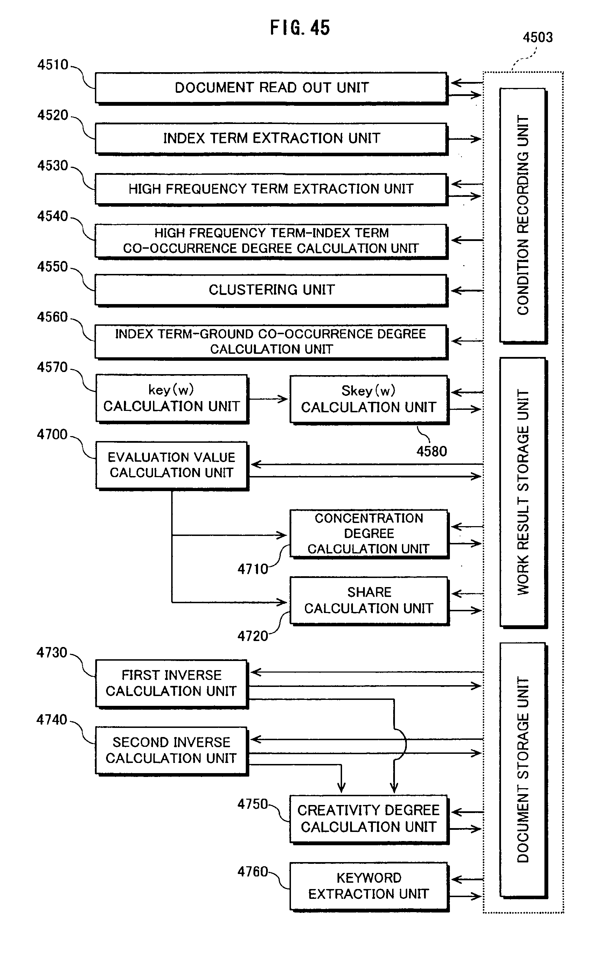

Hereinafter, the structure of a processing device used to extract a keyword will be described with reference to the block diagram in Fig. 45. In the following description, for the ease of understanding the content of the processing, all the necessary functions for extracting keywords among the functions of the first analysis server 513 and the second analysis server 514 are described.

A document read out unit 4510 reads out from a document storage unit of a recording device 4503 a document group E including a plurality of documents $D_1$ to $D_{N(E)}$ to be analyzed based on a reading condition stored in a condition recording unit in the recording device 4503. The data of the read out document group is directly sent to an index term extraction unit 4520 to be used for the processing therein and sent to an work result storage unit in the recording device 4503 to be stored therein.

Note that the data sent to the index term extraction unit 4520 or the work result storage unit from the document read out unit 4510 may be the entire data including the document data of the read out document group E. Alternatively, the data may be only the bibliographic data (such as application numbers or publication numbers in patent documents) that specifies documents D that belong to the document group E. In the latter case, if necessary in subsequent processing, the data of each document D may be read out again from the document storage unit based on the bibliographic data.

The index term extraction unit 4520 extracts index terms in each document from the document group read out by the document read out unit 4510. The index term data of each of the documents is directly sent to the high-frequency extraction unit 4530 and used for processing therein or sent to the work result storage unit in the recording device 4503 and stored therein.

The high frequency extraction unit 4530 extracts a prescribed number of index terms whose high occurrence frequency in a document group E is included in evaluation with large weights based on the index terms in each document extracted in the index term extraction unit 4520 according to a high-frequency term extracting condition stored in the condition recording unit in the recording device 4503.

More specifically, the occurrence frequency of each index term, GF(E) in the document group E is calculated. The IDF(P) of each index term is calculated, and GF(E)*IDF(P), the product of GF(E) and IDF(P) is preferably calculated. Then, a prescribed number of index terms having larger values as a result for GF(E) or GF(E)*IDF(P) as the weight of each of the calculated index terms are extracted as high frequency terms.

The data of the extracted high frequency terms is directly sent to the high frequency term-index term co-occurrence degree calculation unit 4540 and used for processing therein, or sent to the work result storage unit in the recording device 4503. The calculated GF(E) of the index terms and the IDF(P) of the index terms to be preferably calculated are preferably sent to the work result storage unit in the recording device 4503 and stored therein.

The high frequency term-index term co-occurrence degree calculation unit 4540 calculates co-occurrence degrees in a document group E based on the presence/absence of the co-occurrence of the high frequency terms extracted by the high frequency term extraction unit 4530 and the index terms extracted by the index term extraction unit 4520 and stored in the work result storage unit. If p index terms are extracted and q high frequency terms are extracted from the p index terms, matrix data of p rows and q columns results.

The co-occurrence degree data calculated by the high frequency term-index term co-occurrence calculation unit 4540 is directly sent to a clustering unit 4550 and used for processing therein or sent to the work result storage unit in the recording device 4503 and stored therein.

The clustering unit 4550 cluster-analyzes the q high frequency terms according to a clustering condition stored in the condition recording unit in the recording device 4503 based on the co-occurrence degree data calculated by the high frequency term-index term co-occurrence degree calculation unit 4540.

In order to carry out the cluster analysis, similarity measures among the co-occurrence degrees between the q high frequency terms and the index terms are operated.

Then, based on the result of calculation of the similarity measures and according to a tree-like diagram creating condition stored in the condition recording unit in the recording device 4503, a tree-like diagram connecting the high frequency terms in a tree-like form is created. As such a tree-like diagram, a dendrogram in which the dissimilarity measures between the high frequency terms are reflected as the height of the connecting position (connecting distance) is desirably created.

Then, according to a tree-like diagram cutting condition recorded in the condition recording unit in the recording device 4503, the created tree-like diagram is cut. As the result of cutting, the q high frequency terms are clustered based on the similarity measures of co-occurrence degree with the index terms. Individual clusters created by the clustering will be referred to as "ground" $g_h$ (h=1, 2, ..., b).

The ground data formed by the clustering unit 4550 is directly sent to an index term-ground co-occurrence degree calculation unit 4560 and used for processing therein or sent to the work result storage unit in the recording device 4503 and stored therein.

The index term-ground co-occurrence degree calculation unit 4560 calculates the co-occurrence degrees between the index terms extracted by the index term extraction unit 4520 and stored in the work result storage unit in the recording device 4503 and bases formed by the clustering unit 4550. The co-occurrence degree data calculated for each index term is directly sent to a key(w) calculation unit 4570 and used for processing therein or sent to the work result storage unit in the recording device 4503 and stored therein.

The key(w) calculation unit 4570 calculates a key(w) that is the evaluation score of each index term based on the co-occurrence degrees between the index terms and the grounds calculated in the index term-ground co-occurrence degree calculation unit 4560. The calculated key(w) data is directly sent to a Skey(w) calculation unit 4580 and used for processing therein or sent to the work result storage unit in the recording device 4503 and stored therein.

The Skey(w) calculation unit 4580 calculates Skey(w) scores based on the key(w) scores of the index terms calculated by the key(w) calculation unit 4570, the GF(E) of the index terms calculated by the high frequency term extraction unit 4530 and stored in the work result storage unit in the recording device 4503, and the IDF(P) of the index terms. The calculated Skey(w) data are sent to the work result storage unit in the recording device 4503 and stored therein.

An evaluation value calculation unit 4700 reads index terms $w_i$ in each document extracted by the index term extraction unit 4520 regarding a set of document groups S including a plurality of document groups $E_u$. Alternatively, the evaluation value calculation unit 4700 reads out the Skey(w) of the index terms calculated for each of the document groups $E_u$ by the Skey(w) calculation unit 4580 from the work result storage unit. If necessary, the evaluation value calculation unit 4700 may read out the data of each document group $E_u$ read out by the document read out unit 4510 from the work result storage unit and count the number of documents $N(E_u)$. $GF(E_u)$ and IDF(P) calculated in the process of extracting high frequency terms by the high frequency term extraction unit 4530 may be read out from the work result storage unit.

The evaluation value calculation unit 4700 calculates an evaluation value $A(W_i, E_u)$ based on the occurrence frequency of each index term $w_i$ in each of the document groups $E_u$ according to the read out information. The calculated evaluation values are sent to the work result storage unit and stored therein or directly sent to a concentration degree calculation unit 4710 and a share calculation unit 4720 and used for processing therein.

The concentration degree calculation unit 4710 reads out the evaluation value $A(w_i, E_u)$ for each of the index terms $w_i$ calculated by the evaluation value calculation unit 4700 in each of the document group $E_u$ or receives the value directly from the evaluation value calculation unit 4700.

The concentration degree calculation unit 4710 calculates the concentration degree of the distribution of each of the index terms $w_i$ in the document group set S for each index term $w_i$ based on the obtained evaluation value $A(w_i, E_u)$. The concentration degree is created for each index term $w_i$ by calculating the sum of the evaluation values $A(w_i, E_u)$ in all the document groups $E_u$ that belong to the document group set S and the ratio of the evaluation $A(w_i, E_u)$ in each document group $E_u$ relative to the sum for each document group $E_u$ and creating the squares of the ratios and the sum of the squares of the ratios in all the document group $E_u$ that belong to the document group set S. The calculated concentration degrees are sent to the work result storage unit and stored therein.

The share calculation unit 4720 reads out the evaluation value $A(w_i, E_u)$ of each index term $w_i$ in each document group $E_u$ calculated by the evaluation value calculation unit 4700 from the work result storage unit or directly receives from the evaluation value calculation unit 4700.

The share calculation unit 4720 calculates the share of each index term $w_i$ in each document group $E_u$ based on the obtained evaluation value $A(w_i, E_u)$. The share is created by summing up the evaluation value $A(w_i, E_u)$ of each index term $w_i$ in the document group $E_u$ for all the index terms $w_i$ extracted from each document group $E_u$ that belongs to the above-described document group set S, and calculating the ratio of the evaluation value $A(w_i, E_u)$ of each index term $w_i$ relative to the sum. The calculated concentration degree is sent to the work result storage unit and stored therein.

A first inverse calculation unit 4730 reads out the index terms $w_i$ in each document extracted in the index terms extraction unit 4520 regarding the document group set S including a plurality of document groups $E_u$ from the work result storage unit.

' The first inverse calculation unit 4730 calculates a function value of the inverse of the occurrence frequency of each index term $w_i$ in the document group set S (such as normalized IDF(S) that will be described) based on the data of the index terms $w_i$ in each document in the read out document group set S. The calculated function value of the inverse of the occurrence frequency in the document group set S is sent to the work result storage unit and stored therein or directly sent to a creativity degree calculation unit 4750.

The second inverse calculation unit 4740 calculates a function value of the inverse of the occurrence frequency in the large document set including the document group set S. As the large document set, all the documents P are used. In this case, IDF(P) calculated in the process of extracting a high frequency term in the high frequency term extraction unit 4530 is read out from the work result storage unit and its function value (such as normalized IDF(P) that will be described) is calculated. The calculated function value of the inverse of the occurrence frequency in the large document set P is sent to the work result storage unit and stored therein or directly sent to the creativity degree calculation unit 4750 and used for processing therein.

The creativity degree calculation unit 4750 reads out the function value of the inverse of each occurrence frequency calculated in the first inverse calculation unit 4730 and the second inverse calculation unit 4740 or directly receives the value from the first inverse calculation unit 4730 and the second inverse calculation unit 4740. GF(E) calculated in the process of extracting a high frequency term in the high frequency extraction unit 4530 is read out from the work result storage unit.

The uniqueness calculation unit 4750 calculates as a creativity degree a function value of what is obtained by subtracting the calculation result of the second inverse calculation unit 4740 from the calculation result of the first inverse calculation unit 4730. The function value may be obtained by subtracting the result of calculation by the second inverse calculation unit 4740 from the result of calculation by the first inverse calculation unit 4730 and dividing the result by the sum of the calculation results by the first inverse calculation unit 4730 and the second inverse calculation unit 4740 or by multiplying the result by $GF(E_u)$ in each document group $E_u$. The calculated creativity degree is sent to the work result storage unit and stored therein.

The keyword extraction unit 4760 reads out data including Skey(w) calculated by the Skey(w) calculation unit 4580, the concentration degrees calculated by the concentration degree calculation unit 4710, the shares calculated by the share calculation unit 4720, creativity degrees calculated by the creativity degree calculation unit 4570 from the work result storage unit.

The keyword extraction unit 4760 extracts keywords based on two or more indexes selected from the four indexes, the read out Skey(w), the concentration degrees, the shares, and the creativity degrees. The keywords may be extracted for example by determining whether the total values of the selected multiple indexes is not less than a prescribed threshold or within a prescribed range of ranks or by categorizing the keywords based on the combinations of the selected multiple indexes.

The extracted keyword data is sent to the work result storage unit in the recording device 4503 and stored therein.

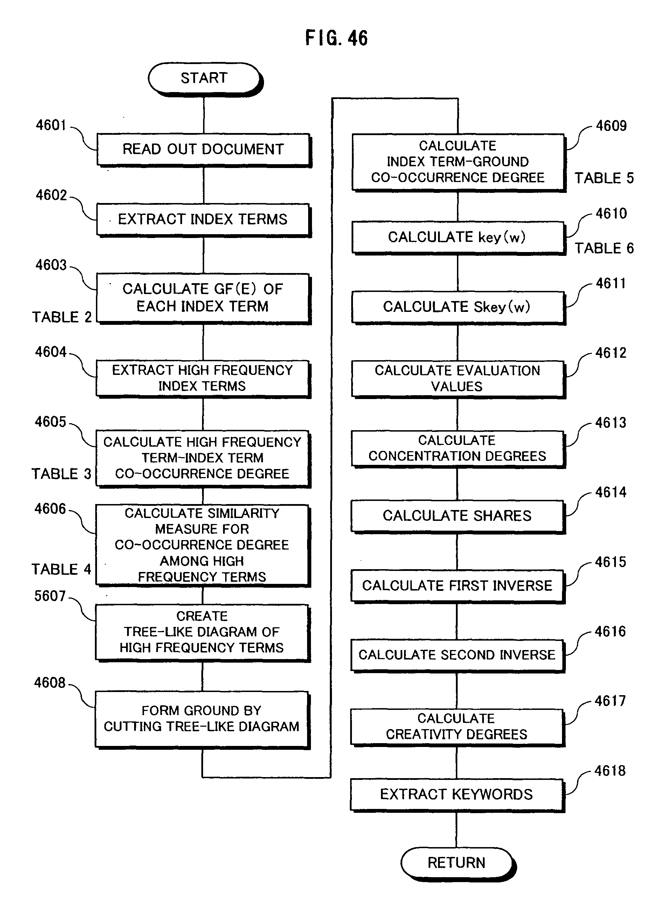

Now, the process of extracting keywords will be described with reference to the flowchart in Fig. 46.

1. Reading Document

The document read out unit 4510 reads out a document group E including a plurality of documents $D_1$ to $D_N$ (E) to be analyzed from the document storage unit in the recording device 4503 (step S4601).

2. Extracting Index Terms

Now, the index term extraction unit 4520 extracts index terms in each document from the document group read out in the document reading step S4610 (step S4602). The index term data in each document may be expressed for example by a vector including as a component a function value of the appearance times of each index term in each document D (index term frequency TF(D)) included in the document group E.

3. Extracting High frequency Terms

The high frequency term extraction unit 4530 extracts a prescribed number of index terms whose high occurrence frequencies in the document group E are included in evaluation having large weights based on the index term data in each document extracted in the index term extracting step S4602.

More specifically, GF(E) as the occurrence frequency in the document group E is calculated for each index term (step S4603). In order to calculate the GF(E) of each index term, the index term frequency TF(D) of each index term in each document calculated in the index term extracting step S4602 may be summed up for the documents $D_1$ to $D_{N(E)}$ that belong to the document group E.

For the ease of description, virtual examples of TF(D) and GF(E) when 14 index terms $w_1$ to $w_{14}$ in total are included in a document E including six documents $D_1$ to $D_6$ are given in the following table. In the following description, the virtual examples will be referred to as required.

Table 2

| TF (D) and GF (E) of each index term | | | | | | | | |
|---|---|---|---|---|---|---|---|---|
| | | document | | | | | | GF (E) |
| | | $D_1$ | $D_2$ | $D_3$ | $D_4$ | $D_5$ | $D_6$ | |
| index term | $w_1$ | 3 | 3 | 3 | 0 | 0 | 0 | 9 |
| | $w_2$ | 3 | 0 | 3 | 3 | 0 | 0 | 9 |
| | $w_3$ | 3 | 3 | 3 | 3 | 0 | 0 | 12 |
| | $w_4$ | 3 | 3 | 3 | 3 | 3 | 0 | 15 |
| | $w_5$ | 0 | 0 | 3 | 3 | 3 | 3 | 12 |
| | $w_6$ | 0 | 3 | 0 | 3 | 3 | 3 | 12 |
| | $w_7$ | 0 | 0 | 0 | 3 | 3 | 3 | 9 |
| | $w_8$ | 1 | 1 | 1 | 1 | 1 | 1 | 6 |
| | $w_9$ | 1 | 0 | 0 | 0 | 0 | 0 | 1 |
| | $w_{10}$ | 0 | 1 | 0 | 0 | 0 | 0 | 1 |
| | $w_{11}$ | 0 | 0 | 1 | 0 | 0 | 0 | 1 |
| | $w_{12}$ | 0 | 0 | 0 | 1 | 0 | 0 | 1 |
| | $w_{13}$ | 0 | 0 | 0 | 0 | 1 | 0 | 1 |
| | $w_{14}$ | 0 | 0 | 0 | 0 | 0 | 1 | 1 |

Now, based on the calculated GF(E) of each index term, a prescribed number of index terms with highest occurrence frequencies are extracted (step S4604). The number of extracted high frequency terms is for example ten. In this case, if the tenth and eleventh terms are in the same place in the ranking, the eleventh term is extracted as a high frequency term as well.

When the high frequency terms are extracted, a prescribed number of index terms with high GF(E)*IDF(P) are preferably extracted by calculating the IDF(P) of each index term. In the following description of the above virtual examples, terms with the highest seven GF(E) are high frequency terms for the ease of description. More specifically, the index terms $w_1$ to $w_7$ are extracted as the high frequency terms.

Note that in order to extract high frequency terms from index terms, it is preferable that unnecessary terms are removed from all the index terms in advance and high frequency terms are extracted from the remaining terms. However, as for a Japanese document, for example, terms are segmented differently using different kinds of morpheme analysis software, and therefore it is impossible to create a sufficient unnecessary term list. Therefore, a minimum amount of unnecessary terms is preferably removed. As an unnecessary term list may include the following examples for patent documents.

Meaningless word as Keywords

said, the above-described, the, the following, description, claims, claim, patent, number, formula, general, foregoing, as follows, means, characteristic

Terms with Little Importance as Keywords, Unit Symbols, Roman Numerals

entire, range, kind, class, system, for, %, mm, ml, nm, $\mu$m, etc.

In this example, the generalized capability is at issue, and therefore the above described examples are selected as unnecessary terms, while a necessary list may be added as required depending on the kind of morpheme analysis software to be used or the field of the document group.

4. Calculating High Frequency Term-Index Term Co-occurrence Degree

Now, the high frequency term-index term co-occurrence degree calculation unit 4540 calculates the degree of co-occurrence between each high frequency term extracted in the above-described high frequency term extracting step S4604 and each index term extracted in the above index term extracting step S4602 (step S4605).

The degree of co-occurrence C $(w_i, w_j)$ of the index terms $w_i$ and $w_j$ in the document group E is for example calculated by the following expression.

$$C(w_i, w_j) = \Sigma_{\{D \in E\}} [\beta(w_i, D) \times \beta(w_j, D) \times DF(w_i, D) \times DF(w_j, D)] \quad \ldots(1)$$

where $\beta(w_i, D)$ is the weight of an index term $w_i$ in the document D, and can be for example any of the following.

$$\beta(w_i, D) = 1$$

$$\beta(w_i, D) = TF(w_i, D)$$

$$\beta(w_i, D) = TF(w_i, D) \times IDF(w_i, P)$$

Since DF($w_i$,D) is 1 if the index term $w_i$ is included in the document D and zero if it is not included, DF $(w_i, D) \times DF(w_j,D)$ is 1 if the index terms $w_i$ and $w_j$ co-occur in one document D and zero if they do not. This is calculated for all the documents D that belong to the document group E (after weighted with $\beta(w_i, D)$ and $\beta(w_j, D)$), the results are totaled. The totaled result represents the degree of co-occurrence C($w_i, w_j$) of the index terms $w_i$ and $w_j$.

Note that in a similar example of the above Expression (1), the co-occurrence degree c($w_i,w_j$) in the document D calculated based on the presence/absence of the co-occurrence of the index terms $w_i$ and $w_j$ in a sentence may be used instead of [$\beta(w_i, D) \times \beta(w_j, D)$]. The co-occurrence degree c($w_i, w_j$) in the document D may be calculated for example by the following expression.

$$C(w_i, w_j) = \Sigma_{\{sen \in D\}} [TF(w_i, sen) \times TF(w_j, sen)] \ldots \quad (2)$$

where sen means each sentence in the document D. If the index terms $w_i$ and $w_j$ co-occur in a certain sentence, $[TF(w_i, sen) \times TF(w_j, sen)]$ returns at least 1 and zero if they do not. This is carried out for every sentence sen in the document D and the result is totaled as the degree of co-occurrence $c(w_i, w_j)$ in the document D.

Based on the above-described virtual examples, the co-occurrence degrees may be calculated while the weight $\beta(w_1, D) = 1$ from Expression (1) as follows. The index terms $w_1$ and $w_1$ are the same index terms would co-occur in three documents $D_1$ to $D_3$ in total, in other words, the co-occurrence degree $C(w_1, w_1) = 3$. The index terms $w_2$ and $w_1$ would co-occur in two documents $D_1$ and $D_3$ in total, in other words, the co-occurrence degree $C(w_2, w_1) = 2$. Similarly, if the co-occurrence degree $C(w_i, w_j)$ is calculated based on combinations of any one of the index terms $w_1$ to $w_{14}$ and any one of the high frequency terms $w_1$ to $w_7$, the following matrix data including 14 rows and 7 columns results.

Table 3

| Co-occurrence degree of each high frequency term with each index term | | | | | | |
|---|---|---|---|---|---|---|
| | high frequency term $w_j$ | | | | | |
| | $w_1$ | $w_2$ | $w_3$ | $w_4$ | $w_5$ | $w_6$ | $w_7$ |
| $C(w_1, w_j)$ | 3 | 2 | 3 | 3 | 1 | 1 | 0 |
| $C(w_2, w_j)$ | 2 | 3 | 3 | 3 | 2 | 1 | 1 |
| $C(w_3, w_j)$ | 3 | 3 | 4 | 4 | 2 | 2 | 1 |
| $C(w_4, w_j)$ | 3 | 3 | 4 | 5 | 3 | 3 | 2 |
| $C(w_5, w_j)$ | 1 | 2 | 2 | 3 | 4 | 3 | 3 |
| $C(w_6, w_j)$ | 1 | 1 | 2 | 3 | 3 | 4 | 3 |
| $C(w_7, w_j)$ | 0 | 1 | 1 | 2 | 3 | 3 | 3 |
| $C(w_8, w_j)$ | 3 | 3 | 4 | 5 | 4 | 4 | 3 |
| $C(w_9, w_j)$ | 1 | 1 | 1 | 1 | 0 | 0 | 0 |
| $C(W_{10}, w_j)$ | 1 | 0 | 1 | 1 | 0 | 1 | 0 |
| $C(w_{11}, w_j)$ | 1 | 1 | 1 | 1 | 1 | 0 | 0 |
| $C(w_{12}, w_j)$ | 0 | 1 | 1 | 1 | 1 | 1 | 1 |
| $C(w_{13}, w_j)$ | 0 | 0 | 0 | 1 | 1 | 1 | 1 |
| $C(w_{14}, w_j)$ | 0 | 0 | 0 | 0 | 1 | 1 | 1 |

5. Clustering

Then, the clustering unit 4550 carries out cluster-analysis to the high frequency terms based on the co-occurrence degrees calculated in the high frequency term-index term co-occurrence calculating step S4605.

In order to carry out the cluster analysis, the similarity measures are operated for the co-occurrence degrees with the index terms for the high frequency terms and (step S4606).

In the above-described virtual examples, a result of calculation when co-relation coefficients between 14-dimensional column vectors for each of the high frequency terms $w_1$ to $w_7$ are employed as similarity measures is as given in the following Table.

Table 4

| Similarity measure for co-occurrence degree (correlation coefficient) | | | | | | | |
|---|---|---|---|---|---|---|---|
| | $w_1$ | $w_2$ | $w_3$ | $w_4$ | $w_5$ | $w_6$ | $w_7$ |
| $w_1$ | 1 | 0.845 | 0.939 | 0.840 | 0.315 | 0.281 | 0.011 |
| $w_2$ | - | 1 | 0.944 | 0.892 | 0.589 | 0.412 | 0.300 |
| $w_3$ | - | - | 1 | 0.948 | 0.548 | 0.499 | 0.279 |
| $w_4$ | - | - | - | 1 | 0.738 | 0.706 | 0.523 |
| $w_5$ | - | - | - | - | 1 | 0.898 | 0.924 |
| $w_6$ | - | - | - | - | - | 1 | 0.928 |
| $w_7$ | - | - | - | - | - | - | 1 |

The lower left half and the upper right half overlap and are therefore omitted. In the table, for example for high frequency terms $w_1$ to $w_4$, any combinations of these terms each have a correlation coefficient of more than 0.8. For high frequency terms $w_5$ to $w_7$, any combinations of these terms have each a correlation coefficient of more than 0.8. Conversely, for combinations of any of high frequency terms $w_1$ to $w_4$ and any of high frequency terms $w_5$ to $w_7$, the correlation coefficients are all less than 0.8.

Now, based on the result of calculation of the similarity measures, a tree-like diagram in which high frequency terms are connected like a tree is created (step S4607).

As a tree-like diagram, a dendrogram in which dissimilarity measures between the high frequency terms are reflected on the height of the connecting positions (connecting distances) is desirably created. According to the principle of creating such a dendrogram, based on the dissimilarity measures between the high frequency terms, the high frequency terms having the minimum dissimilarity measure (the largest similarity measure) are connected with each other to form a connected body. Then, the connected body is connected to another high frequency term or such a connected body and another connected body are connected one after another in the ascending order of similarity measures. In this way, a hierarchical representation is formed. The dissimilarity measure between a connected body and another high frequency term or the dissimilarity measure between connected bodies is updated based on the dissimilarities between the high frequency terms. The updating may be carried out for example according to a known Ward method.

Then, the clustering unit 4550 cuts the above-created tree-like diagram (step S4608). For example, the diagram is cut at the position of $<D>+\delta\sigma_D$ where the connecting distance in the dendrogram is D, $<D>$ is the average of D, $\sigma_D$ is the standard deviation of D, and $\delta$ is given within a range $-3 \leq \delta \leq 3$, preferably $\delta = 0$.

As the result of cutting, the high frequency terms are clustered based on the similarity measures for the co-occurrence degrees with the index terms, and the "ground" $g_h$ (h=1, 2, ..., b) consisting of a high frequency term group that belongs to each cluster is formed. High frequency terms that belong to the same ground $g_h$ have higher similarity measures in the co-occurrence degrees with the index terms, and high frequency terms that belong to different grounds $g_h$ have low similarity measures in the co-occurrence degrees with the index terms.

As for the tree-like diagram and the process of cutting the diagram, the description in connection with the above-described virtual examples is not repeated, while assume that two grounds (the number of grounds b=2), i.e., the ground $g_1$ including high frequency terms $w_1$ to $w_4$ and the ground $g_2$ including high frequency terms $w_5$ to $w_7$ are formed.

6. Calculating Index Term-Ground Co-occurrence Degree

Then, the index term-ground co-occurrence degree calculation unit 4560 calculates the degree of co-occurrence Co (w, g) (index term-ground co-occurrence degree) between each index term extracted in the index term extracting step S4602 and each ground formed in the clustering step S4608 (step S4609).

The index term-ground co-occurrence degree Co(w,g) is for example calculated by the following expression.

$$\text{Co}(w, g) = \sum_{\{w' \in g,\ w' \neq w\}} C(w, w') \qquad \cdots (3)$$

where w' is a high frequency term that belongs to a certain ground g and refers to a term other than the index term w to be measured for the degree of co-occurrence Co(w,g). The degree of co-occurrence Co(w,g) between the index term w and the ground g is the total of the degrees of co-occurrence C(w,w') between all w' and w.

In the above-described virtual examples, the co-occurrence degree $Co(w_1,g_1)$ between the index term $w_1$ and the ground $g_1$ is represented as follows:

$$Co(w_1, g_1) = C(w_1, w_2) + C(w_1, w_3) + C(w_1, w_4)$$

From Table 3, the value equals 2+3+3=8.

The co-occurrence degree $Co(w_1, g_2)$ between the index term $w_1$ and the ground $g_2$ is represented as follows:

$$Co(w_1, g_2) = C(w_1, w_5) + C(w_1, w_6) + C(w_1, w_7) = 1 + 1 + 0 = 2$$

Similarly, all the index terms w and the grounds $g_1$ and $g_2$ are calculated for the co-occurrence degrees and the result is given in the following table.

Table 5

| Co-occurrence degree Co(w,g) of index term w and ground g | | |
|---|---|---|
| | $g_1$ | $g_2$ |
| $w_1$ | $Co(w_1,g_1 = 2 + 3 + 3 = 8$ | $Co(w_1, g_2) = 1 + 1 + 0 = 2$ |
| $w_2$ | $Co(w_2,g_1) = 2 + 3 + 3 = 8$ | $Co(w_2,g_2) = 2 + 1 + 1 = 4$ |
| $w_3$ | $Co(w_3, g_1) = 3 + 3 + 4 = 10$ | $Co(w_3, g_2) = 2 + 2 + 1 = 5$ |
| $w_4$ | $Co(w_4,g_1) = 3 + 3 + 4 = 10$ | $Co(w_4,g_2) = 3 + 3 + 2 = 8$ |
| $w_5$ | $Co(w_5,g_1) = 1 + 2 + 2 + 3 = 8$ | $Co(w_5,g_2) = 3 + 3 = 6$ |
| $w_6$ | $Co(w_6,g_1) = 1 + 1 + 2 + 3 = 7$ | $Co(w_6,g_2) = 3 + 3 = 6$ |
| $w_7$ | $Co(w_7,g_1) = 0 + 1 + 1 + 2 = 4$ | $Co(w_7,g_2) = 3 + 3 = 6$ |
| $w_8$ | $Co(w_8,g_1) = 3 + 3 + 4 + 5 = 15$ | $Co(w_8,g_2) = 4 + 4 + 3 = 11$ |
| $w_9$ | $Co(w_9,g_1) = 1 + 1 + 1 + 1 = 4$ | $Co(w_9,g_2) = 0 + 0 + 0 = 0$ |
| $w_{10}$ | $Co(w_{10},g_1) = 1 + 0 + 1 + 1 = 3$ | $Co(w_{10},g_2) = 0 + 1 + 0 = 1$ |
| $w_{11}$ | $Co(w_{11},g_1 = 1 + 1 + 1 + 1 = 4$ | $Co(w_{11},g_2) = 1 + 0 + 0 = 1$ |
| $w_{12}$ | $Co(w_{12},g_1 = 0 + 1 + 1 + 1 = 3$ | $Co(w_{12},g_2) = 1 + 1 + 1 = 3$ |
| $w_{13}$ | $Co(w_{13},g_1) = 0 + 0 + 0 + 1 = 1$ | $Co(w_{13},g_2) = 1 + 1 + 1 = 3$ |
| $w_{14}$ | $Co(w_{14},g_1) = 0 + 0 + 0 + 0 = 0$ | $Co(w_{14},g_2) = 1 + 1 + 1 = 3$ |
| Note that the index term-ground co-occurrence degree may be calculated by the following expression rather than the above Co (w, g) . | | |

$$Co'(w, g) = \Sigma_{\{D \in E\}} [\beta(w, D) \times DF(w, D) \times \Theta(\Sigma_{\{w' \in g, w' \neq w\}} DF(w', D))]$$

$$... (4)$$

where $\Theta(x)$ is a function that returns 1 if X>0, and 0 if X≤0. According to $\Theta(\Sigma_{\{w' \in g, w' \neq w\}} DF(w', D))$, if at least one w' that is any one of high frequency terms that belong to the ground g and other than the index term w to be measured for the co-occurrence degree is included in a document D, 1 is returned, while if no such term is included, zero is returned. DF (w,D) returns 1 if at least one index term w to be measured for the co-occurrence degree is included in a document D and returns zero if no such term is included. Multiplying DF (w, D) by $\Theta(X)$ returns 1 if w and any w' that belongs to the ground g co-occur in the document D and zero if there is no co-occurrence. This is multiplied by the weight β (w,D) defined above and the total of the results for all the documents D that belong to the document group E is Co'(w,g).

The index term-ground co-occurrence degree Co(w,g) in Expression (3) is created by totaling the presence/absence (1 or 0) of co-occurrence of w with w' in D with a weight β(w, D)× β(w',D) for all E (C(w,w')) and totaling the results for w' in g. On the other hand, the index term-ground co-occurrence degree Co' (w,g) in Expression (4) is created by totaling the presence/absence (1 or 0) of co-occurrence of w and any w' in g in D with a weight β (w, D) for all E.

Therefore, in any of the cases, the number of documents D having co-occurrence with high frequency terms is larger, a larger degree of index term-ground co-occurrence results. The degree of index term-ground co-occurrence Co(w,g) in Expression (3) changes with changes in the number of w' in the ground g that co-occurs with the index term w, while the degree of index term-ground co-occurrence Co' (w, g) in Expression (4) changes based on the presence/absence of w' in the ground g that co-occurs with the index term w independently of increase/decrease in the number of w'. When the degree of index term-ground co-occurrence Co(w,g) in Expression (3) is used, it is preferable that the weight β(w, D) =1, while when the degree of index term-ground co-occurrence Co' (w, g) in Expression (4) is used, it is preferable that the weight β(w,D)=TF(w,D).

7. Calculating key(w)

Then, the key (w) calculation unit 4570 calculates key (w) that is the evaluation score of each index term based on the co-occurrence degree between each index term and the ground calculated in the index term-ground co-occurrence degree calculating step S4609 (step S4610).

For example, key(w) is calculated by the following expression:

$$\mathrm{key}(w) = 1 - \Pi_{\{1 \le h \le b\}} [1 - \mathrm{Co}(w, g_h) / F(g_h)] \qquad \dots (5)$$

where $F(g_h) = \Sigma_{\{w \in E\}} \mathrm{Co}(w, g_h)$ by definition. This is the total of the co-occurrence degrees $\mathrm{Co}(w, g_h)$ between the index terms w and the grounds $g_h$ for all the index terms w. Then, $\mathrm{Co}(w, g_h)$ is divided by $F(g_h)$, the difference between the result and 1 is obtained, the result is multiplied for all the grounds $g_h$ (h=1, 2, ..., b) and the difference between the results and 1 are obtained as key(w).

Note that as the index term-ground co-occurrence degree, Co(w,g) in the above-described Expression (3) is used, while Co'(w,g) in Expression (4) may be used as described above.

From Table 4, $F(g_h)$ is calculated in the above virtual examples as follows.

$$F(g_1) = \mathrm{Co}(w_1, g_1) + \mathrm{Co}(w_2, g_1) + \dots + \mathrm{Co}(w_{14}, g_1) = 85$$

$$F(g_2) = \mathrm{Co}(w_1, g_2) + \mathrm{Co}(w_2, g_2) + \dots + \mathrm{Co}(w_{14}, g_2) = 59$$

Then, key(w) is represented as follows:

$$\mathrm{key}(w_1) = 1 - (1 - \mathrm{Co}(w_1, g_1) / 85)(1 - \mathrm{Co}(w_1, g_2) / 59)$$
$$= 1 - (1 - 8/85)(1 - 2/59) = 0.125$$

$$\mathrm{key}(w_2) = 1 - (1 - \mathrm{Co}(w_2, g_1) / 85)(1 - \mathrm{Co}(w_2, g_2) / 59)$$
$$= 1 - (1 - 8/85)(1 - 4/59) = 0.156$$

Similarly, key(w) is calculated for all the index terms as shown in the following table.

Table 6

| index term | key(w) | rank |
|---|---|---|
| $w_1$ | 1 - (1 - 8/85) (1 - 2/59) = 0.125 | 8 |
| $w_2$ | 1 - (1 - 8/85) (1 - 4/59) = 0.156 | 6 |
| $w_3$ | 1 - (1 - 10/85) (1 - 5/59) = 0.192 | 3 |
| $w_4$ | 1 - (1 - 10/85) (1 - 8/59) = 0.237 | 2 |
| $w_5$ | 1 - (1 - 8/85) (1 - 6/59) = 0.186 | 4 |
| $w_6$ | 1 - (1 - 7/85) (1- 6/59) = 0.176 | 5 |
| $w_7$ | 1 - (1 - 4/85) (1 - 6/59) = 0.144 | 7 |
| $w_8$ | 1 - (1 - 15/85) (1 - 11/59) = 0.330 | 1 |
| $w_9$ | 1 - (1 - 4/85) (1 - 0/59) = 0.047 | 14 |
| $w_{10}$ | 1 - (1 - 3/85) (1 - 1/59) = 0.052 | 12 |
| $w_{11}$ | 1 - (1 - 4/85) (1 - 1/59) = 0.063 | 10 |
| $w_{12}$ | 1 - (1 - 3/85) (1 - 3/59) = 0.084 | 9 |
| $w_{13}$ | 1 - (1 - 1/85) (1 - 3/59) = 0.062 | 11 |
| $w_{14}$ | 1 - (1 - 0/85) (1 - 3/59) = 0.051 | 13 |

The column in the right end of the table indicates the ranking of key(w) when they are arranged in the descending order.

In order to describe the characteristic of key(w), the same content in Table 2 is indicated with the document frequency DF(E) of each of the index terms and the ranking of key(w) described above as follows:

Table 7

| | | | | document | | | | GF(E) | DF(E) | key (w) rank |
|---|---|---|---|---|---|---|---|---|---|---|
| | | $D_1$ | $D_2$ | $D_3$ | $D_4$ | $D_5$ | $D_6$ | | | |
| | $w_1$ | 3 | 3 | 3 | 0 | 0 | 0 | 9 | 3 | 8 |
| | $w_2$ | 3 | 0 | 3 | 3 | 0 | 0 | 9 | 3 | 6 |
| | $w_3$ | 3 | 3 | 3 | 3 | 0 | 0 | 12 | 4 | 3 |
| | $w_4$ | 3 | 3 | 3 | 3 | 3 | 0 | 15 | 5 | 2 |
| | $w_5$ | 0 | 0 | 0 | 3 | 3 | 3 | 12 | 4 | 4 |
| | $w_6$ | 0 | 3 | 0 | 3 | 3 | 3 | 12 | 4 | 5 |
| index term | $w_7$ | 0 | 0 | 0 | 3 | 3 | 3 | 9 | 3 | 7 |
| | $w_8$ | 1 | 1 | 1 | 1 | 1 | 1 | 6 | 6 | 1 |
| | $w_9$ | 1 | 0 | 0 | 0 | 0 | 0 | 1 | 1 | 14 |
| | $w_{10}$ | 0 | 1 | 0 | 0 | 0 | 0 | 1 | 1 | 12 |
| | $w_{11}$ | 0 | 0 | 1 | 0 | 0 | 0 | 1 | 1 | 10 |
| | $w_{12}$ | 0 | 0 | 0 | 1 | 0 | 0 | 1 | 1 | 9 |
| | $w_{13}$ | 0 | 0 | 0 | 0 | 1 | 0 | 1 | 1 | 11 |
| | $w_{14}$ | 0 | 0 | 0 | 0 | 0 | 1 | 1 | 1 | 13 |

TF(D) and GF(E) of each index term, etc.

As can be understood from the table, the ranking of key (w) is greatly affected by the ranking of the document frequencies DF(E) in the document group E. For example, the index term $w_8$ with the maximum DF(E) corresponds to key(w) in the first rank, and the index term $w_4$ with the next largest DF(E) corresponds to key(w) in the second rank, and thereafter, the same applies to the index terms $w_3$, $w_5$, $w_6$, and the like.

For index terms with larger document frequencies DF(E) in the document group E can co-occur with high frequency index terms in a larger number of documents. Therefore, larger index term-ground co-occurrence degree Co (w,g) or Co'(w,g) can be obtained. This is considered to be the reason why the ranking of key(w) is greatly affected by the ranking of DF(E).

Note that the weight β(w,D) used for calculation of the co-occurrence degrees is replaced by TF(w,D), the ranking of key(w) is considered to be more affected by the ranking of a global frequency GF(E) in the document group E.

As can be understood from comparison among index terms $w_9$ to $w_{14}$ in Tables 3 and 7, those co-occurring with high frequency terms covering a larger number of grounds have greater key(w). For example, a high frequency term co-occurring with index terms $w_{10}$ to $w_{13}$ covers two grounds, while a high frequency term co-occurring with the index terms $w_9$ and $w_{14}$ is localized to one ground. The index terms $w_{10}$ to $w_{13}$ have greater key(w) than the index terms $w_9$ and $w_{14}$.

As can be understood from comparison among index terms $w_{10}$ to $w_{13}$ in Tables 3 and 7, those co-occurring with more high frequency terms tend to have greater key(w). For example, the index term $w_{12}$ co-occurring with the largest number of high frequency term among $w_{10}$ to $w_{13}$ has the largest key (w), and $w_{11}$ co-occurring with the second largest number of high frequency terms has the second largest key(w).

Note that the following expression may be used as the evaluation score of each index term instead of the above key (w).

$$ key'(w) = (1/\Phi)(1/b) \times \sum_{h=1}^{b} Co(w, g_h) \qquad \ldots (6) $$

where Φ is an appropriate normalization constant, and for example $\Phi = \Sigma_{n=1}^{b} F(g_h)$. $F(g_h)$ is as defined in the above Expression (5).

key'(w) is created by multiplying the average of the co-occurrence degrees $Co(w,g_h)$ between the index terms w and the grounds $g_h$ in all grounds $g_h$(h=1,..., b) by the constant (1/Φ).

The following expression may be used as the evaluation score of each index term instead of key(w).

$$ key''(w) = (1/b) \times \sum_{h=1}^{b} [Co(w, g_h)/F(g_h)] \qquad \ldots (7) $$

key" (w) is created by dividing the co-occurrence degree $Co(w,g_h)$ between the index term w and the ground $g_h$ by $F(g_h)$ and obtaining the average in all the grounds $g_h$ (h=1, ..., b).

The product part in key (w) in Expression (5) is developed and if the high-order small term $O[(Co(w, g_h)/F(g_h))^2]$ is ignored, the following is established.

$$ key(w) = 1 - [1 - Co(w, g_1)/F(g_1)] \times [1 - Co(w, g_2)/F(g_2)] \times \ldots $$
$$ \approx 1 - 1 + Co(w, g_1)/F(g_1) + Co(w, g_2)/F(g_2) + \ldots $$

Therefore, key" (w) ≈ (1/b) key (w) is established.

8. Calculating Skey(w)

Then, in the Skey (w) calculation unit 4580, Skey(w) score is calculated based on the key(w) score of each index term calculated in the key(w) calculating step S4610 and the GF(E) of each index term and the IDF(P) of each index term calculated in the high frequency term extracting step S4604 (step S4611).

Skey (w) score is calculated by the following expression.

$$Skey(w)=GF(w,E)\times In[key(w)\div(DF(w,P)/N(P))]$$
$$=GF(w,E)\times[IDF(P)+In\ key(w)]\quad\ldots(8)$$

A large value is provided for GF (w, E) of a term occurring very often in a document group E, and a large value is provided for IDF(P) of a term rare in all the documents P and unique to the document group E. As described above, key (w) is affected by DF(E), and a large value is provided to key(w) of a term that co-occurs with a larger number of grounds. The larger the values for GF (w, E), IDF (P), and key (W) are, the larger will be Skey(w).

TF*IDF often used as a weight to an index term is the product of an index term frequency TF and IDF that is the logarithm of the inverse of the occurrence ratio DF(P)/N(P) of an index term in a document set. IDF has effectively reduces the contribution of an index term occurring with high percentage in a document set and can provide a high weight to an index term occurring locally in a particular document. However, the value could sometimes be increased just because the document frequency is small. As will be described, Skey (w) score is used to effectively improve the disadvantage.

In a document group E to be analyzed, when the probability of the occurrence of a document including an index term w is P (A), the probability of the occurrence of a document including a ground (an index term that belongs to a ground) is P (B), and the probability of the occurrence of a document including both an index term w and a ground (the percentage that they co-occur in a document) is P(A∩B), the following expressions are established.

$$P(A)=DF(w,E)/N(E)$$

$$P(A∩B)=key(w)$$

Therefore, in the document group E, the probability (conditional probability) of the co-occurrence of a selected document including an index term w with a ground is represented as follows:
$$P(B|A)=P(A∩B)/P(A)=key(w)\times N(E)/DF(w,E)\quad\ldots(9)$$

Furthermore, if the assumption of uniformity (IDF(E)=IDF(P)) is considered, and the logarithm of the conditional probability is obtained, the following Expression is obtained.
$$In\ P(B|A)=In[key(w)\times N(P)/DF(w,P)]$$
$$=In\ key(w)+IDF(P)\qquad\ldots(10)$$

The value is equal to IDF(P) if key (w) =1. At the limit DF → 0, since N(P)/DF(w, P) → ∞ and key(w) → 0, and therefore, by obtaining the product of N(P)/DF(w,P) and key(w), the disadvantage that the IDF value is singularly raised when the DF value is small can be improved. The Skey(w) score in Expression (8) is created by obtaining the product of GF(w, E) and In key(w)+IDF(P) in Expression 10, and therefore it can be GF(E)*IDF(P) corrected by the degree of co-occurrence.

Note that in the calculation of Skey(w) by Expression 8, instead of key(w) in Expression 5, key'(w) in Expression 6 or key"(w) in Expression 7 may be used as described above.

When key" (w) in Expression 7 is used, the Skey (w) score is represented as Skey ( key"), while when key (w) in Expression 5 is used, the Skey(w) score is represented as Skey (key), and then they can be compared as follows.

$$Skey(key)-Skey(key")=GF(w,E)\times[ln\ key(w)-ln\ key"(w)]$$
$$\approx GF(w,E)\times ln\ b$$

Therefore, the behaviors of Skey(w) using key"(w) in Expression (7) and Skey(w) using key(w) in Expression (5) substantially match excluding the difference in the number of grounds b, and as long as the number of grounds b is not

EP 1 881 423 A1

large, the ranking of Skey(w) scores is not greatly affected.

9. Calculating Evaluation Value

When Skey(w) is calculated, the evaluation value calculation unit 4700 calculates an evaluation value $A(w_1, E_u)$ based on a function value of the occurrence frequency of the index term $w_i$ in each document group $E_u$ for each document group $E_u$ and each index term $w_i$ (step S4612).

As the evacuation value $A(w_i, E_u)$, for example the following Skey(w) may be used as it is, or Skey(w)/N($E_u$) or GF(E)*IDF(P) may be used. For example, for each document group $E_u$ and each index term $w_i$, the following data is obtained. Note that for the ease of description, the genus W of index terms equals 5 and the number of document groups n equals 3.

Table 8

| document group $E_u$ | Evaluation value $A(w_i, E_u)$ of index term $w_i$ | | | | |
|---|---|---|---|---|---|
| | $w_1$ | $w_2$ | $w_3$ | $w_4$ | $w_5$ |
| $E_1$ | 4 | 2 | 10 | 0 | 4 |
| $E_2$ | 12 | 2 | 3 | 0 | 8 |
| $E_3$ | 4 | 4 | 5 | 2 | 0 |

10. Calculating Concentration Degree

Then, the concentration degree calculation unit 4710 calculates the degree of concentration for each index term $w_i$ as follows (step S4613).

For each index term $w_i$, the sum of the evaluation value $A(w_i, E_u)$ in each document group $E_u$ for all the document groups $E_u$ that belong to the document group set S, in other words, the sum $\Sigma_{u=1}^{n} A(w_i, E_u)$ is calculated, and the ratio of the evaluation value $A(w_i, E_u)$ in each document group $E_u$ relative to the sum is calculated for each document group $E_u$ and each index term $w_i$ as follows.

$$A(w_i, E_u) / \Sigma_{u=1}^{n} A(w_i, E_u)$$

The sum of squares of the ratios in all the document groups $E_u$ that belong to the document group set S for each index term $w_i$ represents the concentration degree of the index term $w_i$ in the document group set S.

$$\Sigma_{u=1}^{n} \{ A(w_i, E_u) / \Sigma_{u=1}^{n} A(w_i, E_u) \}^2$$

This is represented as follows in the example of the above table, and the degree of concentration for each index term $w_i$ is calculated.

Table 9

| | | Ratio of evaluation value of index term $w_i$ relative to sum $A(w_i, E_u)/\Sigma_{u=1}^{3} A(w_i, E_u)$ | | | | |
|---|---|---|---|---|---|---|
| | | $w_1$ | $w_2$ | $w_3$ | $w_4$ | $w_5$ |
| document group $E_u$ | $E_1$ | 4/20 | 2/8 | 10/18 | 0/2 | 4/12 |
| | $E_2$ | 12/20 | 2/8 | 3/18 | 0/2 | 8/12 |
| | $E_3$ | 4/20 | 4/8 | 5/18 | 2/2 | 0/12 |
| concentration degree | | (16+144+16)/ 400=0.44 | (4+4+16)/ 64=0.38 | (100+9+25)/ 324=0.41 | (0+0+4)/ 4 = 1.00 | (16+64+0)/ 144=0.56 |

11. Calculating Shares

Then, the share calculation unit 4720 calculates the share of each index term $w_i$ in each document group $E_u$ as follows (step S4614).

In each document group $E_u$, the sum of the evaluation values A ($w_i$,$E_u$) of all the index terms $w_1$ selected from the above-described document group set S, i.e., $\Sigma_{i=1}^w A(w_i, E_u)$ is calculated. The ratio of the evaluation value A ($w_i$, $E_u$) of each index term $w_i$ relative to the sum is calculated as A ($w_i$, $E_u$) / $\Sigma_i$=1$^w$A ($w_i$, $E_u$). This is represented as follows in the example of the above table, and the share of each index term $w_i$ in each document group $E_u$ is determined.

Table 10

| | | Share of index term $w_i$ A ($w_i$, $E_u$) /$\Sigma_{i=1}^5$A ($W_i$, $E_u$) | | | | |
|---|---|---|---|---|---|---|
| | | $W_1$ | $W_2$ | $W_3$ | $W_4$ | $W_5$ |
| document group $E_u$ | $E_1$ | 4/20 | 2/20 | 10/20 | 0/20 | 4/20 |
| | $E_2$ | 12/25 | 2/25 | 3/25 | 0/25 | 8/25 |
| | $E_3$ | 4/15 | 4/15 | 5/15 | 2/15 | 0/15 |

12. Calculating Creativity Degrees

Then, values representing the creativity degrees of the index terms $w_i$ are calculated as follows.

The first inverse calculation unit 4730 calculates a function value of the inverse of the occurrence frequency of each index term $w_i$ in the document group set S (step S4615).

As the occurrence frequency in the document group set S, a document frequency DF(S) for example is used. As a function value of the inverse of the occurrence frequency, the inverse document frequency IDF(S) in the document group set S or a value (normalized IDF(S)) created by normalizing IDF(S) by all index terms extracted from a document group $E_u$ to be analyzed is used as a particularly preferable example. Herein, IDF(S) is the logarithm of "the inverse of DF(S) $\times$ the document number N(S) in the document group set S." An example of the normalization includes the use of a deviation value. The normalization is carried out to sort out the distribution, so that the creativity degree based on the combination with IDF(P) described above can be more easily calculated.

Then, the second inverse calculation unit 4740 calculates a function value of the inverse of the occurrence frequency of each index term $w_i$ in the large document set P including the document group set S (step S4616).

As a function value of the inverse of the occurrence frequency, IDF(P) or a value (normalized IDF(P)) created by normalizing IDF(P) by all index terms extracted from the document group $E_u$ to be analyzed is used as a particularly preferable example. An example of the normalization includes the use of a deviation value. The normalization is carried out to sort out the distribution, so that the creativity degree based on the combination with IDF(S) described above can be more easily calculated.

Then, the creativity degree calculation unit 4750 calculates a function value of { the function value of IDF(S) - the function value of IDF(P) } for each index term $w_i$ as a creativity degree (step S4617). If only IDF(S) and IDF(P) are used for calculating the creativity degree, one value is created for each index term $w_i$ as the creativity degree. If the normalized IDF(S) or the normalized IDF(P) normalized by the document group $E_u$, or GF($E_u$) is separately used as a weight, the creativity degree is calculated for each document group $E_u$ and for each index term $w_i$.

The creativity degree is particularly preferably provided as DEV in the following expression:

$$DEV = normalized\ GF(E_u) \times \frac{normalized\ IDF(S) - normalized\ IDF(P)}{normalized\ IDF(S) + normalized\ IDF(P)}$$

$$...(11)$$

The normalized GF ($E_u$) as the first factor of DEV is created by normalizing the global frequency GF($E_u$) of each index term $w_i$ in the document group $E_u$ to be analyzed by all the index terms extracted from the document group $E_u$ to be analyzed.

When normalization is carried out so that the normalized IDF(S)>0 and the normalized IDF(P)>0, the second factor of DEV is positive if the normalized value of IDF in the document group S is greater than the normalized value of IDF in the large document set P and negative if it is smaller. If IDF in the document group set S is large, that means the term is rare in the document group set S. Among such rare terms in the document group set S, terms with small IDF in the large document set P including the document group set S have creativity when they are used in the field related to the document group set S even if the terms are often used in other fields. Since being divided by {normalized IDF(S) + normalized IDF(P)}, the second factor of DEV is in the range from -1 to +1, which makes it easier to compare among different document groups $E_u$.

Since DEV is in proportion with the normalized $GF(E_u)$, it takes a larger value for a term with a higher frequency in the document group.

When the document group set S includes a plurality of document groups $E_u$ (u=1, 2, ...) in particular, and the creativity degree ranking is created in the document groups $E_u$ as the document group to be analyzed, common index terms in the document group set S are in lower places in the ranking and terms characteristic to each document groups $E_u$ are placed in higher places in the ranking, so that it would be advantageous to grasp the characteristic of each document group $E_u$.

13. Extracting Keywords

Then, the keyword extraction unit 4760 extracts keywords based on at least two indexes selected from four indexes Skey (w), the degree of concentration, the share, and the creativity degree obtained in the foregoing steps (step S4618).

Preferably, using all the four indexes Skey(w), the degree of concentration, the share, and the creativity degree, important terms are extracted as the index terms $w_i$ in the document group $E_u$ as they are sorted into "unimportant terms," and "technical region terms," "main terms," "creative terms," and "other important terms" among important terms. A particularly preferable method of sorting is as follows.

For the first determination, Skey(w) is used. In each document group $E_u$, the descendent ranking of Skey (w) is created, and keywords below a prescribed place in the ranking are determined as "unimportant terms" and removed from the range of keyword extraction. Keywords within the prescribed order range are important terms in each document group $E_u$, and therefore determined as "important terms." Then, these terms will further be sorted in the following determination.

For the second determination, the degree of concentration is used. Terms with low concentration degrees are terms scattered in the entire document group set, and therefore the terms can be understood as widely representing the technical area to which the document group to be analyzed belong. Therefore, the ascending ranking of the concentration degrees is created in the document group set S, and those in places in the ranking equal to or higher than a prescribed place in the ranking are determined as "technical region terms." From the important terms in each document group $E_u$, keywords in coincidence with the technical region terms are sorted as "technical region terms" in the document group $E_u$.

For the third determination, the share is used. Terms with high shares have greater shares in the document group to be analyzed than other terms, and therefore can be understood as terms well explaining the document group to be analyzed (main terms). Therefore, the share descending ranking for the important terms that are not sorted by the second determination is created in each document group $E_u$, and terms within a prescribed place in the ranking are determined as "main terms."

For the fourth determination, the creativity degree is used. In each document group $E_u$, the creativity degree descending ranking for the important terms that are not sorted by the third determination is created and terms within a prescribed place in the ranking are determined as "creative terms." The remaining important terms are determined as "other important terms."

The determination process can be represented by a table as follows:

Table 11

| category/ attribute | Skey(w) | concentration degree | explaining degree | creativity degree |
|---|---|---|---|---|
| unimportant term | low | | | |
| technical region term | high | low | | |
| main terms | | high | high | |
| creative term | | | low | high |
| other important term | | | | low |

In the foregoing determination, Skey(w) is used as an index for importance degrees used in the first determination, while another index indicating the importance degrees in the document group may be used. For example, GF(E)*IDF(P) may be used.

In the foregoing determination, the four indexes, the importance degree, the degree of concentration, the share, and the creativity degree are used, while at least arbitrary two of these indexes may be used to sort the index terms.

As described above, the keywords are sorted using the four indexes, the importance degree, the degree of concentration, the share, and the creativity degree. Eventually, cluster information including the title, the number of publications, the total of IPC classes (top five), and the total of applicants (top five) for each cluster and the important keywords in the clusters is stored in the recording device in the second analysis server 514, and provided to the management server 512. The management server 512 provides the result of processing by the second analysis server 514 to the file creating server 516.

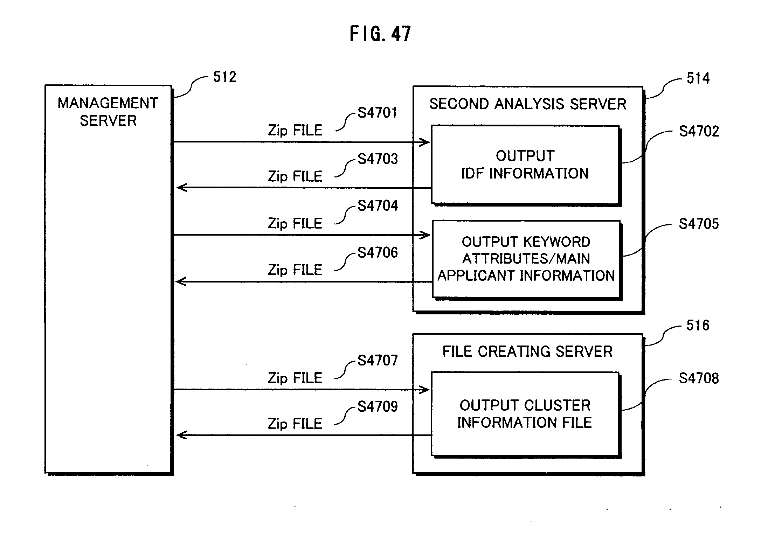

The flow of cluster information output by the management server 512, the second analysis server 514, and the file creating server 516 will be described. Fig. 47 shows the flow of processing until the cluster information is output. As shown in Fig. 47, the management server 512 forms the result of processing by the first analysis server 513 for example into a Zip file and transfers the file to the second analysis server 514 (step S4701).

The second analysis server 514 carries out processing to output IDF information (step S4702). More specifically, the second analysis server 514 operates as follows.

(1) It obtains the result of leaving spaces between keywords in each publication based on the list of publications created at the time of outputting a structure diagram included in the file received from the management server 512.
(2) It calculates IDF (for the population) and IDF (for all the publications) for each keyword obtained in the above (1) .
(3) It creates a file including a file holding the values obtained in the above (2) (such as a CSV file) and all the files included in the file (Zip file) received from the management server 512 and returns the file to the management server 512 (step S4703).

The management server 512 further transfers a file (such as a Zip file) including the result of processing by the first analysis server 513 and the IDF information in step S4702 again to the second analysis server 514 (step S4704).

Upon receiving the file, the second analysis server 514 outputs keyword attributes and main applicant information (step S4705). More specifically, the second analysis server 514 operates as follows:

(1) It obtains the degrees of concentration for each keyword and the ranking of degrees of concentration.
(2) It obtains the following values for each cluster and each keyword attached to a cluster. Importance degrees and importance degree ranking Explaining degree and explaining degree ranking
Creativity degree and creativity degree ranking (for which the IDF information is referred to).
(3) It obtains main applicants, the number of applications, and the ranking of main applicants for each cluster.
(4) It obtains main IPC sub groups for each cluster, the number of publications, and the ranking of main IPC sub groups for each cluster.
(5) It creates a file in the form that includes each file holding the values obtained in the above (1) to (4)(such as a CSV file) and all the files in the file (Zip file) received from the management server and returns the file to the management server 512 (step S4706).

The management server 512 transfers a file (such as a Zip file) including the results of processing by the first analysis server 513 and the second analysis server 514 to the file creating server 516 (step S4707).

The file creating server 516 creates a cluster information file based on the received file (step S4708). More specifically, the file creating server 516 operates as follows:

(1) Based on the values calculated in step S4705 in the second analysis server 514, it determines which category ("technical region," "main aspects (main terms)," "creative aspects (creative terms)," and "others") the keywords attached to the clusters belong to and sets the keywords to their appropriate items (categories).
(2) It sets information about main applicants in the clusters and the main IPC sub groups to the items.
(3) After carrying out the above (1) and (2) for each cluster, a table form file in which keywords or the like are set in the boxes and a file is created in a form that includes the table form file and all the files included in the file (Zip file) received from the management server 512 and returns the file to the management server 512 (S4709).

In this way, the management server 512 can obtain the final file (Zip file) including all the results of processing. The management server 512 transfers the final file to the web server 511. The web server 511 creates a mail having the file received from the management server 512 as an attached file and transmits the mail to the client 502.

[Other Embodiments]

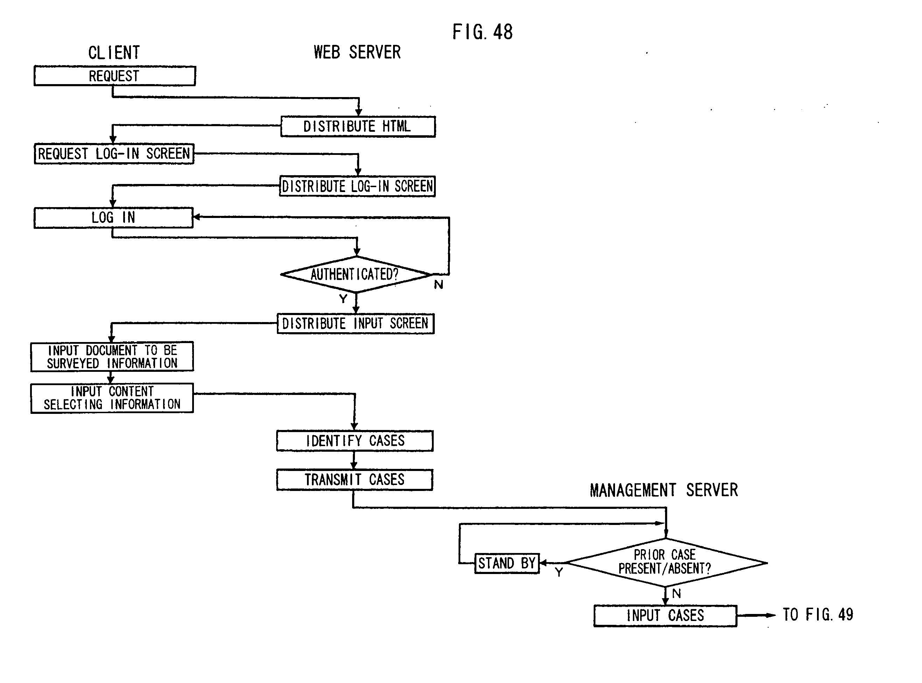

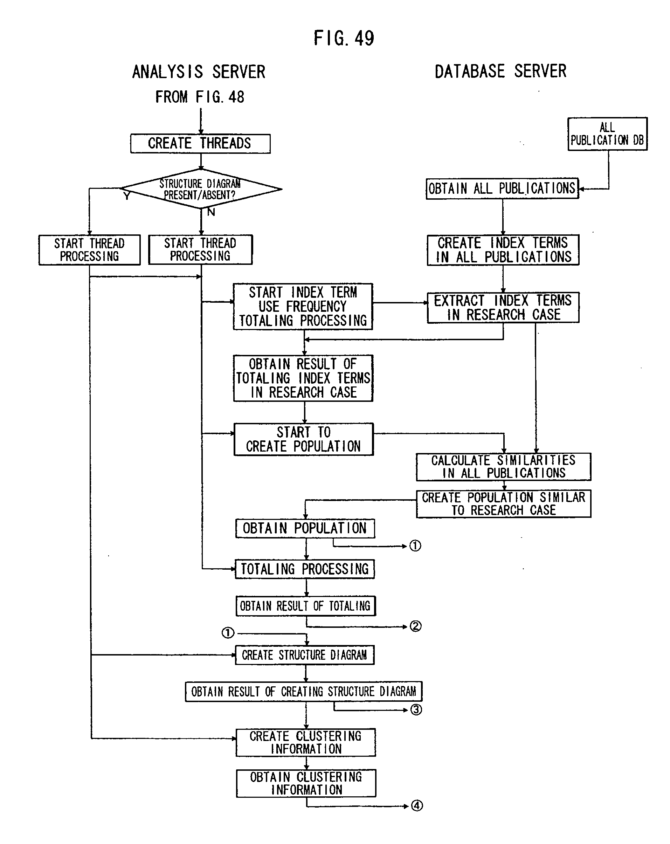

Referring to Figs. 48 to 50, the processing carried out by the client, the web server, the management server, the first and second analysis servers, and the database server will be described. In the following paragraphs, the first analysis server and the second analysis server are not separated and generically referred to as "analysis server." However, the analysis server may include two analysis servers, i.e., the first analysis server and the second analysis server, so that distributed processing can be carried out. According to the embodiment, the analysis server creates a thread, so that various kinds of processing can be carried out simultaneously or in parallel, in other words, a multi-thread processing function is provided.

The web server can serve as an interface with a client and receives and transmits data from and to a client. The web server creates information about a case on which an information analysis report is to be created, in other words, information about a document to be surveyed (hereinafter referred to as "research case information") based on a user input and applies the information to the management server.

The management server queues research cases and requests the analysis server in the order of input. The management server has a queuing mechanism to request the analysis server.

The analysis server carries out processing such as population extraction, various totaling processing, and creating the structure diagram and clustering information.

As shown in Fig. 48, the web server responds to a request from a client to carry out HTML distribution. The client transmits a request for a log-in screen according to the user operation and the web server responds to the log-in screen request to distribute the log-in screen to the client. In response to the log-in from the client, the web server authenticates and if authentication cannot be made, the process returns to the log-in by the user. On the other hand, if the authentication is made, the web server distributes an input screen including document to be surveyed information input box and the request content selecting box to the client.

Similarly to the second embodiment, as shown in Fig. 37A, the search screen includes boxes 3701 to 3704 and a text input box 3705 to specify a patent document. According to the embodiment, the document to be surveyed may be patent laid-open publications, patent publications, or user-input text. As the text, a summary of technology on which the user wishes to file an application may be input.

If the document to be surveyed is a patent document such as laid-open publication, the user operates the client 502 to input necessary information to the boxes 3701 to 3704. Alternatively, the user may input information to be researched in the text input box 3705.

Note that the box 3706 is used to provide service such as emphasizing similar publications for a period based on an input in the box 3706 in a different color at the time of listing similar publications.

When the web server receives the document to be surveyed information and the content selecting information input by the client operated by the user, the web server identifies the case based on the received document to be surveyed information and the content selecting information and transmits the case to the management server. The management server determines the presence/absence of a preceding case being processed by the analysis server and stands by if there is a preceding case. On the other hand if there is no preceding case, the case is input to the analysis server. According to the embodiment, once the document to be surveyed is determined, the research case information is transmitted to the management server from the web server. The management server queues research cases by the queuing mechanism, requests the analysis server for the research case to be processed next and provides the research case data.

As shown in Fig. 49, the analysis server determines the presence/absence of the structure diagram from the content selecting information and creates necessary threads to carry out processing. In this example, a document index term totaling processing thread, a similar document population creating thread, a document attribute totaling processing thread, a structure diagram creating processing thread, and a cluster information creating processing thread are created. These threads are created simultaneously or in parallel. Alternatively, at least one of them may be created.

The database server obtains all the publications from an all publication database (DB) and creates index terms for all the publications (all publication keywords).

The analysis server obtains research case index terms extracted by the database server at the time of carrying out thread processing. Then, the process of totaling the use frequencies of the research case index terms in the documents is carried out. In this way, the analysis server obtains the result of research case index term totaling processing.

Then, the analysis server starts to create a population. The database server responds to a request to start creating a population from the analysis server to calculate all publication similarities based on the created index terms for each of the documents included in all the publications and the obtained result of totaling the research case index terms. The similarity calculation is the same as that described in connection with the first embodiment and therefore the description is not provided. A research case similar population is created from a document group of 3000 documents having the largest all publication similarity ratios. The database server returns the research case similar population to the analysis server. In this way, the analysis server obtains the research case similar population.

The analysis server carries out totaling processing and obtains at least one of the totaling results of the ranking of similarities in the similar document population, the number of documents in the similar document population for each document attribute included in the bibliographic information of the document to be surveyed, the transition of the number of documents in the similar document population or various rankings for each of the document attributes, and an index document frequency scatter diagram.

Similarly to the second embodiment, the analysis server carries out, as totaling, ranking totaling (step S3901), time-series totaling (step S3902), and matrix tabulation (step S3903).

As shown in Fig. 39, the ranking totaling includes keyword totaling, totaling related to applicants, and totaling related to IPC. In the keyword totaling, the distribution graphs as shown in Figs. 18 and 19 are created. The analysis server obtains information about a prescribed number of important keywords (for all the publications) in the descending order of importance degrees and creates a graph representing the number of publications that use each keyword (index term) for each important keyword (for all the publications) (Fig. 18). The analysis server obtains information about the important keywords (for the population) from the recording device and creates a graph representing the number of publications that include each keyword (index terms) for each important keyword (Fig. 19).

The analysis server obtains information about the population from the recording device and totals the publications of the population on an applicant basis (see Figs. 11 and 12). The analysis server obtains information about the population from the recording device and creates a graph in which IPC classes in the publications of the population are summed up for each main group (Fig. 13) and a graph in which the IPC classes are summed up for each of all the classes and sub class in IPC (Fig. 14). The totaling results (the tables and the graphs) are stored in the recording device in the analysis server.

The analysis server obtains information about the population from the recording device and totals the number of applications by top 10 applicants based on the number of filed applications for each filing year and creates a graph representing the transition of the numbers (Fig. 20) and a table representing the cumulative numbers and the numbers on a single year basis (Fig. 21). The analysis server obtains information about the population from the recording device and creates a graph in which for the top five classes of the IPC attached to as classes or sub classes in the publications of the population, the number of the applications are summed up for each year (Fig. 22) and a table representing the number of applications for each single filing year and the cumulative total (Fig. 23). These totaling results are also stored in the recording device in the analysis server.

Furthermore, the analysis server obtains important keywords (for all the publications) from the recording device and creates a graph representing the accumulation of the yearly use frequencies of the important keywords (for all the publications) (Fig. 27) and a table representing the total of the keywords on a single year basis and the cumulative total (for all the publications)(Fig. 28). The analysis server obtains important keywords (for the population) from the recording device and creates a graph representing the accumulation of the yearly use frequency of each of the important keywords (for the population) (Fig. 29) and a table (Fig. 30) representing the total of the important keywords on a single year basis and the cumulative total (for the population). These graphs and tables are also stored in the recording device in the analysis server.

The analysis server creates a graph based on the totaled result of the number of applications for each year in the population in which the abscissa represents the number of publications for each year and the ordinate represents the increase ratio obtained by comparison to the number of applications in the previous year (Fig. 25). In the graph in Fig. 25, the sizes of the plotted circles indicate the accumulation of the numbers of applications. Similarly, the analysis server creates a graph based on the totaled result of the number of applications provided with certain IPC (IPC main group) in the population in which the abscissa represents the number of applications for each year and the ordinate represents the increase ratio obtained by comparison to the number of applications in the previous year (Fig. 26). In Fig. 26, the sizes of the plotted circles indicate the accumulation of the numbers of applications. The graph created in this way is stored in the recording device of the analysis server.

Hereinafter, the matrix tabulation will be described. The analysis server further obtains the information of the population from the recording device and refers to the IPC attached to the applications of the top ten applicants based on the number of applications in the population to create the number of applications provided with the IPC groups into a table in a matrix form including the rows of applicants and the columns of IPC main groups for each applicant and based on the applications by each applicant (see Fig. 15). Using the same information in Fig. 15, a table separately showing the number of publications, the number of registered patents, and the number of utility models (Fig. 16) is also created. The analysis server obtains the information of the population from the recording device, calculates the number of applications attached with the same IPC main group as the IPC class of the document to be surveyed in the publications by the top 20 applicants based on the number of applications in the population, and creates a graph representing the number of applications for each applicant (Fig. 17). In Fig. 17, it is desirable to display separately the number of publications, the number of registered patents, and the number of utility models for each applicant. The result of the matrix tabulation is also stored in the analysis server.

Although not shown, after various kinds of totaling processing ends, the analysis server may obtain the information of the population from the recording device and calculate the inside population similarity measures (step S3904). The inside population similarity measure is the similarity (similarity measure) between the document to be surveyed and each of the documents that belong to the population.

The analysis server carries out the process of calculating coordinates for a frequency scatter diagram (step S3905). As shown in Fig. 31, the frequency scatter diagram represents the distribution of keywords included in the document to be surveyed. The calculation of a coordinate for each keyword for the frequency scatter diagram will be described in detail by referring to the flowchart in Fig. 40. Fig. 40 sequentially shows all the process steps necessary for calculating a coordinate for each keyword for the ease of understanding. Therefore, it is not that all the process steps shown in Fig. 40 are carried out in S3905 in Fig. 39. More specifically, in S3905 in Fig. 39, a value already calculated in the analysis server and stored in the recording device is not re-calculated but used as it is and only process steps that have not been carried out before the processing in step S3905 are carried out.

As shown in Fig. 40, index terms are extracted from a document to be surveyed or documents to be compared (step S4001). Then, the document frequencies DF(P) in P based on the index terms in all the documents (all the documents to be compared) P are calculated (step S4002). The DF(P) corresponds to the keyword importance degree.

Thereafter, the product of TF(d) (the occurrence frequencies of d's index terms ($d_1$, ... , $d_x$) in d) and IDF(P) (the inverse of DF(P) $\times$ the logarithm of the number of documents: ln[N/DF(P)]), i.e., the document vector (d) is calculated (step S4003). Similarly, the product of TF(P) (the occurrence frequencies of P's index terms ($P_1$, ..., $P_{ya}$) in P) and IDF (P), i.e., the document vector (p) is calculated (step S4004).

When the document vector (d) and the document vector (p) are calculated, the inner product of the vectors is obtained as similarity measures (step S4005). Furthermore, a prescribed number of documents are extracted from the documents to be compared P as a population S in the descending order of similarity measures relative to the document to be surveyed d and the information of the documents is stored in the recording device (step S4005). Thereafter, the keyword importance degree DF(S) (the document frequency in S based on S's index terms) is calculated (step S4006).

Thereafter, for each of the index terms ($d_1$, ..., $d_x$) of the document to be surveyed d, the function value IDF of the document frequency is obtained for the documents to be compared P and the population S (steps S4007 and S4008). In step S4007, IDF ($d_1$; P), IDF ($d_2$; P), ... , IDF($d_x$; P) are obtained, and in step S4008, IDF ($d_1$; S), IDF($d_2$;S), ..., IDF ($d_x$;S) are obtained. The analysis server creates a plane by IDF(P) and IDF(S), and for example creates a frequency scatter diagram having the index terms provided in prescribed positions on the plane where the x-axis represents the IDF(P) and the y-axis represents the IDF(S) based on the values of IDF(P) and IDF(S) for each of the index terms ($d_1$, ..., $d_x$) (step S4009).

Note that from step S4009, in the frequency scatter diagram (IDF plan view), the index terms are arranged (scattered), while the scattered index terms are sometimes unevenly localized and become less viewable. Therefore, according to the second embodiment, the density of the index terms provided on the plane is inspected, and if the density in a prescribed region exceeds a prescribed value, the analysis server widens the scale on the axis in the region to expand the region and narrows the scale on the axis in the other region to compress the other region. Therefore, when a region is expanded and the other region is compressed, the analysis server carries out coordinate transformation (step S4010). The IDF plan view has a diamond shape, which can look unusual as a phenogram or can be inconvenient in handling. Therefore, the analysis server may carry out coordinate transformation, so that the plane can be represented in a square form. The information of the frequency scatter diagram is also stored in the recording device in the analysis server.

The analysis server creates a tree-like diagram based on the similarity measures of the documents included in the similar document population and carries out clustering to create a structure diagram. The analysis server also creates the clustering information of the structure diagram including the document to be surveyed based on the created structure diagram data.

As shown in Fig. 49, the information of the research case similar population is used for creating a structure diagram and clustering information.

A more detailed description of the way of creating a patent structure diagram will be the same as that given in connection with the second embodiment and therefore the description is omitted. In this example, with reference to the flowchart in Fig. 42, a general idea of the process of creating a patent structure diagram in the analysis server will be described.

The document read out unit 4110 reads out a plurality of document elements to be analyzed from the document storage unit of the recording device 4103 (step S4210). According to the embodiment, examples of the document elements to be analyzed include population documents or a document to be surveyed and population documents.

Then, the time data extraction unit 4120 extracts the time data of each element from the document element group read out in the document reading step S4210 (step S4220).

The index term data extraction unit 4130 extracts index term data as the content data of each document element from the document element group read out in the document reading step S4210 (step S4230). The index terms are extracted in the same manner as the first embodiment.

Then, the similarity calculation unit 4140 operates similarity measures between the document elements based on the index term data of each of the document elements extracted in the index data extracting step S4230 (step S4240). The similarity measure (similarity) calculation has been described and therefore the description is omitted.

Then, the tree-like diagram creation unit 4150 creates a tree-like diagram of the document element group to be analyzed based on the similarity measures operated in the similarity measure operating step S4240 (step S4250). As the tree-like diagram, a dendrogram in which the similarity measures between the document elements are reflected on the height of the connection positions (connection distances) is desirably used. A specific example of a method of creating such a dendrogram includes a known Ward method.

The cutting condition read out unit 4160 then reads out a tree-like diagram cutting condition recorded in the condition recording unit in the recording device 4103 (step S4260).

The cluster extraction unit 4170 then cuts the tree-like diagram created in the tree-like diagram creating step S4250 based on the cutting condition read out in the cutting condition reading step S4260 and a cluster is extracted (step S4270).

The arrangement condition read out unit 4180 reads out a document element arrangement condition in the cluster recorded in the condition recording unit in the recording device 4103 (step S4280).

The inside cluster element arranging unit 4190 then determines the arrangement of the document elements in the cluster extracted in the cluster extracting step S4270 based on the document element arrangement condition read out in the arrangement condition reading step S4280 (step S4290). The structure diagram according to the embodiment is completed by thus determining the arrangement in the cluster. Note that the arrangement condition may be in common for all the clusters. Therefore, if step S4280 is carried out once for one cluster, the step does not have to be carried out again for the other clusters.

More specifically, the process of creating the structure diagram will be described. According to the embodiment, after parent clusters are extracted by cutting a tree-like diagram at a cutting height $\alpha$ determined by a certain method, a tree-like diagram is created again using only document elements that belong to each of the parent clusters in order to divide each of the parent clusters into child clusters. At the time of creating the partial tree-like diagram, an index term dimension in which the deviation of the component of the document element vector in the parent cluster takes a value smaller than a value determined by a prescribed method is removed before analysis.

Fig. 43 is a flowchart for use in illustrating in detail the process of extracting a cluster according to the embodiment but this is the same as that of the second embodiment and therefore the description is omitted.

As the analysis server carries out the above described processing, the patent structure diagram as shown in Fig. 32 can be obtained. Then, the analysis server creates the clustering information of the structure diagram based on the research case data and the information of the patent structure diagram.

Now, the process of obtaining cluster information will be described. The definition of terms and abbreviations used in the following paragraphs will be described. Cluster information includes titles, the number of publications, the total of IPC classes (top five), and the total of applicants (top five) and cluster important keywords for each cluster. The important keywords represent the ten most important keywords extracted from all the publications that belongs to the cluster and the keywords are divided into the following four kinds for display.

Technical Region Terms: Among the cluster important keywords, those used in common for other clusters. Keywords used in common among many clusters are generally keywords that represent the technical region to which the clusters belong.

Main Terms: Among the cluster important keywords excluding the "technical region terms," those particularly used for the cluster. The main terms are not much used for other clusters, and often represent the main technical elements of the cluster. The main terms typically distinguish the cluster from other clusters.

Characteristic Terms: It is often the case that the cluster important keywords excluding the "technical region terms" and the "main terms" are keywords related to means or structures. Among all, general terms much used but not much used in the group of publications to be analyzed (with the top 300 all publication similarity measures) would be keywords that could suggest characteristic aspects in means or structures. Such keywords are calculated according to a prescribed standard and indicated as "characteristic terms."

Other Important Terms: Terms that do not correspond to any one of the above three kinds among the cluster important keywords. It is often the case that "the other important terms" are technical terms that do not belong to any of the above-three aspects and related to technical terms related to means and structures.

Hereinafter, the configuration of a processing device used to extract a keyword will be described with reference to the block diagrams in Figs. 45 and 47. In the following description, for the ease of understanding the content of the processing, all the necessary functions for extracting keywords among the functions of the analysis server are described.

The document read out unit 4510 reads out a document group E including a plurality of documents $D_1$ to $D_{N(E)}$ to be analyzed from the document storage unit in the recording device 4503 based on a reading condition stored in the condition recording unit in the recording device 4503. The data of the read out document group is directly sent to the index term extraction unit 4520 to be used for processing therein and sent to the work result storage unit in the recording device 4503 to be stored therein.

Note that the data sent to the index term extraction unit 4520 or the work result storage unit from the document read out unit 4510 may be the entire data including the document data of the read out document group E. Alternatively, the data may be only the bibliographic data (such as application numbers or publication numbers in patent documents) used to specify each of the documents D that belong to the document group E. In the latter case, if necessary in subsequent processing, the data of each document D may be read out again from the document storage unit based on the bibliographic data.

The index term extraction unit 4520 extracts index terms in each document from the document group read out by the document read out unit 4510. The index term data of each of the documents is directly sent to the high-frequency extraction unit 4530 to be used for processing therein and sent to the work result storage unit in the recording device 4503 to be stored therein.

The high frequency extraction unit 4530 extracts a prescribed number of index terms with a large weight whose high occurrence frequency in the document group E is included in evaluation based on the index terms in each document extracted in the index term extraction unit 4520 and according to a high frequency term extracting condition stored in the condition recording unit in the recording device 4503.

More specifically, for each index term, the occurrence frequency GF(E) in the document group E is calculated. The IDF (P) of each index term is calculated, and GF(E)*IDF(P), the product of GF(E) and IDF(P) is preferably calculated. Then, a prescribed number of index terms having larger values as a result for GF(E) or GF(E)*IDF(P) as the weight of each of the calculated index terms are extracted as high frequency terms.

The data of the extracted high frequency terms is directly sent to the high frequency term-index term co-occurrence degree calculation unit 4540 to be used for processing therein and also sent to the work result storage unit in the recording device 4503 to be stored therein. The GF(E) of the calculated index terms and the IDF(P) of the index terms desired to be calculated are preferably sent to the work result storage unit in the recording device 4503 and stored therein.

The high frequency term-index term co-occurrence calculation unit 4540 calculates co-occurrence degrees in the document group E based on the presence/absence of co-occurrence on a document basis between the high frequency terms extracted by the high frequency term extraction unit 4530 and the index terms extracted by the index term extraction unit 4520 and stored in the work result storage unit. If p index terms are extracted and q high frequency terms are extracted from the p index terms, matrix data of p rows and q columns results.

The co-occurrence degree data calculated by the high frequency term-index term co-occurrence degree calculation unit 4540 is directly sent to a clustering unit 4550 and used for processing therein or sent to the work result storage unit in the recording device 4503 and stored therein.

The clustering unit 4550 cluster-analyzes q high frequency terms according to a clustering condition stored in the condition recording unit in the recording device 4503 based on the co-occurrence degree data calculated by the high frequency term-index term co-occurrence degree calculation unit 4540.

In order to carry out the cluster analysis, similarity measures for the co-occurrence degrees between the q high frequency terms and the index terms are operated.

Then, based on the result of calculation of the similarity measures and according to a tree-like diagram creating condition stored in the condition recording unit in the recording device 4503, a tree-like diagram connecting the high frequency terms in a tree-like form is created. As such a tree-like diagram, a dendrogram in which the dissimilarity measures between the high frequency terms are reflected as the height of the connecting positions (connecting distances) is desirably created.

Then, according to a tree-like diagram cutting condition recorded in the condition recording unit in the recording device 4503, the created tree-like diagram is cut. As the result of cutting, the q high frequency terms are clustered based on the similarity measures for the co-occurrence degree with the index terms. Individual clusters created by the clustering will be referred to as "grounds" $g_h$ (h=1, 2, ..., b).

The ground data formed by the clustering unit 4550 is directly sent to an index term-ground co-occurrence degree calculation unit 4560 and used for processing therein or sent to the work result storage unit in the recording device 4503 and stored therein.

The index term-ground co-occurrence degree calculation unit 4560 calculates the co-occurrence degrees between the index terms extracted by the index term extraction unit 4520 and stored in the work result storage unit in the recording device 4503 and the grounds formed by the clustering unit 4550. The co-occurrence data calculated for each index term is directly sent to a key(w) calculation unit 4570 and used for processing therein or sent to the work result storage unit in the recording device 4503 and stored therein.

The key(w) calculation unit 4570 calculates key(w) that is the evaluation score of each index term based on the co-occurrence degrees of the index terms and the grounds calculated by the index term-ground co-occurrence degree calculation unit 4560. The calculated key(w) data is directly sent to a Skey (w) calculation unit 4580 and used for processing therein or sent to the work result storage unit in the recording device 4503 and stored therein.

The Skey(w) calculation unit 4580 calculates Skey(w) scores based on the key (w) scores of the index terms calculated by the key(w) calculation unit 4570, the GF(E) of the index terms and the IDF(P) of the index terms calculated by the high frequency term extraction unit 4530 and stored in the work result storage unit in the recording device 4503. The calculated Skey(w) data is sent to the work result storage unit in the recording device 4503 and stored therein.

An evaluation value calculation unit 4700 reads index terms $w_i$ in each document extracted by the index term extraction unit 4520 regarding a set of document groups S including a plurality of document groups $E_u$. Alternatively, the evaluation value calculation unit 4700 reads out the Skey(w) of the index terms calculated for each of the document $E_u$ by the Skey (w) calculation unit 4580 from the work result storage unit. If necessary, the evaluation value calculation unit 4700 may read out the data of each document group $E_u$ read out by the document read out unit 4510 from the work result storage unit and count the number of documents $N(E_u)$. $GF(E_u)$ and IDF(P) calculated in the process of extracting high frequency terms by the high frequency term extraction unit 4530 may be read out from the work result storage unit.

The evaluation value calculation unit 4700 calculates an evaluation value A ($W_i$, $E_u$) based on the occurrence frequency of each index term $w_i$ in each of the document groups $E_u$ according to the read out information. The calculated evaluation values are sent to the work result storage unit and stored therein or directly sent to a concentration degree calculation unit 4710 and a share calculation unit 4720 and used for processing therein.

The concentration degree calculation unit 4710 reads out the evaluation value $A(w_i, E_u)$ for each of the index terms $w_i$ in each of the document group $E_u$ calculated by the evaluation value calculation unit 4700 from the work result storage unit or receives the value directly from the evaluation value calculation unit 4700.

The concentration degree calculation unit 4710 calculates the concentration degree of the distribution of each of the index terms $w_i$ in the document group set S based on the obtained evaluation $A(w_i, E_u)$. The concentration degree is created by calculating the sum of the evaluation values A ($w_i$, $E_u$) of each index term $w_i$ in all the document groups $E_u$ that belong to the document group set S and the ratio of the evaluation value $A(w_i, E_u)$ in each document group $E_u$ relative to the sum for each document group $E_u$ and creating the squares of the ratios and the sum of the squares of the ratios in all the document group $E_u$ that belong to the document group set S. The calculated concentration degrees are sent to the work result storage unit and stored therein.

The share calculation unit 4720 reads out the evaluation value A ($w_i$, $E_u$) of each index term $w_i$ in each document group $E_u$ calculated by the evaluation value calculation unit 4700 from the work result storage unit or directly receives the value from the evaluation value calculation unit 4700.

The share calculation unit 4720 calculates the share of each index term $w_i$ in each document group $E_u$ based on the obtained evaluation value $A(w_i, E_u)$. The share is created by summing up the evaluation value $A(w_i, E_u)$ of each index term $w_i$ for all the index terms $w_i$ extracted from each document group $E_u$ that belongs to the above-described document group set S, and calculating the ratio of the evaluation value $A(w_i,E_u)$ of each index term $w_i$ relative to the sum. The calculated concentration degree is sent to the work result storage unit and stored therein.

The first inverse calculation unit 4730 reads out the index term $w_i$ in each document extracted in the index term extraction unit 4520 regarding the document group set S including a plurality of document groups $E_u$ from the work result storage unit.

The first inverse calculation unit 4730 calculates a function value of the inverse of the occurrence frequency of each index term $w_i$ in the document group set S (such as normalized IDF(S) that will be described) based on the data of the index terms $w_i$ in each document in the read out document group set S. The calculated function value of the inverse of the occurrence frequency in the document group set S is sent to the work result storage unit and stored therein or directly sent to a creativity degree calculation unit 4750 and used for processing therein.

The second inverse calculation unit 4740 calculates a function value of the inverse of the occurrence frequency in a large document set including the document group set S. As the large document set, all the documents P are used. In this case, IDF(P) calculated in the process of extracting a high frequency term in the high frequency term extraction unit 4530 is read out from the work result storage unit and a function value thereof (such as normalized IDF(P) that will be described) is calculated. The calculated function value of the inverse of the occurrence frequency in the large document set P is sent to the work result storage unit and stored therein or directly sent to the creativity degree calculation unit 4750 and used for processing therein.

The creativity degree calculation unit 4750 reads out the function values of the inverses of the occurrence frequencies calculated in the first inverse calculation unit 4730 and the second inverse calculation unit 4740 from the work result storage unit or directly receives the values from the first inverse calculation unit 4730 and the second inverse calculation unit 4740. GF(E) calculated in the process of extracting a high frequency term in the high frequency extraction unit 4530 is read out from the work result storage unit.

The creativity degree calculation unit 4750 calculates as a creativity degree a function value of what is obtained by subtracting the calculation result of the second inverse calculation unit 4740 from the calculation result of the first inverse calculation unit 4730. The function value may be obtained by subtracting the result of calculation by the second inverse calculation unit 4740 from the result of calculation by the first inverse calculation unit 4730 and dividing the result by the sum of the calculation results by the first inverse calculation unit 4730 and the second inverse calculation unit 4740 or by multiplying the result by GF ($E_u$) in each document group $E_u$. The calculated creativity degree is sent to the work result storage unit and stored therein.

The keyword extraction unit 4760 reads out various kinds of data including Skey (w) calculated by the Skey (w) calculation unit 4580, the concentration degrees calculated by the concentration degree calculation unit 4710, the shares calculated by the share calculation unit 4720, and the creativity degrees calculated by the creativity degree calculation unit 4750 from the work result storage unit.

The keyword extraction unit 4760 extracts keywords based on two or more indexes selected from the four indexes, the read out Skey(w), the concentration degrees, the shares, and the creativity degrees. The keywords may be extracted for example by determining whether the total values of the selected multiple indexes is not less than a prescribed threshold or within a prescribed range of ranks. The extracted keyword data is sent to the work result storage unit in the recording device 4503 and stored therein. Thereafter, clustering information is created based on combinations of multiple selected indexes and keywords extracted for each of the indexes.

More specifically, the keyword extraction unit 4760 creates clustering information based at least two indexes selected from the four indexes Skey(w), the degrees of concentration, the shares, and the creativity degrees obtained in the foregoing steps and the extracted keywords.

Preferably, using all the four indexes Skey(w), the degrees of concentration, the shares, and the creativity degrees, the index terms $w_i$ in the document group $E_u$ are sorted into "unimportant terms," and "technical region terms," "main terms," "creative terms," and "other important terms" among important terms and the clustering information is created accordingly. A particularly preferable method of sorting is as follows.

For the first determination, Skey(w) is used. In each document group $E_u$, the descendent ranking of Skey (w) is created, and keywords below a prescribed place in the ranking order are determined as "unimportant terms" and removed from the range of keyword extraction. Keywords within the prescribed order range are important terms in each document group $E_u$, and therefore determined as "important terms. " Then, these terms will further be sorted in the following determination.

For the second determination, the degree of concentration is used. Terms with low concentration degrees are terms scattered in the entire document group set, and therefore the terms can be understood as widely representing the technical area to which the document group to be analyzed belong. Therefore, the ascending ranking of the concentration degrees in the document group set S is created, and those in places in the ranking equal to or lower than a prescribed place in the ranking are determined as "technical region terms." From the important terms in each document group $E_u$, keywords in coincidence with the technical region terms are sorted as "technical region terms" in the document group $E_u$.

For the third determination, the share is used. Terms with high shares have greater shares in the document group to be analyzed, and therefore can be understood as terms well explaining the document group to be analyzed (main terms). Therefore, in each document group $E_u$, the share descending ranking for the important terms that are not sorted by the second determination is created, and terms within a prescribed range in the ranking are determined as "main terms."

For the fourth determination, the creativity degree is used. In each document group $E_u$, the creativity degree descending ranking for the important terms that are not sorted by the third determination is created and terms within a prescribed range in the ranking are determined as "creative terms." The remaining important terms are determined as "other important terms."

The determination process described above can be represented by Table 11.

In the foregoing determination, Skey(w) is used as an index for importance degree used in the first determination, while another index indicating the importance degree in the document group may be used. For example, GF(E)*IDF(P) may be used.

In the foregoing determination, the four indexes, the importance degree, the degree of concentration, the share, and the creativity degree are used, while at least arbitrary two of these indexes may be used to sort the index terms.

As described above, the keywords are sorted using the four indexes, the importance degree, the degree of concentration, the share, and the creativity degree. Eventually, cluster information including titles, the number of publications, the total of IPC classes (top five), and the total of applicants (top five) for each cluster and the important keywords in the clusters is stored in the recording device in the analysis server and provided to the management server. The process of extracting keywords (see Fig. 46) is the same as that in the second embodiment and therefore the description is omitted.

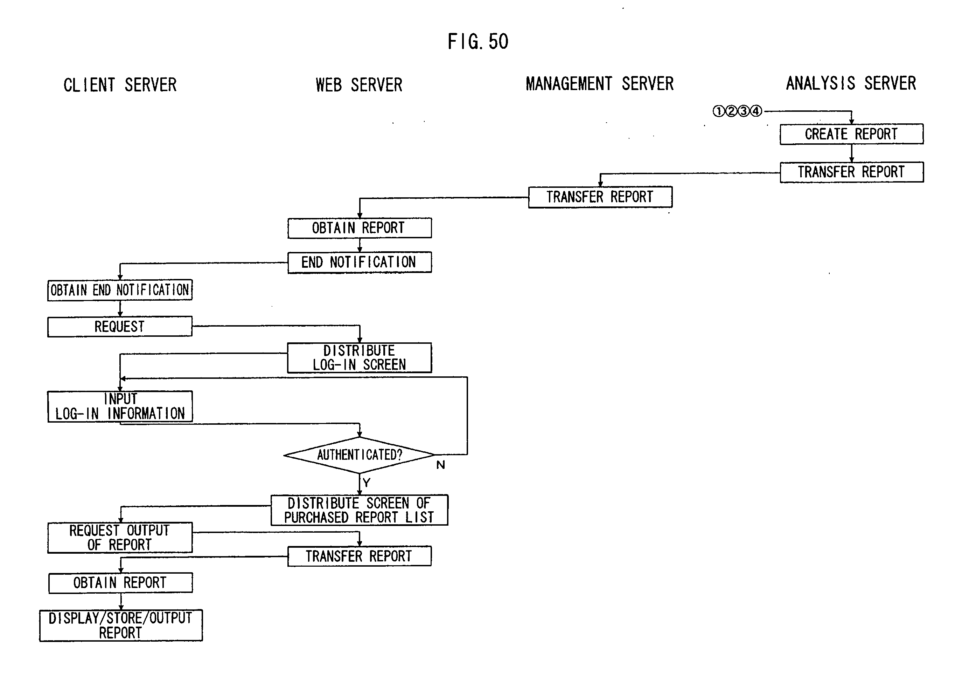

As shown in Fig. 50, the analysis server creates a report based on the result of research case index term totaling processing, the research case similar population, the number of documents, the index term document frequency scatter diagram or the like, the result of various kinds of totaling processing, the result of creating a structure diagram, and the result of creating clustering information. After creating the report, the analysis server transfers the report to the management server and to the web server as well.

Upon receiving the report, the web server creates an end notification indicating the end of the processing and transmits it to the client.

The web server responds to a request from the client to distribute a log-in screen to the client. In response to log-in from the client, the web server carries out authentication, and if the authentication is not successful, the log-in screen by the client is regained. On the other hand, if the authentication is successful, the web server distributes a purchased report list screen to the client.

In response to a report output request from the client, the web server transfers the report to the client. The client thus obtains the report, and then can display it on the display, store it in the recording device or output as a print from a printer or the like.

INDUSTRIAL APPLICABILITY

The invention is applicable to provide a device for automatically creating information analysis report that analyzes a document to be surveyed or document group and displays the characteristics, an automatic analysis report creating program, and a method of automatically creating an information analysis report.

**Claims**

1. A device for automatically creating information analysis report that creates a report representing characteristics of a document to be surveyed relative to a document to be comparaed in information analysis of the document to be surbeyed, said device comprising at least:

   input means for accepting input of said document to be surveyed;
   selecting means for selecting a population document group from the information of a document group to be compared stored in a database based on said input document to be surveyed, said population document group being a set of population documents similar to the document to be surveyed;
   extracting means for extracting an index term characteristic in relation to said population documents of said document to be surveyed;
   creating means for creating an information analysis report representing characteristics of said document to be surveyed based on said population documents and said index terms; and
   output means for outputting said information analysis report to display means, recording means, or communicating means.

2. The device for automatically creating information analysis report according to claim 1, further comprising calculating means for calculating a similarity relative to said document to be compared, wherein said selecting means selects population documents based on the result by said calculating means.

3. The device for automatically creating information analysis report according to claim 2, wherein said calculating means calculates a similarity based on a function value of an occurrence frequency per index term in each document and a document frequency.

4. The device for automatically creating information analysis report according to claim 2, further comprising at least one of:

   map creating means for having said population or said index terms distributed in a map state;
   output data obtaining means for obtaining part of the data of said population or said index terms;
   fixed comment obtaining means for obtaining a fixed comment corresponding to the content of said map and data; and
   comment entering means for entering a free comment,
   said creating means creating an information analysis report representing characteristics of said document to be surveyed by combining said map, said data and/or said comment.

5. The device for automatically creating information analysis report according to any one of claims 1 to 4, wherein said creating means carries out totaling for each of said index terms or prescribed items in said population documents, said totaling including keyword totaling, time-series totaling representing the time-series transition of keywords or prescribed items in the population documents, and/or matrix totaling for a plurality of prescribed items in the population documents and creates an information analysis report including the results of totaling.

**6.** The device for automatically creating information analysis report according to claim 5, wherein said creating means creates a portfolio represented by the totaling result of prescribed items in the keywords or the population documents and a matrix of the time-series increase ratio of the totaling result in said time-series totaling, and creates an information analysis report including the portfolio.

**7.** The device for creating information analysis report according to any one of claims 1 to 6, wherein said creating means, comprises:

first occurrence value frequency calculating means for calculating a function value of the occurrence frequency of said extracted index term in said document to be compared group;
second occurrence value frequency calculating means for calculating a function value of the occurrence frequency of said extracted index term in said population document group; and
frequency scatter diagram creating means for creating a frequency scatter diagram including each index term and their positioning data based on a combination of the function value of the occurrence frequency in said calculate document to be compared and the function value of the occurrence frequency in said population document group for each index term.

**8.** The device for creating information analysis report according to any one of claims 1 to 7, wherein said creating means comprises:

extracting means for extracting the content data and time data of said population documents or said document to be surveyed and said population documents;
tree-like diagram creating means for creating a tree-like diagram representing the co-relation between said plurality of documents based on the content data of each said document;
clustering means for cutting said tree-like diagram according to a prescribed rule and extracting a cluster; and inside cluster arranging means for determining the arrangement of the document group belonging to each said cluster in the cluster based on the time data of each said document.

**9.** The device for creating information analysis report according to claim 8, wherein said clustering means cuts said tree-like diagram to extract a parent cluster, creates a partial tree-like diagram representing the co-relation of the document group belonging to said parent cluster based on the content data of each document belonging to said parent cluster, and cuts the created partial tree-like diagram according to a prescribed rule to extract a descendant cluster.

**10.** The device for creating information analysis report according to claim 9, wherein said clustering means removes from each document vector a vector component whose deviation among a plurality of documents belonging to said parent cluster is smaller than a value determined by a prescribed method in order to create said partial tree-like diagram.

**11.** The device for creating information analysis report according to any one of claims 8, wherein said creating means comprises:

evaluation value calculating means for calculating an evaluation value in each said cluster for each index term;
concentration degree calculating means for calculating the sum of the evaluation values in said each cluster for each index term in all the clusters, calculating the ratio of the evaluation values in each cluster relative to the sum, calculating a square of each said ratio, and calculating the degree of concentration in the distribution of each index term in said cluster obtained by calculating the sum of the square of the ratio in all the clusters;
share calculating means for calculating the sum of the evaluation values of the index terms in said clusters to be analyzed for all the index terms extracted from each said cluster, and calculating the share of each index term in said cluster to be analyzed obtained by calculating the ratio of each index term relative to the sum for each index term;
first inverse calculating means for calculating a function value of the inverse of the occurrence frequency of each index term in said cluster;
second inverse calculating means for calculating a function value of the inverse of the occurrence frequency of each index term in all the documents including said cluster;
creativity degree calculating means for calculating a creativity degree by a function value of the result of subtracting the result of calculation by said second inverse calculating means from the result of calculating by said first inverse calculating means; and

keyword extracting means for extracting keywords based on a combination of a degree of concentration calculated by said concentration degree calculating means, a share calculated for each of said document group for analysis by said share calculating means, and a creativity degree calculated by said creativity degree calculating means.

**12.** The device for creating information analysis report according to any one of claims 1 to 12, further comprising a web server connected to a network and accepting input of a document to be surveyed from a client connected through the network;

a management server that queues said document to be surveyed and requests the analysis server to process a document to be surveyed to be processed next; and

the analysis server that responds to said request to select a population document group that is a set of population documents similar to the document to be surveyed from information of a document to be compared group stored in a database based on said input document to be surveyed, extract characteristic index terms of said document to be surveyed relative to the population document group, and creates an information analysis report representing characteristics of said document to be surveyed.

**13.** A program for automatically creating information analysis report that creates a report representing characteristics of a document to be surveyed relative to a document to be compared in information analysis of the document to be surveyed, said program enabling a computer to function at least as:

input means for accepting input of said document to be surveyed;

selecting means for selecting a population document group from the information of a document group to be compared stored in a database based on said input document to be surveyed, said population documents being a set of population documents similar to the document to be surveyed;

extracting means for extracting an index term characteristic in relation to said population documents of said document to be surveyed;

creating means for creating an information analysis report representing characteristics of said document to be surveyed based on said population documents and said index terms; and

output means for outputting said information analysis report to display means, recording means, or communicating means.

**14.** The program for automatically creating information analysis report according to claim 13, further enabling the computer to function as calculating means for calculating a similarity relative to said document to be compared, wherein said selecting means selects population documents based on the result by said calculating means.

**15.** The device for automatically creating information analysis report according to claim 14, wherein said calculating means calculates a similarity based on a function value of an occurrence frequency per index term in each document and a document frequency.

**16.** The program for automatically creating information analysis report according to claim 14, further enabling the computer to function as at least one of:

map creating means for having said population or said index terms distributed in a map state;

output data obtaining means for obtaining part of the data of said population or said index terms;

fixed comment obtaining means for obtaining a fixed comment corresponding to the content of said map and data; and

comment entering means for entering a free comment,

said creating means creating an information analysis report representing characteristics of said document to be surveyed by combining said map, said data and/or said comment.

**17.** The program for automatically creating information analysis report according to any one of claims 13 to 16, wherein said creating means carries out totaling for each of said index terms or prescribed items in said population documents, said totaling including keyword totaling, time-series totaling representing the time-series transition of keywords or prescribed items in the population documents, and/or matrix totaling of a plurality of prescribed items in the population documents, and creates an information analysis report including the results of totaling.

**18.** The program for automatically creating information analysis report according to claim 17, wherein said creating means creates a portfolio represented by the totaling result of prescribed items in the keywords or the population documents and a matrix of the time-series increase ratio of the totaling result in said time-series totaling, and creates an information analysis report including the portfolio.

19. The program for creating information analysis report according to any one of claims 13 to 18, wherein said creating means, comprises:

first occurrence value frequency calculating means for calculating a function value of the occurrence frequency of said extracted index term in said document to be compared group;
second occurrence value frequency calculating means for calculating a function value of the occurrence frequency of said extracted index term in said population document group; and
frequency scatter diagram creating means for creating a frequency scatter diagram including each index term and their positioning data based on a combination of the function value of the occurrence frequency in said calculate document to be compared and the function value of the occurrence frequency in said population document group for each index term.

20. The program for creating information analysis report according to any one of claims 13 to 19, wherein said creating means comprises:

extracting means for extracting the content data and time data of said population documents or said document to be surveyed and said population documents;
tree-like diagram creating means for creating a tree-like diagram representing the co-relation between said plurality of documents based on the content data of each said document;
clustering means for cutting said tree-like diagram according to a prescribed rule and extracting a cluster; and
inside cluster arranging means for determining the arrangement of the document group belonging to each said cluster in the cluster based on the time data of each said document.

21. The program for creating information analysis report according to claim 20, wherein said clustering means cuts said tree-like diagram to extract a parent cluster, creates a partial tree-like diagram representing the co-relation of the document group belonging to said parent cluster based on the content data of each document belonging to said parent cluster, and cuts the created partial tree-like diagram according to a prescribed rule to extract a descendant cluster.

22. The program for creating information analysis report according to claim 21, wherein said clustering means removes from each document vector a vector component whose deviation among a plurality of documents belonging to said parent cluster is smaller than a value determined by a prescribed method in order to create said partial tree-like diagram.

23. The program for creating information analysis report according to 20, wherein said creating means further comprises:

evaluation value calculating means for calculating an evaluation value in each said cluster for each index term;
concentration degree calculating means for calculating the sum of the evaluation values in said each cluster for each index term in all the clusters, calculating the ratio of the evaluation values in each cluster relative to the sum, calculating a square of each said ratio, and calculating the degree of concentration in the distribution of each index term in said cluster obtained by calculating the sum of the square of the ratio in all the clusters;
share calculating means for calculating the sum of the evaluation value of each index term in said cluster for analysis for all the index terms extracted from each said cluster, and calculating the share of each index term in said cluster for analysis obtained by calculating the ratio of the evaluation value of each index term relative to the sum;
first inverse calculating means for calculating a function value of the inverse of the occurrence frequency in said cluster for each index term;
second inverse calculating means for calculating a function value of the inverse of the occurrence frequency in all the documents including said cluster for each index term;
creativity degree calculating means for calculating a creativity degree by a function value of the result of subtracting the result of calculation by said second inverse calculating means from the result of calculating by said first inverse calculating means; and
keyword extracting means for extracting keywords based on a combination of a degree of concentration calculated by said concentration degree calculating means, a share calculated for each of said document group for analysis by said share calculating means, and a creativity degree calculated by said creativity degree calculating means.

**24.** A method for automatically creating information analysis report method that creates a report representing characteristics of a document to be surveyed relative to a document to be compared in information analysis of the document to be surveyed, said method comprising the steps of:

inputting by accepting input of at least said document to be surveyed;

selecting a population document group from the information of a document group to be compared stored in a database based on said input document to be surveyed, said population document group being a set of population documents similar to the document to be surveyed;

extracting an index term characteristic in relation to said population documents of said document to be surveyed;

creating an information analysis report representing characteristics of said document to be surveyed based on said population documents and said index terms; and

outputting said information analysis report to display means, recording means, or communicating means.

**25.** The method for automatically creating information analysis report according to claim 24, further comprising the step of calculating a similarity relative to said document to be compared, wherein in said selecting step, population documents are selected based on the result by said calculating step.

**26.** The method for automatically creating information analysis report according to claim 25, wherein in said calculating step, a similarity is calculated based on a function value of an occurrence frequency per index term in each document and a document frequency.

**27.** The method for automatically creating information analysis report according to claim 25, further comprising at least one of:

a map creating step of having said population or said index terms distributed in a map state;

an output data obtaining step of obtaining part of the data of said population or said index terms;

a fixed comment obtaining step of obtaining a fixed comment corresponding to the content of said map and data; and

a comment entering step of entering a free comment, wherein in said creating step, information analysis report representing characteristics of said document to.be surveyed is created by combining said map, said data and/or said comment.

**28.** The method for automatically creating information analysis report according to any one of claims 24 to 27, wherein said creating step includes the step of totaling for each of said index terms or prescribed items in said population documents including keyword totaling, time-series totaling representing the time-series transition of keywords or prescribed items in the population documents, and/or matrix totaling of a plurality of prescribed items in the population documents, and creating an information analysis report including the results of totaling.

**29.** The method for automatically creating information analysis report according to claim 28, wherein said creating step includes the step of creating a portfolio represented by the totaling result of prescribed items in the keywords or the population documents and a matrix of the time-series increase ratio of the totaling result in said time-series totaling, and creating an information analysis report including the portfolio.

**30.** The method for creating information analysis report according to any one of claims 24 to 29, wherein said creating step comprises:

a first occurrence value frequency calculating step of calculating a function value of the occurrence frequency of said extracted index terms in said document to be compared group;

a second occurrence value frequency calculating step of calculating a function value of the occurrence frequency of said extracted index terms in said population document group; and

a frequency scatter diagram creating step of creating a frequency scatter diagram including each index term and their positioning data based on a combination of the function value of the occurrence frequency in said calculate document to be compared and the function value of the occurrence frequency in said population document group for each index term.

**31.** The method for creating information analysis report according to any one of claims 24 to 30, wherein said creating step comprises the steps of:

> extracting the content data and time data of said population documents or said document to be surveyed and said population documents;
> creating a tree-like diagram representing the co-relation between said plurality of documents based on the content data of each said document;
> clustering by cutting said tree-like diagram according to a prescribed rule and extracting a cluster; and
> inside cluster arranging by determining the arrangement of the document group belonging to each said cluster in the cluster based on the time data of each said document.

32. The method for creating information analysis report according to claim 31, wherein in said clustering step, said tree-like diagram is cut to extract a parent cluster, a partial tree-like diagram representing the co-relation of the document group belonging to said parent cluster is created based on the content data of each document belonging to said parent cluster, and the created partial tree-like diagram is cut according to a prescribed rule to extract a descendant cluster.

33. The method for creating information analysis report according to claim 32, wherein in said clustering step, a vector component whose deviation among a plurality of documents belonging to said parent cluster is smaller than a value determined by a prescribed method is removed from each document vector in order to create said partial tree-like diagram.

34. The method for creating information analysis report according of claims 32, wherein said creating step comprises:

> an evaluation value calculate step of calculating an evaluation value in each said cluster for each index term;
> a concentration degree calculating step of calculating the sum of the evaluation value in said each cluster for each index term in all the clusters, calculating the ratio of the evaluation values in each cluster relative to the sum, calculating a square of each said ratio, and calculating the degree of concentration in the distribution of each index term in said cluster obtained by calculating the sum of the square of the ratio in all the clusters;
> a share calculating step of calculating the sum of the evaluation value of each index term in said clusters to be analyzed for all the index terms extracted from each said cluster, and calculating the share of each index term in said cluster to be analyzed obtained by calculating the ratio of each index term relative to the sum for each index term;
> a first inverse calculating step of for calculating a function value of the inverse of the occurrence frequency in said cluster for each index term;
> a second inverse calculating step of calculating a function value of the inverse of the occurrence frequency in all the documents including said cluster for each index term;
> a creativity degree calculating step of calculating a creativity degree by a function value of the result of subtracting the result of calculation by said second inverse calculating means from the result of calculating by said first inverse calculating means; and
> a keyword extracting step of extracting keywords based on a combination of a degree of concentration calculated by said concentration degree calculating step, a share calculated for each of said document group for analysis by said share calculating step, and a creativity degree calculated by said creativity degree calculating step.

35. A document information analyzing system including at least one web server and at least one client computer, said web server comprising:

> input.screen distributing means responsive to a request from said client computer for distributing an input screen including an input box for document to be surveyed information and a request content selecting box;
> receiving means for receiving information input in said input screen by said client computer;
> case identifying means for identifying a case from the document to be surveyed information and the content selecting information received by said receiving means;
> document data obtaining means for obtaining document data including bibliographic information and content information;
> index term extracting means for extracting index terms for each document included in said document data;
> multi-thread processing means for creating at least one of the following threads simultaneously or in parallel and processing each thread, said threads being a document index term totaling processing thread in which the inside document use frequency of each of index terms extracted from said case identified document is totaled, a similar document population creating thread in which similarities between said case identified document and said documents included in said document data are calculated based on the result of index term totaling processing of said case identified document and the index terms extracted for each of said documents included in said document data and a similar document population is created from a document group having a prescribed number of top values among said calculated similarities, a document attribute totaling processing thread in which at least one result of totaling the ranking of said similarities included in said similar document population, the number of documents, a document number transition, or ranking for each document attribute included in said bibliographic information and an index term document frequency scatter diagram is obtained, a structure diagram creating processing thread in which a tree-like diagram is created based on said similarities of the documents included in said similar document population, clustering is carried out, and a structure diagram is created, and a cluster information creating processing thread in which the cluster information of said case identified document is created based on said created structure diagram data;

document information analysis report creating means for creating a document information analysis report in a prescribed format based on the result of processing of each thread processed by said multi-thread processing means;

end notifying means for notifying said client computer of the end of creating of said document information analysis report; and

document information analysis report transmitting means for transmitting the document information analysis report in response to a request to transmit said document information analysis report from said client computer based on the end notification,

said client computer being adapted to receive said document information analysis report requested for transmission in response to the reception of said end notification from said web server.

**36.** A method for analyzing document information in a document information analyzing system including at least one web server and at least one client computer, said method comprising:

in said web server,

an input screen distributing step responsive to a request from said client computer for distributing an input screen including an input box for document to be surveyed information and a request content selecting box;

a receiving step of receiving information input in said input screen by said client computer;

a case identifying step of identifying a case from the document to be surveyed information and the content selecting information received in said receiving step;

a document data obtaining step of obtaining document data including bibliographic information and content information;

an index term extracting step of extracting index terms for each document included in said document data;

a multi-thread processing step of creating at least one of the following threads simultaneously or in parallel and processing each of the threads, said threads being a document index term totaling processing thread in which the inside document use frequency of each of index terms extracted from said case identified document is totaled, a similar document population creating thread in which similarities between said case identified document and said documents included in said document data are calculated based on the result of index term totaling processing of said case identified document and the index terms extracted for each of said documents included in said document data and a similar document population is created from a document group having a prescribed number of top values among said calculated similarities, a document attribute totaling processing thread in which at least one result of totaling from the ranking of said similarities included in said similar document population, the number of documents, a document number transition or ranking for each document attribute included in said bibliographic information, and an index term document frequency scatter diagram is obtained, a structure diagram creating processing thread in which a tree-like diagram is created based on said similarities of the documents included in said similar document population, clustering is carried out, and a structure diagram is created, and a cluster information creating processing thread in which the cluster information of said case identified document is created based on said created structure diagram data;

a document information analysis report creating step of creating a document information analysis report in a prescribed format based on the result of processing of each thread processed by said multi-thread processing means;

an end notifying step of notifying said client computer of the end of creating of said document information analysis report; and

a document information analysis report transmitting step of transmitting the document information analysis.report in response to a request to transmit said document information analysis report from said client computer based on the end notification,

said client computer being adapted to receive said document information analysis report requested for transmission in response to the reception of said end notification from said web server.

**37.** A computer program used to analyze document information in a document information analyzing system including at least one web server and at least one client computer, said program enabling said web server to carry out:

an input screen distributing function responsive to a request from said client computer for distributing an input screen including an input box for document to be surveyed information and a request content selecting box;
a receiving function of receiving information input in said input screen by said client computer;
a case identifying function of identifying a case from the document to be surveyed information and the content selecting information received by said receiving means;
a document data obtaining function of obtaining document data including bibliographic information and content information;
an index term extracting function of extracting index terms for each document included in said document data;
a multi-thread processing function of creating at least one of the following threads simultaneously or in parallel and processing each of the threads, said threads being a document index term totaling processing thread in which the inside document use frequency of each of index terms extracted from said case identified document is totaled, a similar document population creating thread in which similarities between said case identified document and said documents included in said document data are calculated based on the result of index term totaling processing of said case identified document and the index terms extracted for each of said documents included in said document data and a similar document population is created from a document group having a prescribed number of top values among said calculated similarities, a document attribute totaling processing thread in which at least one result of totaling the ranking of said similarities included in said similar document population, the number of documents, a document number transition or ranking for each document attribute included in said bibliographic information, and an index term document frequency scatter diagram is obtained, a structure diagram characteristic in relation to said population documents of said document to be surveyedstructure diagram data;
a document information analysis report creating function of creating a document information analysis report in a prescribed format based on the result of processing of each thread processed by said multi-thread processing function;
an end notifying function of notifying said client computer of the end of creating of said document information analysis report; and
a document information analysis report transmitting function of transmitting the document information analysis report in response to a request to transmit said document information analysis report from said client computer based on the end notification,
said client computer being adapted to receive said document information analysis report requested for transmission in response to the reception of said end notification from said web server.

**38.** A web server for analyzing document information in response to a request from at least one client computer, at least one said web server being provided,
said web server comprising:

input screen distributing means responsive to a request from said client computer for distributing an input screen including an input box for document to be surveyed information and a request content selecting box;
receiving means for receiving information input in said input screen by said client computer;
case identifying means for identifying a case from the document to be surveyed information and the content selecting information received by said receiving means;
document data obtaining means for obtaining document data including bibliographic information and content information;
index term extracting means for extracting index terms for each document included in said document data;
multi-thread processing means for creating at least one of the following threads simultaneously or in parallel and processing each of the threads, said threads being a document index term totaling processing thread in which the inside document use frequency of each of index terms extracted from said case identified document is totaled, a similar document population creating thread in which similarities between said case identified document and said documents included in said document data are calculated based on the result of index term totaling processing of said case identified document and the index terms extracted for each of said documents included in said document data and a similar document population is created from a document group having a prescribed number of top values among said calculated similarities, a document attribute totaling processing thread in which at least one result of totaling the ranking of said similarities included in said similar document population, the number of documents, a document number transition or ranking for each document attribute included in said bibliographic information, and an index term document frequency scatter diagram is obtained,

**EP 1 881 423 A1**

a structure diagram creating processing thread in which a tree-like diagram is created based on said similarities of the documents included in said similar document population, clustering is carried out, and a structure diagram is created, and a cluster information creating processing thread in which the cluster information of said case identified document is created based.on said created structure diagram data;

document information analysis report creating means for creating a document information analysis report in a prescribed format based on the result of processing of each thread processed by said multi-thread processing means;

end notifying means for notifying said client computer of the end of creating of said document information analysis report; and

document information analysis report transmitting means for transmitting the document information analysis report in response to a request to transmit said document information analysis report from said client computer based on the end notification.

59

FIG. 1

<u>1 0 0</u>

INPUT DEVICE ~ 2

PROCESSING DEVICE ~ 1

3

RECORDING DEVICE

OUTPUT DEVICE ~ 4

## FIG. 2

FIG. 3

```
                    ┌─────────┐
                    │  START  │
                    └────┬────┘
                         │
                         ▼
              ┌─────────────────────┐
              │   INITIALIZATION    │────  S 2 0 1
              └──────────┬──────────┘
                         │
   S 2 0 2               │
         ◇───────────────┴───────────────◇
  ┌─────◇        CONDITION                ◇──────────┐
  │      ◇       SEPARATION              ◇           │
  │       ◇───────────┬───────────────◇             │
  │                    │                             │
  ▼                    ▼                             ▼
S 2 1 0            S 2 3 0                    S 2 2 0
┌──────────────┐  ┌──────────────┐         ┌──────────────┐
│ DOCUMENT TO BE│  │  EXTRACTION  │         │ DOCUMENT TO BE│
│ SURVEYED d   │  │ CONDITION AND│         │  COMPARED P  │
│CONDITION INPUT│  │ OTHERS INPUT │         │CONDITION INPUT│
└──────┬───────┘  └──────┬───────┘         └──────┬───────┘
       │                 │                        │
S 2 1 1│          S 2 3 1│                 S 2 2 1│
    ◇──┴──◇  NO       ◇──┴──◇  NO             ◇──┴──◇  NO
   ◇CONDITION◇───┐   ◇CONDITION◇───┐        ◇CONDITION◇───┐
   ◇CONFIRMATION◇│   ◇CONFIRMATION◇│        ◇CONFIRMATION◇│
    ◇  OK?  ◇    │    ◇  OK?  ◇    │         ◇  OK?  ◇    │
     ◇───◇       │     ◇───◇       │          ◇───◇       │
     YES         │     YES         │          YES         │
      │          │      │          │           │          │
      └──────────┘      │          │           └──────────┘
                        │          │
      ┌─────────────────┴──────────────────────┐
      │                 │                       │
S 3 1 0                  ▼
      └────────► ┌──────────────┐
                 │  CONDITION   │
                 │  RECORDING   │
                 └──────┬───────┘
                        │
                        ▼
                  ┌─────────┐
                  │   END   │
                  └─────────┘
```

## FIG. 4

START

INITIALIZATION — S 1 0 1

S 1 0 2
READING
SEPARATION

S 1 1 0 — DOCUMENT TO BE
SURVEYED d READ OUT

DOCUMENT TO BE
COMPARED P READ OUT — S 1 3 0

S 1 2 0 — EXTRACT INDEX TERM (d)
IN DOCUMENT TO BE
SURVEYED d

EXTRACT INDEX TERM (P)
IN DOCUMENT TO BE
COMPARED P — S 1 4 0

S 1 4 1                              S 1 4 2

S 1 2 1 — TF (d) CALCULATION

TF (P) CALCULATION

IDF (P) CALCULATION

S 1 5 0 — SIMILARITY
CALCULATION

S 1 5 1 — POPULATION
NARROWING

S 1 6 0 — POPULATION
DOCUMENT S SELECTION

S 1 7 0 — EXTRACT INDEX TERMS (S)
IN POPULATION DOCUMENTS S

S 1 7 1 — IDF (S) CALCULATION

CHARACTERISTIC INDEX TERM ,
SIMILARITY IN POPULATION ,
FREQUENCY SCATTER DIAGRAM ,
STRUCTURE DIAGRAM CALCULATION

S 1 8 0

END

## FIG. 5

FIG. 6

DEVICE FOR CREATING INFORMATION ANALYSIS REPORT
INPUT CONDITION SETTING (1)

SELECT CONDITIONS FROM THE FOLLOWING WINDOWS

| SET |
|---|
| RETURN |

SUBJECT DOCUMENT

| DOCUMENT TO BE SURVEYED | ■ |
|---|---|
| DOCUMENT TO BE COMPARED | □ |

CONTENT OF DOCUMENT

| PATENT PUBLICATION | ■ |
|---|---|
| REGISTERED PATENT | □ |
| UTILITY MODEL | □ |
| SCIENTIFIC LITERATURE | □ |

DATA READ OUT

| COMPANY'S OWN DB1 | □ |
|---|---|
| COMPANY'S OWN DB2 | □ |
| JPO IPDL | □ |
| PATOLIS | □ |
| OTHER COMMERCIAL DB1 | □ |
| OTHER COMMERCIAL DB2 | □ |

| FD | DOCUMENT 1 | □ |
|---|---|---|
| CD | DOCUMENT 2 | □ |
| MO | DOCUMENT 3 | ■ |
| DVD | DOCUMENT 4 | □ |
| OTHERS | DOCUMENT 5 | □ |
| | DOCUMENT 6 | □ |

FIG. 7

DEVICE FOR CREATING INFORMATION ANALYSIS REPORT
INPUT CONDITION SETTING (2)

SELECT CONDITIONS FROM THE FOLLOWING WINDOWS

| SET |
|---|
| RETURN |

SUBJECT DOCUMENT

| DOCUMENT TO BE SURVEYED | □ |
|---|---|
| DOCUMENT TO BE COMPARED | ■ |

CONTENT OF DOCUMENT

| PATENT PUBLICATION | ■ |
|---|---|
| REGISTERED PATENT | ■ |
| UTILITY MODEL | □ |
| SCIENTIFIC LITERATURE | □ |

CONTENT FOR EXTRACTION

| CLAIMS | ■ |
|---|---|
| BACKGROUND ART | □ |
| OBJECT OF INVENTION | □ |
| MEANS/ADVANTAGES | □ |
| EMBODIMENT | □ |
| DESCRIPTION OF DRAWINGS | □ |
| DRAWINGS | □ |
| ABSTRACT | ■ |
| BIBLIOGRAPHICAL ITEMS | □ |
| PROCEDURE INFORMATION | □ |
| REGISTRATION INFORMATION | □ |
| OTHERS | □ |

DATA READ OUT

| COMPANY'S OWN DB1 | ■ |
|---|---|
| COMPANY'S OWN DB2 | □ |
| JPO IPDL | □ |
| PATOLIS | □ |
| OTHER COMMERCIAL DB1 | □ |
| OTHER COMMERCIAL DB2 | □ |
| FD | □ |
| CD | □ |
| MO | □ |
| DVD | □ |
| OTHERS | □ |

## FIG. 8

**DEVICE FOR CREATING INFORMATION ANALYSIS REPORT**
**INPUT CONDITION SETTING (3)**

SELECT CONDITIONS FROM THE FOLLOWING WINDOWS

| SET |
|---|
| RETURN |

EXTRACTION CONDITION OF INDEX TERM

| | |
|---|---|
| COMPANY'S OWN KEYWORD SEGMENTING 1 | ■ |
| COMPANY'S OWN KEYWORD SEGMENTING 2 | ☐ |
| COMMERCIAL KEYWORD SEGMENTING 1 | ☐ |
| COMMERCIAL KEYWORD SEGMENTING 2 | ☐ |

SIMILARITY CALCULATING METHOD

| | |
|---|---|
| SIMILARITY 1 | ■ |
| SIMILARITY 2 | ☐ |
| SIMILARITY 3 | ☐ |
| SIMILARITY 4 | ☐ |
| SIMILARITY 5 | ☐ |
| SIMILARITY 6 | ☐ |

SELECT POPULATION DOCUMENTS

| | | |
|---|---|---|
| NUMBER OF POPULATION DOCUMENTS | TOP 100 | ☐ |
| NUMBER OF NON-POPULATION DOCUMENTS | TOP 1000 | ☐ |
| | TOP 3000 | ■ |
| | TOP 5000 | ☐ |
| | INPUT NUMERICAL VALUE | ☐ |

POPULATION NARROWING UNIT

| | | |
|---|---|---|
| APPLICANTS WITH LARGE NUMBER OF APPLICATIONS | | ☐ |
| APPLICANTS WITH SMALL NUMBER OF APPLICATIONS | | ☐ |
| SPECIFY IPC | (INPUT) | ■ |
| SPECIFY COMPANY NAME, INDUSTRY | | ☐ |

## FIG. 9

**DEVICE FOR CREATING INFORMATION ANALYSIS REPORT**
**OUTPUT CONDITION SETTING**

SELECT CONDITIONS FROM THE FOLLOWING WINDOWS

| SET |
|---|
| RETURN |

MAP CALCULATION METHOD

| | |
|---|---|
| x-AXIS: NUMBER OF INDEX TERMS | ■ |
| y-AXIS: ORDER OF INDEX TERMS | ☐ |

MAP POSITION

| | |
|---|---|
| ONE MAP | ■ |
| TWO MAPS | ☐ |
| ONE MAP WITH DATA | ☐ |
| TWO MAPS WITH DATA | ☐ |
| ONE MAP WITH COMMENT | ☐ |
| TWO MAPS WITH COMMENT | ☐ |
| ONE MAP WITH DATA AND COMMENT | ☐ |
| TWO MAPS WITH DATA AND COMMENT | ☐ |

OUTPUT DATA

| | | |
|---|---|---|
| TFIDF DESCENDING ORDER | NONE | ☐ |
| TFIDF ASCENDING ORDER | TOP 5 | ☐ |
| | TOP 10 | ☐ |
| | TOP 15 | ☐ |
| | TOP 25 | ■ |
| | INPUT NUMERICAL VALUE | ☐ |

COMMENT

| |
|---|
| (FREE COMMENT) |

## FIG. 10

### FIXED COMMENT

> ·The population was extracted from patent related publications (published unexamined patent application, published unexamined patent application based on international application, published examined patent application, patent publication, published unexamined utility model application, published registered utility model, published unexamined utility model application based on international application, re-published unexamined utility model application based on international application, published examined utility model application, published examined utility model registration) made into electronic data from January 1993 to November 2003 included in JP-ROM yearly index CD-ROM.
> ·Based on the publications, a keyword importance degree (for all the publications) was calculated for each of keywords extracted from a researched case. Top 20 terms and their keyword importance degrees are as follows:

MAP                     ▨▨▨ KEYWORD IMPORTANCE DEGREE (FOR ALL PUBLICATIONS)

| | | Value | 0 | 20 | 40 | 60 | 80 |
|---|---|---|---|---|---|---|---|
| 1 | SAMPLE | 75.8 | | | | | |
| 2 | ANALYSIS | 23.5 | | | | | |
| 3 | MASS | 19.8 | | | | | |
| 4 | SOLID | 17.2 | | | | | |
| 5 | LASER | 16.7 | | | | | |
| 6 | HOLDER | 15.8 | | | | | |
| 7 | SPECTROMETER | 15.2 | | | | | |
| 8 | ION | 15.1 | | | | | |
| 9 | GLYCERIN | 13.0 | | | | | |
| 10 | IONIZATION | 12.8 | | | | | |
| 11 | SOLUTION | 12.2 | | | | | |
| 12 | VISCOSITY | 11.7 | | | | | |
| 13 | APPLICATION | 10.9 | | | | | |
| 14 | CREATING | 10.8 | | | | | |
| 15 | FINE PARTICLE | 10.0 | | | | | |
| 16 | VAPOR | 9.7 | | | | | |
| 17 | SOLVENT | 8.6 | | | | | |
| 18 | MIXING | 8.4 | | | | | |
| 19 | PULSE | 7.8 | | | | | |
| 20 | LIQUID | 7.6 | | | | | |

KEYWORD IMPORTANCE DEGREE (FOR ALL PUBLICATIONS) RANKING

> ·for each publication in the publication group, all publication similarity for the research case was calculated and the publications with top 3000 ratios were selected as the population representing the entire technical development field, a summary of which is as follows.

| | |
|---|---|
| RANGE OF SIMILARITIES OF EXTRACTED PUBLICATIONS | 80.2~46.8 |
| NUMBER OF APPLICANTS (NAME SORTED NUMBER) | 553 |
| NUMBER OF INVENTORS (NAME SORTED NUMBER) | 3,771 |
| INTERNATIONAL PATENT CLASSIFICATION (IPC) MAIN GROUP NUMBER (CLASS) | 139 |
| INTERNATIONAL PATENT CLASSIFICATION (IPC) MAIN GROUP NUMBER (CLASS/SUB CLASS) | 294 |
| NUMBER OF KEYWORD (NAME SORTED NUMBER) | 6,478 |

FIXED COMMENT

DATA

FIXED COMMENT

> ※This patent information analysis was carried out by research and analysis based on the publication group made into electronic data from January 1993 to November 2003 included in JP-ROM yearly index CD-ROM and the publications before the period (or after the period) that are not in electronic forms are not taken into consideration. It should be noted that if there are cases before the period (or after the period) that are not in electronic forms similar to the researched case, the cases are not touched upon in this patent information analysis.

> X X X · · ·

FREE COMMENT

## FIG. 11

APPLICANT RANKING (ENTIRE PERIOD)

# FIG. 12

APPLICANT RANKING (RECENT 3 YEARS)

# FIG. 13

INTERNATIONAL PATENT CLASSIFICATION (IPC) RANKING (CLASS)

EP 1 881 423 A1

# FIG. 14

INTERNATIONAL PATENT CLASSIFICATION (IPC) RANKING (CLASS/SUB CLASS)

Legend: ■ PUBLISHED NUMBER   ▨ REGISTERED NUMBER   □ UTILITY MODEL NUMBER

| RANKING | INTERNATIONAL PATENT CLASSIFICATION | Number |
|---|---|---|
| 1 | G01N 1 | 818 |
| 2 | G01N 23 | 585 |
| 3 | H01J 37 | 573 |
| 4 | H01L 21 | 400 |
| 5 | G01N 21 | 379 |
| 6 | G01N 27 | 247 |
| 7 | G01N 33 | 243 |
| 8 | G01N 35 | 217 |
| 9 | G01N 25 | 149 |
| 10 | H01J 49 | 147 |
| 11 | G01N 30 | 140 |
| 12 | G01N 31 | 73 |
| 13 | G01N 3 | 73 |
| 14 | G01B 11 | 69 |
| 15 | G02B 21 | 64 |
| 16 | C23F 4 | 61 |
| 17 | G01N 37 | 55 |
| 18 | G01N 5 | 52 |
| 19 | G01B 21 | 51 |
| 20 | G01N 13 | 50 |

EP 1 881 423 A1

FIG. 15

TOP 10 APPLICANTS (ENTIRE PERIOD) × INTERNATIONAL PATENT CLASSIFICATION (IPC) TOP 5 CLASSES (CLASS/SUB CLASS)

## FIG. 16

TOP 10 APPLICANTS (ENTIRE PERIOD) ×
INTERNATIONAL PATENT CLASSIFICATION (IPC) TOP 5 CLASSES (CLASS/SUB CLASS)

| | | G01N 1 | G01N 23 | H01J 37 | H01L 21 | G01N 21 |
|---|---|---|---|---|---|---|
| COMPANY A | PUBLISHED | 60 | 42 | 128 | 92 | 18 |
| | REGISTERED | 10 | 6 | 34 | 56 | 4 |
| | UTILITY MODEL | 1 | 0 | 0 | 1 | 1 |
| | TOTAL | 71 | 48 | 162 | 149 | 23 |
| COMPANY B | PUBLISHED | 40 | 34 | 19 | 4 | 48 |
| | REGISTERED | 14 | 22 | 18 | 0 | 20 |
| | UTILITY MODEL | 6 | 12 | 2 | 0 | 8 |
| | TOTAL | 60 | 68 | 39 | 4 | 76 |
| COMPANY C | PUBLISHED | 40 | 38 | 113 | 13 | 2 |
| | REGISTERED | 11 | 5 | 30 | 0 | 1 |
| | UTILITY MODEL | 5 | 3 | 13 | 0 | 0 |
| | TOTAL | 56 | 46 | 156 | 13 | 3 |
| COMPANY D | PUBLISHED | 30 | 68 | 0 | 3 | 10 |
| | REGISTERED | 9 | 21 | 0 | 3 | 5 |
| | UTILITY MODEL | 16 | 20 | 0 | 0 | 0 |
| | TOTAL | 55 | 109 | 0 | 6 | 15 |
| COMPANY E | PUBLISHED | 23 | 43 | 0 | 0 | 0 |
| | REGISTERED | 2 | 9 | 0 | 0 | 0 |
| | UTILITY MODEL | 4 | 8 | 0 | 0 | 0 |
| | TOTAL | 29 | 60 | 0 | 0 | 0 |
| COMPANY F | PUBLISHED | 13 | 9 | 48 | 5 | 2 |
| | REGISTERED | 3 | 1 | 9 | 1 | 0 |
| | UTILITY MODEL | 0 | 0 | 0 | 0 | 0 |
| | TOTAL | 16 | 10 | 57 | 6 | 2 |
| COMPANY G | PUBLISHED | 12 | 6 | 16 | 8 | 5 |
| | REGISTERED | 2 | 1 | 7 | 5 | 0 |
| | UTILITY MODEL | 1 | 3 | 0 | 0 | 0 |
| | TOTAL | 15 | 10 | 23 | 13 | 5 |
| COMPANY H | PUBLISHED | 15 | 15 | 17 | 19 | 1 |
| | REGISTERED | 10 | 9 | 5 | 14 | 1 |
| | UTILITY MODEL | 0 | 0 | 1 | 0 | 0 |
| | TOTAL | 25 | 24 | 23 | 33 | 2 |
| COMPANY I | PUBLISHED | 12 | 8 | 4 | 11 | 0 |
| | REGISTERED | 3 | 3 | 2 | 9 | 2 |
| | UTILITY MODEL | 0 | 0 | 0 | 0 | 0 |
| | TOTAL | 15 | 11 | 6 | 20 | 2 |
| COMPANY J | PUBLISHED | 21 | 13 | 1 | 0 | 10 |
| | REGISTERED | 2 | 5 | 0 | 0 | 2 |
| | UTILITY MODEL | 5 | 4 | 0 | 0 | 3 |
| | TOTAL | 28 | 22 | 1 | 0 | 15 |

## FIG. 17

| RANKING | APPLICANT | | Legend |
|---------|-----------|---|--------|
| | | | PUBLISHED NUMBER (black) |
| | | | REGISTERED NUMBER (hatched) |
| | | | UTILITY MODEL NUMBER (white) |

| RANKING | APPLICANT | Value |
|---------|-----------|-------|
| 1 | COMPANY A | 14 |
| 2 | COMPANY B | 2 |
| 3 | COMPANY C | 3 |
| 4 | COMPANY D | 3 |
| 5 | COMPANE E | 3 |
| 6 | COMPANY F | 3 |
| 7 | COMPANY G | 1 |
| 8 | COMPANY H | 5 |
| 9 | COMPANY I | 4 |
| 10 | COMPANE J | 2 |
| 11 | COMPANY K | 6 |
| 12 | COMPANY L | 4 |
| 13 | COMPANY M | 0 |
| 14 | COMPANY N | 5 |
| 15 | COMPANE O | 5 |
| 16 | COMPANY P | 5 |
| 17 | COMPANY Q | 6 |
| 18 | COMPANY R | 1 |
| 19 | COMPANY S | 4 |
| 20 | COMPANE T | 1 |

TOP 20 APPLICANTS (ENTIRE PERIOD) × INTERNATIONAL PATENT CLASSIFICATION (IPC)  G06F 17

## FIG. 18

EP 1 881 423 A1

**PUBLISHED NUMBER** ■■■    **REGISTERED NUMBER** ▨▨▨    **UTILITY MODEL NUMBER** ☐

| RANKING | KEYWORD | | 0 | 500 | 1000 | 1500 | 2000 | 2500 | 3000 |
|---------|---------|------|---|-----|------|------|------|------|------|
| 1 | SAMPLE | 3000 | | | | | | | |
| 2 | ANALYSIS | 1335 | | | | | | | |
| 3 | MASS | 270 | | | | | | | |
| 4 | SOLID | 123 | | | | | | | |
| 5 | LASER | 156 | | | | | | | |
| 6 | HOLDER | 388 | | | | | | | |
| 7 | SPECTROMETER | 136 | | | | | | | |
| 8 | ION | 449 | | | | | | | |
| 9 | GLYCERIN | 1 | | | | | | | |
| 10 | IONIZATION | 130 | | | | | | | |
| 11 | SOLUTION | 171 | | | | | | | |
| 12 | VISCOSITY | 17 | | | | | | | |
| 13 | APPLICATION | 33 | | | | | | | |
| 14 | CREATING | 104 | | | | | | | |
| 15 | FINE PARTICLE | 35 | | | | | | | |
| 16 | VAPOR | 41 | | | | | | | |
| 17 | SOLVENT | 76 | | | | | | | |
| 18 | MIXING | 141 | | | | | | | |
| 19 | PULSE | 84 | | | | | | | |
| 20 | LIQUID | 283 | | | | | | | |

CASE NUMBER DISTRIBUTION FOR EACH IMPORTANT KEYWORD (FOR ALL PUBLICATIONS)

# FIG. 19

EP 1 881 423 A1

Legend: PUBLISHED NUMBER ▮ | REGISTERED NUMBER ▨ | UTILITY MODEL NUMBER □

| RANKING | KEYWORD | Count | 0 | 100 | 200 | 300 | 400 | 500 |
|---|---|---|---|---|---|---|---|---|
| 1 | GLYCERIN | 1 | | | | | | |
| 2 | APPLICATION | 33 | | | | | | |
| 3 | SOLID | 123 | | | | | | |
| 4 | LASER | 156 | | | | | | |
| 5 | VISCOSITY | 17 | | | | | | |
| 6 | CREATING | 104 | | | | | | |
| 7 | MASS | 270 | | | | | | |
| 8 | MIXING | 141 | | | | | | |
| 9 | FINE PARTICLE | 35 | | | | | | |
| 10 | SOLUTION | 171 | | | | | | |
| 11 | VAPOR | 41 | | | | | | |
| 12 | ION | 449 | | | | | | |
| 13 | SOLVENT | 76 | | | | | | |
| 14 | MAKE | 2 | | | | | | |
| 15 | PULSE | 84 | | | | | | |
| 16 | IONIZATION | 130 | | | | | | |
| 17 | SPECTROMETER | 136 | | | | | | |
| 18 | HOLDER | 388 | | | | | | |
| 19 | GUIDE | 164 | | | | | | |
| 20 | HOMOGENEOUS | 16 | | | | | | |

CASE NUMBER DISTRIBUTION FOR EACH IMPORTANT KEYWORD (FOR POPULATION)

FIG. 20

TRANSITION OF CASE NUMBER FOR EACH APPLICANT (ACCUMULATION)

EP 1 881 423 A1

## FIG. 21

TRANSITION OF CASE NUMBER FOR EACH APPLICANT (SINGLE-YEAR TOTAL AND CUMULATIVE TOTAL)

| | | 1992 | 1993 | 1994 | 1995 | 1996 | 1997 | 1998 | 1999 | 2000 | 2001 |
|---|---|---|---|---|---|---|---|---|---|---|---|
| COMPANY A | SINGLE-YEAR TOTAL | 54 | 42 | 36 | 49 | 21 | 31 | 32 | 30 | 24 | 16 |
| | CUMULATIVE TOTAL | 54 | 96 | 132 | 181 | 202 | 233 | 265 | 295 | 319 | 335 |
| COMPANY B | SINGLE-YEAR TOTAL | 29 | 20 | 25 | 34 | 25 | 16 | 17 | 13 | 12 | 11 |
| | CUMULATIVE TOTAL | 29 | 49 | 74 | 108 | 133 | 149 | 166 | 179 | 191 | 202 |
| COMPANY C | SINGLE-YEAR TOTAL | 31 | 21 | 18 | 13 | 16 | 22 | 16 | 21 | 18 | 17 |
| | CUMULATIVE TOTAL | 31 | 52 | 70 | 83 | 99 | 121 | 137 | 158 | 176 | 193 |
| COMPANY D | SINGLE-YEAR TOTAL | 14 | 16 | 7 | 3 | 11 | 23 | 14 | 12 | 13 | 7 |
| | CUMULATIVE TOTAL | 14 | 30 | 37 | 40 | 51 | 74 | 88 | 100 | 113 | 120 |
| COMPANY E | SINGLE-YEAR TOTAL | 11 | 8 | 9 | 16 | 10 | 6 | 3 | 3 | 3 | 7 |
| | CUMULATIVE TOTAL | 11 | 19 | 28 | 44 | 54 | 60 | 63 | 66 | 69 | 76 |
| COMPANY F | SINGLE-YEAR TOTAL | 7 | 10 | 8 | 9 | 3 | 8 | 5 | 10 | 4 | 9 |
| | CUMULATIVE TOTAL | 7 | 17 | 25 | 34 | 37 | 45 | 50 | 60 | 64 | 73 |
| COMPANY G | SINGLE-YEAR TOTAL | 4 | 15 | 2 | 3 | 1 | 5 | 9 | 10 | 5 | 3 |
| | CUMULATIVE TOTAL | 4 | 19 | 21 | 24 | 25 | 30 | 39 | 49 | 54 | 57 |
| COMPANY H | SINGLE-YEAR TOTAL | 4 | 5 | 3 | 8 | 10 | 11 | 14 | 4 | 1 | 0 |
| | CUMULATIVE TOTAL | 4 | 9 | 12 | 20 | 30 | 41 | 55 | 59 | 60 | 60 |
| COMPANY I | SINGLE-YEAR TOTAL | 8 | 4 | 4 | 3 | 8 | 6 | 3 | 5 | 1 | 5 |
| | CUMULATIVE TOTAL | 8 | 12 | 16 | 19 | 27 | 33 | 36 | 41 | 42 | 47 |
| COMPANY J | SINGLE-YEAR TOTAL | 7 | 5 | 7 | 6 | 2 | 2 | 7 | 8 | 4 | 2 |
| | CUMULATIVE TOTAL | 7 | 12 | 19 | 25 | 27 | 29 | 36 | 44 | 48 | 50 |

EP 1 881 423 A1

FIG. 22

TRANSITION OF CASE NUMBER FOR EACH INTERNATIONAL PATENT CLASSIFICATION (IPC)
(TOP 5 CLASSES FOR EACH OF CLASSES AND SUB CLASSES)    (ACCUMULATION)

INTERNATIONAL
PATENT
CLASSIFICATION

—◇—G01N 1
—■—G01N 23
—▲—G01J 37
—✕—G01L 21
—✳—G01N 21

(CASES)

(YEAR)

## FIG. 23

TRANSITION OF CASE NUMBER FOR EACH INTERNATIONAL PATENT CLASSIFICATION (IPC)
(TOP 5 CLASSES FOR EACH OF CLASSES AND SUB CLASSES) (SINGLE-YEAR TOTAL AND CUMULATIVE TOTAL)

| | | 1992 | 1993 | 1994 | 1995 | 1996 | 1997 | 1998 | 1999 | 2000 | 2001 |
|---|---|---|---|---|---|---|---|---|---|---|---|
| G01N 1 | SINGLE-YEAR TOTAL | 68 | 78 | 79 | 63 | 89 | 74 | 68 | 58 | 46 | 55 |
| | CUMULATIVE TOTAL | 68 | 146 | 225 | 288 | 377 | 451 | 519 | 577 | 623 | 678 |
| G01N 23 | SINGLE-YEAR TOTAL | 52 | 55 | 42 | 47 | 44 | 54 | 46 | 50 | 45 | 41 |
| | CUMULATIVE TOTAL | 52 | 107 | 149 | 196 | 240 | 294 | 340 | 390 | 435 | 476 |
| G01J 37 | SINGLE-YEAR TOTAL | 64 | 63 | 49 | 47 | 34 | 53 | 49 | 43 | 37 | 36 |
| | CUMULATIVE TOTAL | 64 | 127 | 176 | 223 | 257 | 310 | 359 | 402 | 439 | 475 |
| G01L 21 | SINGLE-YEAR TOTAL | 34 | 25 | 28 | 35 | 24 | 29 | 30 | 22 | 18 | 23 |
| | CUMULATIVE TOTAL | 34 | 59 | 87 | 122 | 146 | 175 | 205 | 227 | 245 | 268 |
| G01N 21 | SINGLE-YEAR TOTAL | 26 | 41 | 42 | 18 | 34 | 29 | 30 | 23 | 20 | 19 |
| | CUMULATIVE TOTAL | 26 | 67 | 109 | 127 | 161 | 190 | 220 | 243 | 263 | 282 |

EP 1 881 423 A1

FIG. 24

TRANSITION OF CASE NUMBER FOR EACH INTERNATIONAL PATENT CLASSIFICATION (IPC) (G01N 21)

EP 1 881 423 A1

# FIG. 25

INCREASE RATIO (%)

(1) 1993
(2) 1994
(3) 1995
(4) 1996
(5) 1997
(6) 1998
(7) 1999
(8) 2000
(9) 2001

PORTFOLIO OF ENTIRE POPULATION

EP 1 881 423 A1

FIG. 26

INCREASE RATIO (%)

(1) 1993
(2) 1994
(3) 1995
(4) 1996
(5) 1997
(6) 1998
(7) 1999
(8) 2000
(9) 2001

PORTFOLIO OF INTERNATIONAL PATENT CLASSIFICATION (IPC) G01N 1

FIG. 27

TRANSITION OF CASE NUMBER FOR EACH IMPORTANT KEYWORD
(FOR ALL PUBLICATIONS) (ACCUMULATION)

EP 1 881 423 A1

## FIG. 28

### TRANSITION OF CASE NUMBER FOR EACH IMPORTANT KEYWORD
### (FOR ALL PUBLICATIONS) (SINGLE-YEAR TOTAL AND CUMULATIVE TOTAL)

| | | 1992 | 1993 | 1994 | 1995 | 1996 | 1997 | 1998 | 1999 | 2000 | 2001 |
|---|---|---|---|---|---|---|---|---|---|---|---|
| SAMPLE | SINGLE-YEAR TOTAL | 287 | 312 | 241 | 242 | 221 | 229 | 203 | 206 | 158 | 159 |
| | CUMULATIVE TOTAL | 287 | 599 | 840 | 1082 | 1303 | 1532 | 1735 | 1941 | 2099 | 2258 |
| ANALYSIS | SINGLE-YEAR TOTAL | 121 | 146 | 117 | 104 | 113 | 120 | 103 | 96 | 63 | 70 |
| | CUMULATIVE TOTAL | 121 | 267 | 384 | 488 | 601 | 721 | 824 | 920 | 983 | 1053 |
| MASS | SINGLE-YEAR TOTAL | 21 | 28 | 22 | 25 | 21 | 26 | 27 | 13 | 15 | 13 |
| | CUMULATIVE TOTAL | 21 | 49 | 71 | 96 | 117 | 143 | 170 | 183 | 198 | 211 |
| SOLID | SINGLE-YEAR TOTAL | 13 | 18 | 11 | 5 | 8 | 7 | 8 | 5 | 3 | 4 |
| | CUMULATIVE TOTAL | 13 | 31 | 42 | 47 | 55 | 62 | 70 | 75 | 78 | 82 |
| LASER | SINGLE-YEAR TOTAL | 13 | 13 | 5 | 13 | 19 | 16 | 18 | 7 | 11 | 8 |
| | CUMULATIVE TOTAL | 13 | 26 | 31 | 44 | 63 | 79 | 97 | 104 | 115 | 123 |
| HOLDER | SINGLE-YEAR TOTAL | 49 | 37 | 32 | 29 | 38 | 37 | 25 | 33 | 20 | 23 |
| | CUMULATIVE TOTAL | 49 | 86 | 118 | 147 | 185 | 222 | 247 | 280 | 300 | 323 |
| SPECTROMETER | SINGLE-YEAR TOTAL | 7 | 18 | 14 | 14 | 8 | 10 | 13 | 7 | 8 | 7 |
| | CUMULATIVE TOTAL | 7 | 25 | 39 | 53 | 61 | 71 | 84 | 91 | 99 | 106 |
| ION | SINGLE-YEAR TOTAL | 35 | 50 | 36 | 45 | 30 | 44 | 46 | 26 | 22 | 28 |
| | CUMULATIVE TOTAL | 35 | 85 | 121 | 166 | 196 | 240 | 286 | 312 | 334 | 362 |
| GLYCERIN | SINGLE-YEAR TOTAL | 0 | 0 | 0 | 0 | 0 | 0 | 0 | 0 | 0 | 0 |
| | CUMULATIVE TOTAL | 0 | 0 | 0 | 0 | 0 | 0 | 0 | 0 | 0 | 0 |
| IONIZATION | SINGLE-YEAR TOTAL | 9 | 13 | 9 | 14 | 9 | 10 | 13 | 9 | 8 | 8 |
| | CUMULATIVE TOTAL | 9 | 22 | 31 | 45 | 54 | 64 | 77 | 86 | 94 | 102 |

## FIG. 29

### TRANSITION OF CASE NUMBER FOR EACH IMPORTANT KEYWORD
### (FOR POPULATION) (ACCUMULATION)

KEYWORD
- ◇ GLYCERIN
- ■ APPLICATION
- ▲ SOLID
- ✕ LASER
- ✳ VISCOSITY
- ● CREATING
- + MASS
- △ MIXING
- ○ FINE PARTICLE
- ◆ SOLUTION

(CASES)

(YEAR)

## FIG. 30

### TRANSITION OF CASE NUMBER FOR EACH IMPORTANT KEYWORD
### (FOR POPULATION) (SINGLE-YEAR TOTAL AND CUMULATIVE TOTAL)

| | | 1992 | 1993 | 1994 | 1995 | 1996 | 1997 | 1998 | 1999 | 2000 | 2001 |
|---|---|---|---|---|---|---|---|---|---|---|---|
| GLYCERIN | SINGLE-YEAR TOTAL | 0 | 0 | 0 | 0 | 0 | 0 | 0 | 0 | 0 | 0 |
| | CUMULATIVE TOTAL | 0 | 0 | 0 | 0 | 0 | 0 | 0 | 0 | 0 | 0 |
| APPLICATION | SINGLE-YEAR TOTAL | 2 | 7 | 1 | 3 | 5 | 0 | 2 | 2 | 0 | 2 |
| | CUMULATIVE TOTAL | 2 | 9 | 10 | 13 | 18 | 18 | 20 | 22 | 22 | 24 |
| SOLID | SINGLE-YEAR TOTAL | 13 | 18 | 11 | 5 | 8 | 7 | 8 | 5 | 3 | 4 |
| | CUMULATIVE TOTAL | 13 | 31 | 42 | 47 | 55 | 62 | 70 | 75 | 78 | 82 |
| LASER | SINGLE-YEAR TOTAL | 13 | 13 | 5 | 13 | 19 | 16 | 18 | 7 | 11 | 8 |
| | CUMULATIVE TOTAL | 13 | 26 | 31 | 44 | 63 | 79 | 97 | 104 | 115 | 123 |
| VISCOSITY | SINGLE-YEAR TOTAL | 1 | 2 | 2 | 0 | 1 | 1 | 0 | 3 | 3 | 2 |
| | CUMULATIVE TOTAL | 1 | 3 | 5 | 5 | 6 | 7 | 7 | 10 | 13 | 15 |
| CREATING | SINGLE-YEAR TOTAL | 7 | 8 | 7 | 8 | 5 | 8 | 15 | 11 | 7 | 9 |
| | CUMULATIVE TOTAL | 7 | 15 | 22 | 30 | 35 | 43 | 58 | 69 | 76 | 85 |
| MASS | SINGLE-YEAR TOTAL | 21 | 28 | 22 | 25 | 21 | 26 | 27 | 13 | 15 | 13 |
| | CUMULATIVE TOTAL | 21 | 49 | 71 | 96 | 117 | 143 | 170 | 183 | 198 | 211 |
| MIXING | SINGLE-YEAR TOTAL | 11 | 11 | 15 | 5 | 4 | 10 | 17 | 8 | 7 | 9 |
| | CUMULATIVE TOTAL | 11 | 22 | 37 | 42 | 46 | 56 | 73 | 81 | 88 | 97 |
| FINE PARTICLE | SINGLE-YEAR TOTAL | 2 | 4 | 4 | 0 | 3 | 3 | 5 | 2 | 1 | 6 |
| | CUMULATIVE TOTAL | 2 | 6 | 10 | 10 | 13 | 16 | 21 | 23 | 24 | 29 |
| SOLUTION | SINGLE-YEAR TOTAL | 20 | 14 | 23 | 8 | 16 | 15 | 11 | 12 | 4 | 7 |
| | CUMULATIVE TOTAL | 20 | 34 | 57 | 65 | 81 | 96 | 107 | 119 | 123 | 130 |

EP 1 881 423 A1

FIG. 31

KEYWORD DISTRIBUTION IN RESEARCH CASE
"METHOD OF CREATING SAMPLE FOR LASER IONIZATION MASS SPECTROMETER AND SAMPLE HOLDER"

FIG. 32

S59-267521
SIMPLIFIED DEVICE
FOR MAKING KOJI
USED IN
GINJO BREWING

H04-238856
STEAMING
METHOD

H04-240604
RICE MOISTURE
ADSORPTION
CONTROLLER

H07-083537
METHOD AND
DEVICE FOR
DIPPING
WASHED RICE
USED IN
GINJO BREWING

H03-036269
CONTROLLING
AND BREWING
METHOD OF
MATERIAL RICE
FOR BREWING

H04-093432
IMMERSING
AND DRAINING
DEVICE OF
BREWER'S RICE

H05-254638
PURE SAKE AND
MANUFACTURING
METHOD THEREOF

H07-036799
PURIFYING
METHOD OF
MATERIAL RICE
FOR SAKE
BREWING

H05-164551
BREWING METHOD
OF SAKE AND
CONTROLLER
IN FERMENTING
PROCESS USED
THEREIN

H06-179929
METHOD FOR
MANUFACTURING
SAKE WITH HIGH
CONTENT OF
VITAMIN C

H08-295608
METHOD AND
DEVICE FOR
BREWING SAKE
MASH BY
CENTRIFUGAL
METHOD

H04-322465
METHOD FOR
MANUFACTURING
ALCOHOLIC
DEVERAGES

H06-195085
IMPROVING AGENT
OF ALCOHOLIC
BEVERAGES
AND METHOD
FOR IMPROVING
THEREOF

H05-319084
FILTERING
DEVICE FOR
SAKE BREWING

H06-180844
METHOD FOR
DEACTIVATING
OXYGEN IN
LIQUID FOOD

H04-297745
MANUFACTURING
METHOD OF
ROASTED BARLEY
SHOCHU BY
SUPERHEATED
STEAM

H08-089085
MANUFACTURING
METHOD OF
ALCOHOLIC
BEVERAGES FROM
NON-STEAM
COOKED GRAINS

## FIG. 33

SIMILARITY RANKING AND PUBLICATION CONTENT ABSTRACTS
USING SIMILARITY WITHIN POPULATION

TOP ○○ PUBLICATION LIST

| RANKING | KIND OF DOCUMENT | PUBLICATION No. | INVENTION TITLE | IPC | APPLICANT | SIMILARITY |
|---|---|---|---|---|---|---|
| 1 | B | S61-123456 | LASER IONIZATION··· | G01N 1/28 | ○○CORPORATION | 56.0 |
| 2 | A | H05-098765 | LASER IONIZATION··· | H01J 49/10 . | ○○PHONE | 38.5 |
| 3 | B | S61-076543 | LASER IONIZATION··· | H01J 49/10 | ○○CORPORATION | 36.9 |
| 4 | B9 | H01-260987 | LASER CVD··· | H01L 21/205 | ○○ELECTRONIC | 33.7 |
| 5 | B9 | S62-153212 | LASER IONIZATION··· | H01J 37/256 | ○○CORPORATION | 31.7 |
| 6 | A | H07-012345 | LASER EQUIPMENT··· | G01N 1/22 | ○○ELECTRIC | 30.0 |
| 7 | · | · | · | · | · | · |
| 8 | · | · | · | · | · | · |
| 9 | · | · | · | · | · | · |
| 10 | · | · | · | · | · | · |
| | | | | | | |
| | | | | | | |

## FIG. 34

6 0 0

6 0 2 → INPUT DEVICE

6 0 1 → PROCESSING DEVICE

6 0 3 → RECORDING DEVICE

OUTPUT DEVICE — 6 0 4

## F I G. 35A

| TF(d) | 1 | 2 | 4 | 0 |
|---|---|---|---|---|
| TF(p) | 2 | 0 | 0 | 1 |
| DF(P) | 30 | 20 | 45 | 13 |

## F I G. 35B

| INDEX TERM | RED | BLUE | YELLOW | WHITE |
|---|---|---|---|---|
| DOCUMENT d | 1 * ln(50/30) | 2 * ln(50/20) | 4 * ln(50/45) | 0 |
| DOCUMENT p | 2 * ln(50/30) | 0 | 0 | 1 * ln(50/13) |

EP 1 881 423 A1

FIG. 36

EP 1 881 423 A1

## FIG. 37A

■NUMBER INPUT                                                — 3701

[ SELECT KIND OF PUBLICATION ▽ ]                             — 3702

[ SELECT KIND OF NUMBER ▽ ]                                  — 3704

[ SELECT YEAR ▽ ] [                    ]
3703

■TEXT INPUT

[                                                          ]  — 3705


SET PRIOR APPLICATION PERIOD

[                  ]IN AND BEFORE          [ NEXT ]

■CONTENT SELECTION 3706
  ■BASIC PACKAGE
  □FULL PACKAGE
  □STRUCTURE DIAGRAM

## FIG. 37B

■CONFIRMATION SCREEN

APPLICATION No.: ＊ ＊ ＊ ＊
PUBLICATION No.: ＊ ＊ ＊ ＊
PATENT No.(PUBLISHED No.) : ＊ ＊ ＊ ＊
APPLICANT：○○○
TITLE OF THE INVENTION：○○○○

[ RETURN ] [ NEXT ]

## FIG. 38

START

PRE-PROCESS RESEARCH CASE DATA — S3801

CREATE POPULATION — S3802

TOTALING PROCESSING — S3803

CREATE
PATENT STRUCTURE DIAGRAM — S3804

OBTAIN IPC DATA — S3805

CREATE DATA IN
PRESCRIBED OUTPUT FORM — S3806

END NOTIFICATION TO
MANAGEMENT SERVER — S3807

RETURN

## FIG. 39

```
        TOTALING
       PROCESSING

                                              S3901
     RANKING TOTALING

                                              S3902
    TIME-SERIES TOTALING

                                              S3903
     MATRIX TABULATION

                                              S3904
   CALCULATE INSIDE POPULATION
       SIMILARITY MEASURE

                                              S3905
     CALCULATE COORDINATES
        FOR IDF PLAN VIEW
   (FREQUENCY SCATTER DIAGRAM)

          RETURN
```

## FIG. 40

```
        ┌─────────────────────────┐
        │  CREATING FREQUENCY     │
        │  SCATTER DIAGRAM        │
        └─────────────────────────┘
                    │
        ┌─────────────────────────┐  S4001
        │   EXTRACT INDEX TERMS   │
        └─────────────────────────┘
                    │
        ┌─────────────────────────┐  S4002
        │   CALCULATE KEYWORD     │
        │ IMPORTANCE DEGREES DF(P)│
        └─────────────────────────┘
                    │
        ┌─────────────────────────┐  S4003
        │ CALCULATE TF(d) AND IDF(P),│
        │ CREATE DOCUMENT VECTOR(d)  │
        └─────────────────────────┘
                    │
        ┌─────────────────────────┐  S4004
        │ CALCULATE TF(p) AND IDF(P),│
        │ CREATE DOCUMENT VECTOR(p)  │
        └─────────────────────────┘
                    │
        ┌─────────────────────────┐  S4005
        │   CALCULATE SIMILARITIES, │
        │ SEGMENT POPULATION DOCUMENTS S│
        └─────────────────────────┘
                    │
        ┌─────────────────────────┐  S4006
        │   CALCULATE KEYWORD     │
        │ IMPORTANCE DEGREES DF(S)│
        └─────────────────────────┘
                    │
        ┌─────────────────────────┐  S4007
        │   CALCULATE IDF(P) FOR  │
        │     EACH KEYWORD IN d   │
        └─────────────────────────┘
                    │
        ┌─────────────────────────┐  S4008
        │   CALCULATE IDF(S) FOR  │
        │     EACH KEYWORD IN d   │
        └─────────────────────────┘
                    │
        ┌─────────────────────────┐  S4009
        │  SCATTER KEYWORDS USIND │
        │IDF(P) AS x-AXIS AND IDF(S) AS y-AXIS│
        └─────────────────────────┘
                    │
        ┌─────────────────────────┐  S4010
        │  DEVELOP PHENOGRAM BY   │
        │ COORDINATE TRANSFORMATION│
        └─────────────────────────┘
                    │
             ┌──────────┐
             │  RETURN  │
             └──────────┘
```

## FIG. 41

```
DOCUMENT READ OUT UNIT                                    ┌─ 4110
    │                                                      CONDITION
    │                                                      RECORDING UNIT  ╌─ 4013
    ▼                              ┌─ 4130
TIME DATA          INDEX TERM DATA
EXTRACTION UNIT    EXTRACTION UNIT
    │                     │
   420                    ▼      ┌─ 4140
              SIMILARITY
              CALCULATION UNIT
                     │
                     ▼      ┌─ 4150
              TREE-LIKE DIAGRAM          ──►   WORK RESULT
              CREATING UNIT                    STORAGE UNIT
                     │
                     ▼      ┌─ 4160
              CUTTING CONDITION   ◄──
              READ OUT UNIT
                     │
                     ▼      ┌─ 4170
              CLUSTER            ──►
              EXTRACTION UNIT    ◄──
                     │
                     ▼      ┌─ 4180          DOCUMENT
              ARRANGEMENT CONDITION ◄──     STORAGE UNIT
              READ OUT UNIT      ──►
                     │
    │                ▼      ┌─ 4190
INSIDE CLUSTER ELEMENT ARRANGEMENT UNIT
```

EP 1 881 423 A1

# FIG. 42

START

READ OUT DOCUMENT — S4210

EXTRACT TIME DATA — S4220

EXTRACT INDEX TERM DATA — S4230

SIMILARITY CALCULATION — S4240

CREATE TREE-LIKE DIAGRAM — S4250

READ OUT CUTTING CONDITION — S4260

EXTRACT CLUSTER — S4270

READ OUT ARRANGEMENT CONDITION — S4280

ARRANGE INSIDE CLUSTER ELEMENTS — S4290

RETURN

# FIG. 43

START

S4310 — READ OUT DOCUMENT

S4320 — EXTRACT TIME DATA

S4330 — EXTRACT INDEX TERM DATA

S4340 — SIMILARITY CALCULATION

S4350 / FIG.44A — CREATE TREE-LIKE DIAGRAM

S4360 — READ OUT CUTTING CONDITION

S4371 / FIG.44B — CUT CUTTING HEIGHT $\alpha = a$

S4372 — CUT ?

YES

NO

S4373 — CUT CUTTING HEIGHT $\alpha = a*$

S4374 / FIG.44C — ARRANGE OLDEST ELEMENT AT HEAD FOR EACH CLUSTER

S4375 — DELETE INDEX DIMENSION WITH SMALL DEVIATION

S4376 / FIG.44D — CREATE PARTIAL TREE-LIKE DIAGRAM

S4377 — NUMBER OF INSIDE CLUSTER ELEMENTS EXCLUDING OLDEST ELEMENT $\leqq 3$ ?

NO

YES

S4378 / FIG.44E — CUT CUTTING HEIGHT $\alpha = a$

S4380 — READ OUT ARRANGEMENT CONDITION

S4390 / FIG.44F — ARRANGE INSIDE CLUSTER ELEMENTS FOR EACH CLUSTER

RETURN

## FIG. 44A

$E_1$

$E_2$   $E_3$   $E_4$   $E_5$   $E_6$   $E_7$   $E_8$   $E_9$   $E_{10}$

## FIG. 44B

$E_1$

$- - - - - - - - - - - - - - - - - - - - - \alpha$

$E_2$   $E_3$   $E_4$   $E_5$   $E_6$   $E_7$   $E_8$   $E_9$   $E_{10}$

## FIG. 44C

$E_1$

$E_2$   $E_7$

$E_3$   $E_4$   $E_5$   $E_6$   $E_8$   $E_9$   $E_{10}$

## FIG. 44D

$E_2$   $E_7$

$- - - - - - - - - - - - - \alpha$

$E_3$   $E_4$   $E_5$   $E_6$   $E_8$   $E_9$   $E_{10}$

## FIG. 44E

$E_7$

$\alpha = a_x - - - - - - - - -$

$\alpha = a_y - - - - - - - - -$

$\alpha = a_z - - - - - - - - -$

$E_8$   $E_9$   $E_{10}$

## FIG. 44F

$E_1$

$E_2$   $E_7$

$E_3$   $E_5$   $E_8$

$E_4$   $E_6$   $E_9$

$E_{10}$

## FIG. 45

4503

4510 — DOCUMENT READ OUT UNIT

4520 — INDEX TERM EXTRACTION UNIT

4530 — HIGH FREQUENCY TERM EXTRACTION UNIT

4540 — HIGH FREQUENCY TERM-INDEX TERM CO-OCCURRENCE DEGREE CALCULATION UNIT

4550 — CLUSTERING UNIT

4560 — INDEX TERM-GROUND CO-OCCURRENCE DEGREE CALCULATION UNIT

4570 — key(w) CALCULATION UNIT

Skey(w) CALCULATION UNIT

4580

4700 — EVALUATION VALUE CALCULATION UNIT

CONCENTRATION DEGREE CALCULATION UNIT

4710

SHARE CALCULATION UNIT

4720

4730 — FIRST INVERSE CALCULATION UNIT

4740 — SECOND INVERSE CALCULATION UNIT

4750 — CREATIVITY DEGREE CALCULATION UNIT

4760 — KEYWORD EXTRACTION UNIT

CONDITION RECORDING UNIT

WORK RESULT STORAGE UNIT

DOCUMENT STORAGE UNIT

## FIG. 46

START

4601 — READ OUT DOCUMENT

4602 — EXTRACT INDEX TERMS

4603 — CALCULATE GF(E) OF EACH INDEX TERM
TABLE 2

4604 — EXTRACT HIGH FREQUENCY INDEX TERMS

4605 — CALCULATE HIGH FREQUENCY TERM-INDEX TERM CO-OCCURRENCE DEGREE
TABLE 3

4606 — CALCULATE SIMILARITY MEASURE FOR CO-OCCURRENCE DEGREE AMONG HIGH FREQUENCY TERMS
TABLE 4

5607 — CREATE TREE-LIKE DIAGRAM OF HIGH FREQUENCY TERMS

4608 — FORM GROUND BY CUTTING TREE-LIKE DIAGRAM

4609 — CALCULATE INDEX TERM-GROUND CO-OCCURRENCE DEGREE
TABLE 5

4610 — CALCULATE key(w)
TABLE 6

4611 — CALCULATE Skey(w)

4612 — CALCULATE EVALUATION VALUES

4613 — CALCULATE CONCENTRATION DEGREES

4614 — CALCULATE SHARES

4615 — CALCULATE FIRST INVERSE

4616 — CALCULATE SECOND INVERSE

4617 — CALCULATE CREATIVITY DEGREES

4618 — EXTRACT KEYWORDS

RETURN

# FIG. 47

MANAGEMENT SERVER  — 512

SECOND ANALYSIS SERVER  — 514

Zip FILE  — S4701

OUTPUT IDF INFORMATION  — S4702

Zip FILE  — S4703

Zip FILE  — S4704

OUTPUT KEYWORD ATTRIBUTES/MAIN APPLICANT INFORMATION  — S4705

Zip FILE  — S4706

FILE CREATING SERVER  — 516

Zip FILE  — S4707

OUTPUT CLUSTER INFORMATION FILE  — S4708

Zip FILE  — S4709

**FIG. 48**

CLIENT  WEB SERVER

```
┌─────────────────────┐
│      REQUEST         │
└─────────────────────┘
            │
            │              ┌─────────────────────┐
            │              │   DISTRIBUTE HTML    │
            │              └─────────────────────┘
┌─────────────────────┐            │
│ REQUEST LOG-IN SCREEN│────────────┤
└─────────────────────┘            │
            │              ┌──────────────────────────┐
            │              │ DISTRIBUTE LOG-IN SCREEN  │
            │              └──────────────────────────┘
┌─────────────────────┐            │
│      LOG IN          │◄───────────┐
└─────────────────────┘            │
            │                       │
                    ◇ AUTHENTICATED? ◇──── N ──┘
                           │
                           Y
                  ┌──────────────────────────┐
                  │  DISTRIBUTE INPUT SCREEN  │
                  └──────────────────────────┘
┌──────────────────────┐
│ INPUT DOCUMENT TO BE  │
│ SURVEYED INFORMATION  │
└──────────────────────┘
┌──────────────────────┐
│   INPUT CONTENT       │
│ SELECTING INFORMATION │
└──────────────────────┘
                  ┌──────────────────────┐
                  │    IDENTIFY CASES     │
                  └──────────────────────┘
                  ┌──────────────────────┐
                  │    TRANSMIT CASES     │
                  └──────────────────────┘
```

MANAGEMENT SERVER

```
                    ◇ PRIOR CASE       ◇
  ┌─────────┐◄── Y ─┤ PRESENT/ABSENT?  │
  │ STAND BY│        └──────────────────┘
  └─────────┘                │
                             N
                  ┌──────────────────────┐
                  │     INPUT CASES       │────► TO FIG. 49
                  └──────────────────────┘
```

# FIG. 49

ANALYSIS SERVER                                    DATABASE SERVER

FROM FIG. 48

## FIG. 50

CLIENT SERVER          WEB SERVER          MANAGEMENT SERVER          ANALYSIS SERVER

①②③④ ──────────────┐

CREATE REPORT

TRANSFER REPORT

TRANSFER REPORT

OBTAIN REPORT

END NOTIFICATION

OBTAIN END NOTIFICATION

REQUEST

DISTRIBUTE
LOG-IN SCREEN

INPUT
LOG-IN INFORMATION

AUTHENTICATED?   N

Y

DISTRIBUTE SCREEN OF
PURCHASED REPORT LIST

REQUEST OUTPUT
OF REPORT

TRANSFER REPORT

OBTAIN REPORT

DISPLAY/STORE/OUTPUT
REPORT

EP 1 881 423 A1

**INTERNATIONAL SEARCH REPORT**

| International application No. |
| --- |
| PCT/JP2006/308669 |

| A. CLASSIFICATION OF SUBJECT MATTER |
| --- |
| *G06F17/30*(2006.01)i, *G06F19/00*(2006.01)i |
| According to International Patent Classification (IPC) or to both national classification and IPC |

| B. FIELDS SEARCHED |
| --- |
| Minimum documentation searched (classification system followed by classification symbols)<br>G06F17/30, G06F19/00 |
| Documentation searched other than minimum documentation to the extent that such documents are included in the fields searched<br>Jitsuyo Shinan Koho    1922-1996   Jitsuyo Shinan Toroku Koho   1996-2006<br>Kokai Jitsuyo Shinan Koho   1971-2006   Toroku Jitsuyo Shinan Koho   1994-2006 |
| Electronic data base consulted during the international search (name of data base and, where practicable, search terms used) |

**C. DOCUMENTS CONSIDERED TO BE RELEVANT**

| Category* | Citation of document, with indication, where appropriate, of the relevant passages | Relevant to claim No. |
| --- | --- | --- |
| P,X<br><br>P,A | JP 2005-128978 A (Intellectual Property Bank Corp.),<br>19 May, 2005 (19.05.05),<br>Full text; all drawings<br>(Family: none) | 1-6,12-18,<br>24-29<br>7-11,19-23,<br>30-38 |
| A | WO 2004/061714 A1 (Intellectual Property Bank Corp.),<br>22 July, 2004 (22.07.04),<br>Full text; all drawings<br>(Family: none) | 1-38 |

☒ Further documents are listed in the continuation of Box C. ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>31 July, 2006 (31.07.06) | Date of mailing of the international search report<br>08 August, 2006 (08.08.06) |
| --- | --- |
| Name and mailing address of the ISA/<br>Japanese Patent Office | Authorized officer |
| Facsimile No. | Telephone No. |

Form PCT/ISA/210 (second sheet) (April 2005)

0000000000000000000000000000000000000000000000000000 **EP 1 881 423 A1**

**INTERNATIONAL SEARCH REPORT**

International application No.

PCT/JP2006/308669

C (Continuation). DOCUMENTS CONSIDERED TO BE RELEVANT

| Category* | Citation of document, with indication, where appropriate, of the relevant passages | Relevant to claim No. |
|---|---|---|
| A | Kazunari ISHIDA, Toshizumi OTA, "Yogokan Kankei ni Chakumoku shita Bunshokan Kankei ni Kansuru Tokeiteki Bunseki to Bunseki Shien System no Kaihatsu", Information Processing Society of Japan Kenkyu Hokoku, 16 July, 1999 (16.07.99), Vol.99, No.57, pages 49 to 56; 99-FI-55-7 | 1-38 |

Form PCT/ISA/210 (continuation of second sheet) (April 2005)

108

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11073415 A